# EUROPEAN PATENT SPECIFICATION

(11) **EP 3 544 688 B1**
(45) Date of publication and mention of the grant of the patent: **08.03.2023**
(21) Application number: 17803882.4
(22) Date of filing: 17.11.2017
(51) Int. Cl.: A61Q 5/02, A61Q 5/06, A61K 8/81, C11D 3/37, C11D 1/29, C08F 220/28, C08F 220/54, C08F 226/04

(54) **COSMETIC COMPOSITION COMPRISING A CATIONIC COPOLYMER**
KOSMETISCHE ZUSAMMENSETZUNG MIT EINEM KATIONISCHEN COPOLYMER
COMPOSITION COSMÉTIQUE COMPRENANT UN COPOLYMÈRE CATIONIQUE

(30) Priority: 28.11.2016 EP 16201010
(43) Date of publication of application: 02.10.2019
(73) Proprietor: Clariant International Ltd, 4132 Muttenz (CH)
(72) Inventor: STRICANE, Charlotte, 31000 Toulouse (FR); HAEUSSLER, Matthias, 06246 Bad Lauchstaedt (DE); SAHL, Mike, 65520 Bad Camberg (DE); GABEL, Dorothee, 65207 Wiesbaden (DE); KITA-TOKARCZYK, Katarzyna, 65812 Bad Soden (DE)
(74) Representative: Kampen, Daniela
(86) International application number: PCT/EP2017/079605
(87) International publication number: WO 2018/095813

(56) References cited:
- EP-A1- 2 110 117
- FR-A1- 2 985 727
- JP-A- 2001 181 354
- DATABASE WPI Week 201626 1 June 2016 (2016-06-01) Thomson Scientific, London, GB; AN 2015-741090 XP002769807, & CN 105 037 644 A (GUANGDONG LINGJIE MFG CHEM CO LTD) 11 November 2015 (2015-11-11)
- Nn: "Innovative Ingredients for Cosmetics", , 1 January 2015 (2015-01-01), pages 1-12, XP055368798, Retrieved from the Internet: URL:http://www.goo-chem.co.jp/english/prod uct/pdf/cosmetic/cosmetics_catalogue_en_20 13.pdf [retrieved on 2017-05-03]

## Description

### FIELD OF THE INVENTION

The present invention relates to a cosmetic composition comprising a specific copolymer and a cosmetically acceptable component. It also relates to methods of using such cosmetics compositions, uses thereof, as well as processes for formulating such a cosmetic composition.

### BACKGROUND OF THE INVENTION

Methods for cleaning and conditioning the hair, skin and body are known for centuries. Frequently, the cleansing methods require an alkaline agent, such as soap. Cleansing the skin, scalp, and hair is very important for general hygiene and removal of unwanted materials such as sebum, oils, dirt, makeup, and all cosmetic products applied with the intent of beautification. It is a known practice to use foaming cleansing products to clean the skin, body and hair. Whilst surfactant-based system as foaming cleansing products are known in the art, consumers expect that their skin, body and hair are not excessively stripped of its condition. Moreover, certain consumers may desire other performance benefits from cleansing products, such as hair body, hair volume, skin conditioning etc.

The interaction of alkaline agents with keratinous fibres results in weakening of the fibres' mechanical properties and increases the porosity of hair fibres. Many known shampooing components result in dry hair that is damaged. Furthermore, the colour of hair often fades with time due to washing and upon exposure to environmental factors such as sun and pollution. This can lead to dull appearance of the hair and results in more frequent washing than required. Frequent washing of hair may result in more damaged and less conditioned hair. Such damaged hair may also appear flat and lifeless.

In order to improve the condition of hair, to keep a natural look of the hair over a longer period of time, and to improve other hair properties, such as hair volume, improved hair care regimens are required as well as the compositions used in such regimens.

The surface of keratin contains negatively charged amino acids. For this reason, hair conditioners can contain cationic components (e.g. surfactants) which are not washed out completely. The hydrophilic ends of the cationic components can bind to the keratin, whereas the hydrophobic ends of the molecules protect the hair surface.

Modern hair conditioning shampoos compositions are often intended to soften the hair, to improve the gloss of hair and to avoid a greasy look of the hair. Silicone derivatives, and quaternary ammonium compounds (surfactants or polymers) which coat the cuticle of the hair were used as conditioning ingredients in shampoo compositions. Such shampoos can be made transparent or opaque.

There is nevertheless the desire for providing components for cosmetic compositions that can be easily formulated into aqueous cosmetic compositions and provide improved performance advantages to the cosmetic compositions. There is a particular desire for polymeric components that can provide volume and anti-frizz benefits to keratin fibres such as hair without compromising on hair feel.

### SUMMARY OF THE INVENTION

In a first aspect, the present invention relates to a cosmetic composition comprising:
(i) A copolymer comprising:
   (A) from 0.1 mol-% to 99.8 mol-% of one or more cationic structural units (A); and
   (B) from 0.1 mol-% to 99.8 mol-% of one or more macromonomeric structural units (B); and
   (C) from 0.1 mol-% to 99.8 mol-% of one or more structural units (C) which differ from the structural units (A) and (B); and
(ii) A cosmetically acceptable component;
   wherein the one or more cationic structural units (A) are represented by the following general formulae (I) and/or (II): 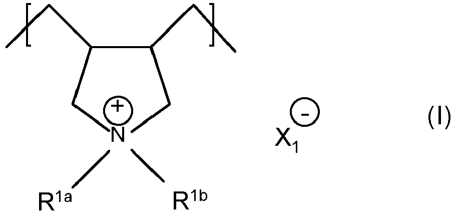 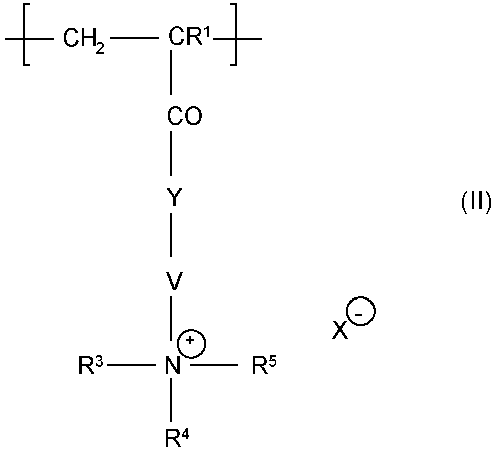
   - R¹ and R^{1a}: are each identical or different and, independently of one another, are each hydrogen and/or a methyl radical,
   - R^{1b}, R³, R⁴ and R⁵: are each identical or different and, independently of one another, are each represented by hydrogen, an aliphatic hydrocarbon radical having 1 to 20 carbon atoms, preferably 1 to 4 carbon atoms, a cycloaliphatic hydrocarbon radical having 5 to 20 carbon atoms, preferably 5 to 8 carbon atoms, an aryl radical having 6 to 14 carbon atoms and/or polyethylene glycol (PEG); preferably are each identical or different and, independently of one another, are each represented by hydrogen and/or methyl; more preferably are each methyl,
   - Y: is identical or different and is represented by oxygen, NH and/or NR³,
   - V: is identical or different and is represented by -(CH₂)ₓ-,

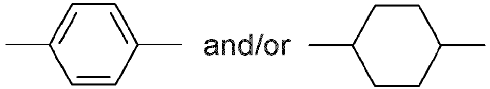

   - x: is identical or different and is represented by an integer from 1 to 6,
   - X and X₁: are each identical or different and, independently of one another, are each represented by a halogen atom, C₁ to C₄ alkyl sulfate and/or C₁ to C₄- alkylsulfonate,
   and the one or more macromonomeric structural units (B) are represented by the general formula (III): 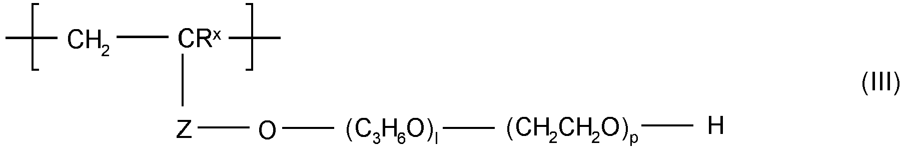 in which
   - R^{x}: is identical or different and is represented by H and/or methyl,
   - Z: is identical or different and is represented by C=O and/or O(CH₂)₄, preferably Z is O(CH₂)₄,
   - I: is on molar average a number from 0 to 6, preferably from 0 to 5, and
   - p: is, on molar average, a number from 1 to 150; preferably from 4 to 150; more preferably from 8 to 150,
   and the one or more structural units (C) are selected from the group consisting of the polymerization product of at least one cyclic N-vinyl-substituted lactam having 5 to 7 ring atoms and structural units of the following general formulae (IV) and (V): 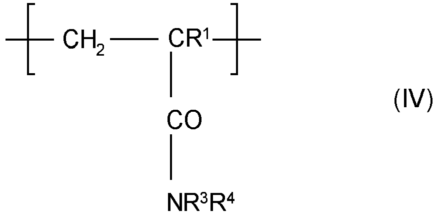 in which
   - R¹: is identical or different and is hydrogen and/or methyl, and
   - R³ and R⁴: are each identical or different and independently of one another are each represented by hydrogen, an aliphatic hydrocarbon radical having 1 to 20, preferably 1 to 4, carbon atoms, a cycloaliphatic hydrocarbon radical having 5 to 20, preferably 5 to 8, carbon atoms, an aryl radical having 6 to 14 Carbon atoms, an alkylaryl radical having 7 to 14 carbon atoms, a branched or unbranched C₁-C₅ monohydroxyalkyl group and/or polyethylene glycol (PEG) 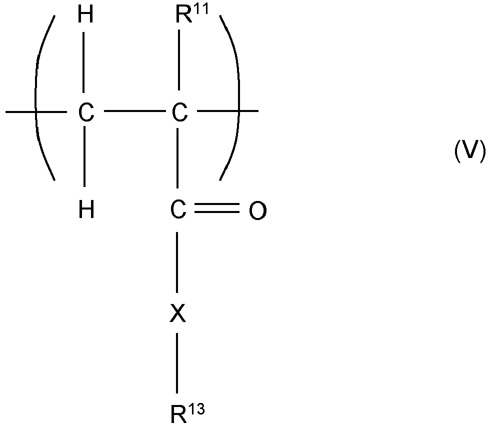
in which
- R¹¹: is identical or different and is represented by H and/or methyl;

- X: is identical or different and is represented by NH-(CₙH₂ₙ) where n = 1, 2, 3 or 4; and
- R¹³: is identical or different and is represented by OH, N(CH₃)₂, SO₃H, PO₃H₂, O-PO₃H₂ and/or para-substituted C₆H₄-SO₃H,
and where, among the structural units of the formula (V) in which R¹³ is N(CH₃)₂, preference is given to those structural units which represent the polymerization product of at least one monomer species selected from the group consisting of [3-(methacryloylamino)propyl]dimethylamine (R¹¹ = methyl; X = NH-(CₙH₂ₙ) where n = 3, and R¹³ = N(CH₃)₂) and [3-(acryloylamino)propyl]dimethylamine (R¹¹ = H; X = NH-(CₙH₂ₙ) where n = 3, and R¹³ = N(CH₃)₂),
and wherein the weight average molecular weight M_{w} of the copolymer is from 10 000 to 200 000 g/mol.

### DETAILED DESCRIPTION OF THE INVENTION

### Definitions and general

In this document, including in all embodiments of all aspects of the present invention, the following definitions apply unless specifically stated otherwise. All percentages are by weight (w/w) of the total composition. All ratios are weight ratios. "wt.-%" means percentage by weight. References to 'parts' e.g. a mixture of 1 part X and 3 parts Y, is a ratio by weight. "QS" or "QSP" means sufficient quantity for 100 % or for 100 g. +/- indicates the standard deviation. All ranges are inclusive and combinable. The number of significant digits conveys neither a limitation on the indicated amounts nor on the accuracy of the measurements. All numerical amounts are understood to be modified by the word "about". All measurements are understood to be made at 23 °C and at ambient conditions, where "ambient conditions" means at 1 atmosphere (atm) of pressure and at 50 % relative humidity. "Relative humidity" refers to the ratio (stated as a percent) of the moisture content of air compared to the saturated moisture level at the same temperature and pressure. Relative humidity can be measured with a hygrometer, in particular with a probe hygrometer from VWR^{®} International. Herein "min" means "minute" or "minutes". Herein "mol" means mole. Herein "g" following a number means "gram" or "grams". "Ex." means "example". All amounts as they pertain to listed ingredients are based on the active level ('solids') and do not include carriers or by-products that may be included in commercially available materials. Herein, "comprising" means that other steps and other ingredients can be in addition. "Comprising" encompasses the terms "consisting of" and "consisting essentially of". The compositions, formulations, methods, uses, kits, and processes of the present invention can comprise, consist of, and consist essentially of the elements and limitations of the invention described herein, as well as any of the additional or optional ingredients, components, steps, or limitations described herein. Embodiments and aspects described herein may comprise or be combinable with elements, features or components of other embodiments and/or aspects despite not being expressly exemplified in combination, unless an incompatibility is stated. "In at least one embodiment" means that one or more embodiments, optionally all embodiments or a large subset of embodiments, of the present invention has/have the subsequently described feature. Where amount ranges are given, these are to be understood as being the total amount of said ingredient in the composition, or where more than one species fall within the scope of the ingredient definition, the total amount of all ingredients fitting that definition, in the composition. For example, if the composition comprises from 1 % to 5 % fatty alcohol, then a composition comprising 2 % stearyl alcohol and 1 % cetyl alcohol and no other fatty alcohol, would fall within this scope.

"Independently selected from," means that the referenced groups can be the same, different, or a mixture thereof, unless the context clearly indicates otherwise. Thus, under this definition, the phrase "X1, X2, and X3 are independently selected from noble gases" would include the scenario where X1, X2, and X3 are all the same, where X1, X2, and X3 are all different, and where X1 and X2 are the same but X3 is different.

"Molecular weight" or "M.Wt.", "Mw", "M_{w}" or "MW' and grammatical equivalents mean the weight average molecular weight, unless otherwise stated. The weight average molecular weight can be measured by gel permeation chromatography (GPC), also referred to as size exclusion chromatography (SEC). The molecular weight of polymers and how this is measured is described in the textbook "Principles of Polymerization" by Georg Odian, third edition, Wiley-Interscience, New York, in chapter 1 - 4, page 19 to 24, ISBN 0-471-61020-8. The weight-average molecular weights M_{w} of the copolymers of the present invention are determined via gel permeation chromatography (GPC) and carried out according to the following procedure and under the following conditions.

| | |
|---|---|
| Column: | PSS Novema 1 x 30 000 Å, 2 x 1000 Å, 10 micron, 300 mm x 8 mm |
| Detector: | RID (refractive index detector) |
| Oven temperature: | 25 °C |
| Flow: | 1 ml/min |
| Injection volume: | 10 microlitres |
| Eluent: | 79.7 vol-% 0.1M NaCl + 0.3 vol-% TFA (trifluoroacetic acid) + 20.0 vol-% ACN (acetonitrile) |
| Calibration method: | poly(2-vinylpyridine) calibration in the molecular weight range of 1,110 to 1,060,000 Dalton |
| Sample preparation: | Weigh approx. 10 mg sample in 10 ml eluent and shake for 15 min. |

"Viscosity" is measured at 25 °C using a HAAKE Rotation Viscometer VT 550 with cooling/heating vessel and sensor systems according to DIN 53019 at a shear rate of 12.9 s⁻¹.

"Water-soluble" refers to any material that is sufficiently soluble in water to form a clear solution to the naked eye at a concentration of 0.1 % by weight of the material in water at 25 °C. The term "water-insoluble" refers to any material that is not "water-soluble".

"Dry" or "substantially dry" means comprising less than 5 %, less than 3 % or, less than 2 %, less than 1 %, or about 0 % of any compound or composition being in liquid form when measured at 25 °C at ambient conditions. Such compounds or compositions being in liquid form include water, oils, organic solvents and other wetting agents. "Anhydrous" means that the composition comprises less than 5 %, less than 3 % or, less than 2 %, less than 1 %, or about 0 % water by total weight of the composition.

"Substantially free from" or "substantially free of" means less than 1 %, or less than 0.8 %, or less than 0.5 %, or less than 0.3 %, or about 0 %, by total weight of the composition or formulation.

"Hair" means mammalian keratin fibres including scalp hair, facial hair and body hair. It includes such hair still being attached to a living subject and also hair that has been removed therefrom such as hair swatches and hair on a doll/mannequin. In at least one embodiment, "hair" means human hair. "Hair shaft" or "hair fibre" means an individual hair strand and may be used interchangeably with the term "hair."

"Cosmetically acceptable" means that the compositions, formulations or components described are suitable for use in contact with human keratinous tissue without undue toxicity, incompatibility, instability, allergic response, and the like. All compositions and formulations described herein which have the purpose of being directly applied to keratinous tissue are limited to those being cosmetically acceptable.

"Derivatives" includes but is not limited to, amide, ether, ester, amino, carboxyl, acetyl, acid, salt and/or alcohol derivatives of a given compound. In at least one embodiment, "derivatives thereof" means the amide, ether, ester, amino, carboxyl, acetyl, acid, salt and alcohol derivatives.

"Monomer" means a discrete, non-polymerised chemical moiety capable of undergoing polymerisation in the presence of an initiator or any suitable reaction that creates a macromolecule e.g. such as polycondensation, polyaddition, anionic or cationic polymerization. "Unit" means a monomer that has already been polymerised i.e. is part of a polymer.

"Polymer" means a chemical formed from the polymerisation of two or more monomers. The term "polymer" shall include all materials made by the polymerisation of monomers as well as natural polymers. Polymers made from only one type of monomer are called homopolymers. Herein, a polymer comprises at least two monomers. Polymers made from two or more different types of monomers are called copolymers. The term "polymer" used herein includes any type of polymer including homopolymers and copolymers.

"Charge density" the cationic charge density of a cationic polymer can be determined according to the Neldahl Method. Those skilled in the art will recognize that the charge density of amino-containing polymers may vary depending upon pH and the isoelectric point of the amino groups. The charge density should be within the above limits at the pH of intended use.

### Explanation Of and Benefits Provided By the Invention

Surprisingly, it has now been found that copolymers according to the present invention can be used in cosmetic compositions. The copolymers work well in cosmetic compositions since they are hydrophilic. Many cosmetic compositions contain water and therefore such copolymers are easily formulated into such water-containing compositions.

Furthermore, it has been found that the hydrophilic copolymers according to the present invention, which contain cationic hair-anchoring groups and hydrophilic macromonomers cause a volume increase when applied to keratin fibres, particularly in a shampoo formulation. Such a finding is totally unexpected as most volume-providing polymers known in the art are hydrophobic. Without wanting to be bound by theory, the hydrophilic macromonomers contain polyethylene glycol chains, which are known to be hydroscopic and thus attract water. The water attracted by these copolymers increases the volume of the hair. These copolymers are readily soluble. The advantage of using such copolymers is that they are easy to formulate due to their hydrophilic nature without causing lumps or fish eyes. The copolymers furthermore are colourless, do not alter the viscosity significantly at the recommended use level and are not pH sensitive, features highly desired for preparing a shampoo formulation.

US 6,410,005 B1 (PMD Holdings Corp.) relates to a block copolymer for hair styling compositions including hydrophilic and hydrophobic blocks which allow for optimization of desirable characteristics of the hair styling composition, such as fiow onto the hair, prevention of curl droop, style retention at high humidity, tack, hardness, resistance to flaking, restylability, volumizing, and washability from the hair. US 6,410,005 B1, however, discloses branched polymers i.e. is made with branching/crosslinking units and discloses mainly hydrophobic polymers.

EP2116225A1, EP2116226A1 and EP2098217A2 (Beiersdorf AG) disclose conditioning compositions. EP2116225A1 discloses a rinse-out conditioner for increased hair volume. EP2116226A1 discloses hair care formulas with cationic amphoteric polymers. EP2098217A2 discloses a volumising hair fixer with ampholytic copolymer. EP2116225A1, for example, fails to disclose macromonomeric units.

DE10358587A1 (2005, Schwarzkopf und Henkel) discloses a volumizing hair preparation containing vinylpyrrolidone-vinylcaprolactam-acrylate monomer terpolymer. EP2116225A1 for example, fails to disclose macromonomeric units.

FR2985727A1 discloses polymers comprising a cationic polymer and a polyethyleneglycol (meth)acrylate-type monomer. JP2001181354 discloses a cationic polymer comprising a monomer having a quaternary cationic group and a monomer having a polyoxyethylene chain. CN105037644 discloses a method of preparing a high-molecular cationic surfactant which involves a macromonomer, a water-soluble monomer and a cationic monomer. "Innovative Ingredients for Cosmetics", GOO Personal Care Product Catalogue For Cosmetics, GOO CHEMICAL CO., LTD. discloses polymers such as POLYQUATERNIUM-48 and POLYQUATERNIUM-49. EP2110117A1 discloses volume giving cleansing compositions.

The details of the invention, its aspects and preferred or optional features are provided hereinafter.

### First Aspect

The first aspect relates to a cosmetic composition comprising: (i) a copolymer; and (ii) a cosmetically acceptable component. The copolymer (i) is a specific copolymer.

In at least one embodiment, the cosmetic composition comprises from 0.1 wt.-% to 15 wt.-% copolymer (i).

### Copolymer (i)

The copolymer comprises from 0.1 mol-% to 99.8 mol-% of one or more cationic structural units (A); and from 0.1 mol-% to 99.8 mol-% of one or more macromonomeric structural units (B). In at least one embodiment, the copolymer copolymer comprises from 5.0 mol-% to 50 mol-%, more preferably from 7.5 mol-% to 33.7 mol-% of one or more cationic structural units (A). In at least one embodiment, the copolymer copolymer comprises from 1.0 mol-% to 50.0 mol-% more preferably from 3.7 mol-% to 43.3 mol-% of one or more macromonomeric structural units (B).

In at least one embodiment, the copolymer consists of one or more cationic structural units (A), one of more macromonomeric structural units (B) and one or more structural units (C), wherein said units (A), (B) and (C) are as defined herein.

In at least one embodiment, the copolymer has a comb structure.

The weight average molecular weight M_{w} of the copolymer is from 10 000 to 200 000 g/mol, preferably from 15 000 to 200 000 g/mol, and more preferably from 20 000 to 200 000 g/mol.

### Cationic structural units (A)

The one or more cationic structural units (A) are represented by the following general formulae (I) and/or (II): 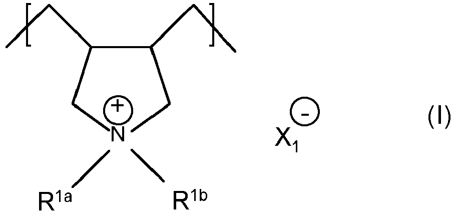 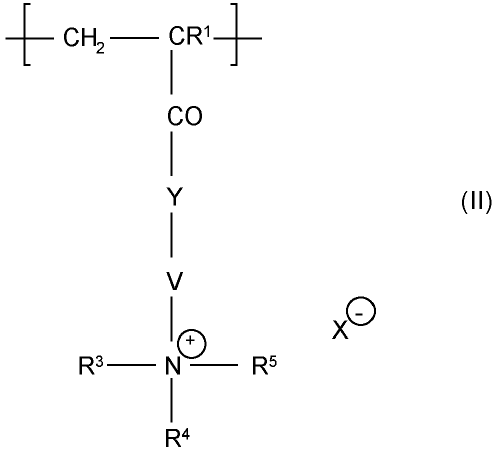
- R¹ and R^{1a}: are each identical or different and, independently of one another, are each hydrogen and/or a methyl radical,
- R^{1b}, R³, R⁴ and R⁵: are each identical or different and, independently of one another, are each represented by hydrogen, an aliphatic hydrocarbon radical having 1 to 20 carbon atoms, preferably 1 to 4 carbon atoms, a cycloaliphatic hydrocarbon radical having 5 to 20 carbon atoms, preferably 5 to 8 carbon atoms, an aryl radical having 6 to 14 carbon atoms and/or polyethylene glycol (PEG); preferably are each identical or different and, independently of one another, are each represented by hydrogen and/or methyl; more preferably are each methyl,
- Y: is identical or different and is represented by oxygen, NH and/or NR³,
- V: is identical or different and is represented by -(CH₂)ₓ-, 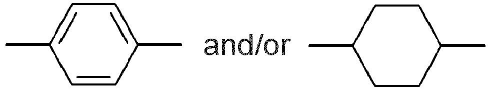
- x: is identical or different and is represented by an integer from 1 to 6,
- X and X₁: are each identical or different and, independently of one another, are each represented by a halogen atom, C₁ to C₄ alkyl sulfate and/or C₁ to C₄- alkylsulfonate.

In at least one embodiment, the one or more cationic structural units (A) of the copolymer represent the polymerization product of at least one monomer species selected from the group consisting of [2-(acryloyloxy)ethyl]trimethylammonium chloride, [2-(acryloylamino)ethyl]trimethylammonium chloride, [2-(acryloyloxy)ethyl]trimethylammonium methosulfate, [2-(methacryloyloxy)ethyl]trimethylammonium chloride and methosulfate, [3-(acryloylamino)propyl]trimethylammonium chloride, [3-(methacryloylamino)-propyl]trimethylammonium chloride and diallyldimethylammonium chloride (DADMAC), preferably selected from the group consisting of [3-(acryloylamino)-propyl]trimethylammonium chloride (AAPTAC), [3-(methacryloylamino)propyl]trimethylammonium chloride (MAPTAC), and diallyldimethylammonium chloride (DADMAC), and more preferably selected from the group consisting of [3-(methacryloylamino)propyl]trimethylammonium chloride (MAPTAC) and diallyldimethylammonium chloride (DADMAC).

In at least one preferred embodiment, the one or more cationic structural units (A) of the copolymer represent the polymerization product of diallyldimethylammonium chloride (DADMAC).

In at least one embodiment, the copolymer comprises from 5.0 mol-% to 50 mol-%, more preferably from 7.5 mol-% to 35 mol-% of one or more cationic structural units (A). In at least one preferred embodiment, the copolymer comprises from 10.0 mol-% to 33.7 mol-%, more preferably from 11 mol-% to 30 mol-%, even more preferably from 12 mol-% to 15 mol-% of one or more cationic structural units (A). In an alternative embodiment, the copolymer comprises from 20.0 mol-% to 35.0 mol-%, more preferably from 22 mol-% to 30 mol-%, even more preferably from 24 mol-% to 28 mol-% of one or more cationic structural units (A).

In at least one embodiment, the copolymer comprises a plurality of different cationic structural units (A) represented by the following general formulae (I) and/or (II).

### Macromonomeric structural units (B)

The one or more macromonomeric structural units (B) are represented by the general formula (III): 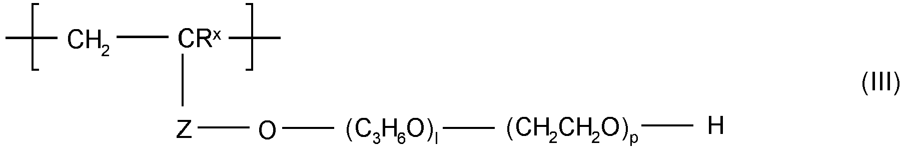 in which
- R^{x}: is identical or different and is represented by H and/or methyl,
- Z: is identical or different and is represented by C=O and/or O(CH₂)₄, preferably Z is O(CH₂)₄,
- I: is on molar average a number from 0 to 6, preferably from 0 to 5, and
- p: is, on molar average, a number from 1 to 150; preferably from 4 to 150; more preferably from 8 to 150.

In at least one embodiment, the one or more macromonomeric structural units (B) of the formula (III) of the copolymer represent the polymerization product of at least one monomer species selected from the group consisting of polyethylene glycol vinyloxybutyl ether, polyethylene glycol-co-polypropylene glycol vinyloxybutyl ether (with I on molar average a number ≥ 1), polyethylene glycol (meth)acrylate and polyethylene glycol-co-polypropylene glycol (meth)acrylate (with I on molar average a number ≥ 1).

In at least one embodiment, the one or more macromonomeric structural units (B) of the formula (III) of the copolymer represent the polymerization product of polyethylene glycol (meth)acrylate and polyethylene glycol-co-polypropylene glycol (meth)acrylate (with I, on molar average, a number ≥ 1).

In at least one embodiment, the one or more macromonomeric structural units (B) are represented by the general formula (III): 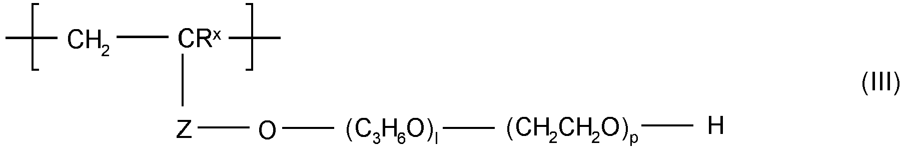 in which
- R^{x}: is identical or different and is represented by H and/or methyl,
- Z: is identical or different and is represented by C=O and/or O(CH₂)₄ and is preferably C=O,
- I: is on molar average a number from 0 to 6 and preferably from 0 to 5, more preferably 1 to 4, even more preferably 2 to 3 and
- p: is, on molar average, a number from 1 to 150, preferably from 4 to 150, and more preferably from 8 to 150, even more preferably from 10 to 25 or from 30 to 50 or from 100 to 130.

In at least one embodiment, the one or more macromonomeric structural units (B) are represented by the general formula (III): 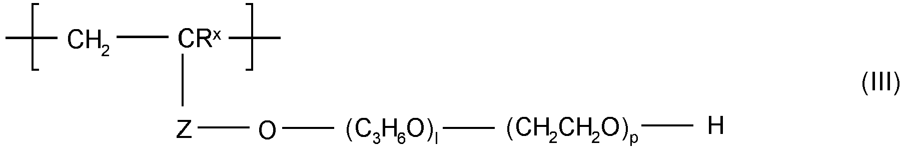 in which
- R^{x}: is identical or different and is represented by methyl,
- Z: is identical or different and is represented by C=O,
- I: is on molar average a number from 0 to 6 and preferably from 0 to 5, more preferably 1 to 4, even more preferably 2 to 3 and
- p: is, on molar average, a number from 1 to 150, preferably from 4 to 150, and more preferably from 8 to 150, even more preferably from 10 to 25 or from 30 to 50 or from 100 to 130.

In at least one embodiment, the copolymer copolymer comprises from 1.0 mol-% to 20.0 mol-% more preferably from 2.0 mol-% to 15.0 mol-%, even more preferably from 2.5 mol-% to 5.0 mol-% of one or more macromonomeric structural units (B). In at least one alternative embodiment, the copolymer copolymer comprises from 5.0 mol-% to 15.0 mol-% more preferably from 8.0 mol-% to 12.0 mol-% of one or more macromonomeric structural units (B). In at least one alternative embodiment, the copolymer copolymer comprises from 25.0 mol-% to 45.0 mol-% more preferably from 30.0 mol-% to 44.0 mol-%, even more preferably from 40.0 mol-% to 45.0 mol-% of one or more macromonomeric structural units (B).

### Structural units (C)

The copolymer comprises not only the structural units (A) and (B) but also one or more structural units (C) which differ from the structural units (A) and (B);
preferably wherein the copolymer comprises from 0.1 to 99.8 mol-% of the one or more structural units (A), from 0.1 to 99.8 mol-% of the one or more structural units (B), and from 0.1 to 99.8 mol-% of one or more structural units (C);
more preferably wherein the copolymer comprises from 20.0 to 80.0 mol-% of the one or more structural units (A), from 0.4 to 20.0 mol-% of the one or more structural units (B), and from 15.5 to 74.6 mol-% of one or more structural units (C);
even more preferably wherein the copolymer comprises from 22.0 to 77.6 mol-% of the one or more structural units (A), from 0.5 to 4.4 mol-% of the one or more structural units (B), and from 18.0 to 74.5 mol-% of one or more structural units (C).

In at least one embodiment, the one or more structural units (C) of the copolymer are selected from the group consisting of the polymerization product of at least one cyclic N-vinyl-substituted lactam having 5 to 7 ring atoms and structural units of the following general formulae (IV) and (V): 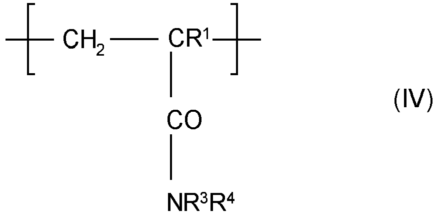 in which
- R¹: is identical or different and is hydrogen and/or methyl, and
- R³ and R⁴: are each identical or different and independently of one another are each represented by hydrogen, an aliphatic hydrocarbon radical having 1 to 20, preferably 1 to 4, carbon atoms, a cycloaliphatic hydrocarbon radical having 5 to 20, preferably 5 to 8, carbon atoms, an aryl radical having 6 to 14 Carbon atoms, an alkylaryl radical having 7 to 14 carbon atoms, a branched or unbranched C₁-C₅ monohydroxyalkyl group and/or polyethylene glycol (PEG) 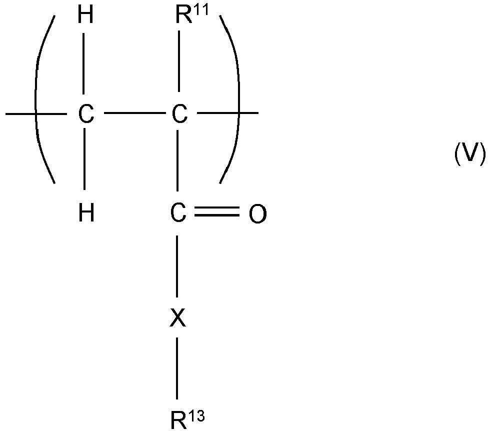
in which
- R¹¹: is identical or different and is represented by H and/or methyl;
- X: is identical or different and is represented by NH-(CₙH₂ₙ) where n = 1, 2, 3 or 4; and
- R¹³: is identical or different and is represented by OH, N(CH₃)₂, SO₃H, PO₃H₂, O-PO₃H₂ and/or para-substituted C₆H₄-SO₃H,
and where, among the structural units of the formula (V) in which R¹³ is N(CH₃)₂, preference is given to those structural units which represent the polymerization product of at least one monomer species selected from the group consisting of [3-(methacryloylamino)propyl]dimethylamine (R¹¹ = methyl; X = NH-(CₙH₂ₙ) where n = 3, and R¹³ = N(CH₃)₂) and [3-(acryloylamino)propyl]dimethylamine (R¹¹ = H; X = NH-(CₙH₂ₙ) where n = 3, and R¹³ = N(CH₃)₂).

In at least one embodiment, the one or more structural units (C) are selected from the general formula (IV).

In at least one embodiment, the one or more structural units (C) are selected from the general formula (V).

In at least one embodiment, the one or more structural units (C) represent the polymerization product of at least one monomer species selected from the group consisting of acrylamide, methacrylamide, N-methylacrylamide, N,N'-dimethylacrylamide, N-ethylacrylamide, N-cyclohexylacrylamide, N-benzylacrylamide, N-methylolacrylamide, N-isopropylacrylamide, and N-tert-butylacrylamide, and preferably selected from the group consisting of N,N'-dimethylacrylamide (DMAA) and N-isopropylacrylamide (NIPAM).

### Structural units (D)

In at least one embodiment, the copolymer comprises one or more structural units (D) which are represented with the general formula (VIII): 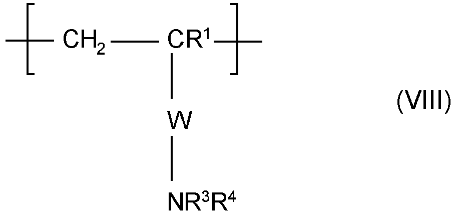 in which
- W: is identical or different and is represented by -CO-O-(CH₂)ₓ,
- x: is an integer from 1 to 6, preferably 2 or 3,
- R¹: is identical or different and is hydrogen and/or methyl, and
- R³ and R⁴: are each identical or different and independently of one another are each represented by hydrogen, an aliphatic hydrocarbon radical having 1 to 20, preferably 1 to 4, carbon atoms, a cycloaliphatic hydrocarbon radical having 5 to 20, preferably 5 to 8, carbon atoms, an aryl radical having 6 to 14 carbon atoms and/or polyethylene glycol (PEG).

In at least one embodiment, the copolymer comprises one or more structural units (D) of the formula (VIII) which represent the polymerization product of at least one monomer species selected from the group consisting of [2-(methacryloyloxy)ethyl]dimethylamine, [2-(acryloyloxy)ethyl]dimethylamine, [2-(methacryloyloxy)ethyl]diethylamine, and [2-(acryloyloxy)ethyl]-diethylamine.

In at least one embodiment, the copolymer comprises one or more structural units (D) of the following general formulae (IX) and/or (X): 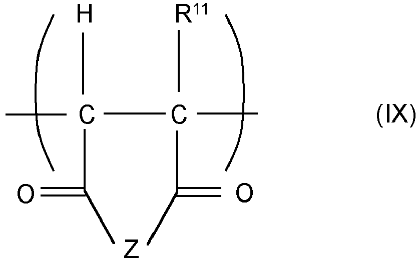 in which
- R¹¹: is identical or different and is represented by H and/or methyl;
- Z: is identical or different and is represented by O and/or NH; 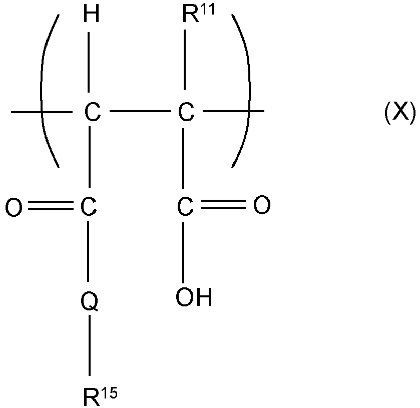
in which
- R¹¹: is identical or different and is represented by H and/or methyl;
- Q: is identical or different and is represented by O and/or NH; and
- R¹⁵: is identical or different and is represented by H, (CₙH₂ₙ)-SO₃H where n = 0, 1, 2, 3 or 4; (CₙH₂ₙ)-OH where n = 0, 1, 2, 3 or 4; (CₙH₂ₙ)-PO₃H₂ where n = 0, 1, 2, 3 or 4; (CₙH₂ₙ)-OPO₃H₂ where n = 0, 1, 2, 3 or 4; (C₆H₄)-SO₃H; (C₆H₄)-PO₃H₂; (C₆H₄)-OPO₃H₂ and/or (CₘH₂ₘ)ₑ-O-(A'O)ᵤ-R¹⁶ where m = 0, 1, 2, 3 or 4, e = 0, 1, 2, 3 or 4, A' = C_{x'}H_{2x'} where x' = 2, 3, 4 or 5, u = an
integer from 1 to 350, and R¹⁶ is identical or different and represented by an unbranched or branched C₁-C₄ alkyl group.

In at least one embodiment, the copolymer comprises one or more structural units (D) of the following general formula (VII): 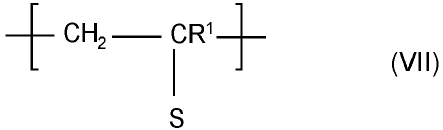 in which
- S: is identical or different and is represented by -COOMₖ,
- R¹: is identical or different and is represented by H and/or an unbranched or branched C₁-C₄ alkyl group, and is preferably represented by H or methyl; and
- M: is a cation selected from the group consisting of hydrogen ion, alkali metal ion, and alkaline earth metal ion, where k = valence.

In at least one embodiment, the one or more structural units (D) of the formula (VII) of the one or more copolymers of component Z1) represent the polymerization product of at least one monomer species selected from the group consisting of acrylic acid, sodium acrylate, methacrylic acid, and sodium methacrylate.

In at least one embodiment, the copolymer comprises one or more structural units (D) selected from the following general formulae (Va), (Vb), and (Vc): 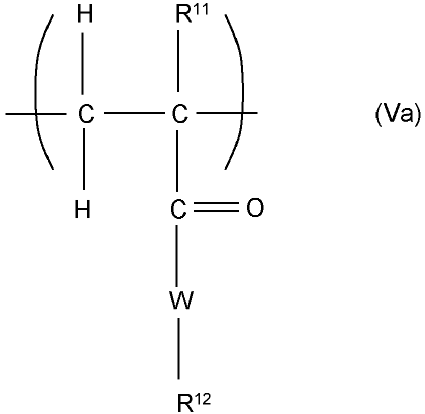 in which
- R¹¹: is identical or different and is represented by H and/or methyl;
- W: is identical or different and is represented by O;
- R¹²: is identical or different and is represented by a branched or unbranched C₁-C₅ monohydroxyalkyl group; 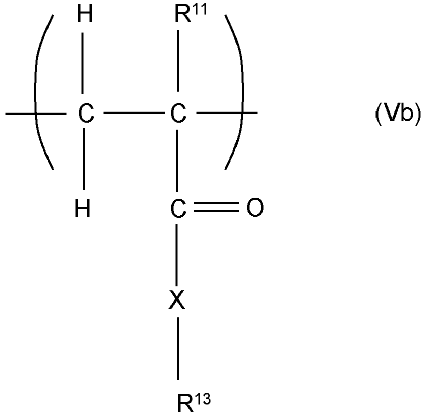
in which
- R¹¹: is identical or different and is represented by H and/or methyl;
- X: is identical or different and is represented by O-(CₙH₂ₙ) where n = 1, 2, 3 or 4;
- R¹³: is identical or different and is represented by OH, SO₃H, PO₃H₂, O-PO₃H₂, and/or para-substituted C₆H₄-SO₃H; 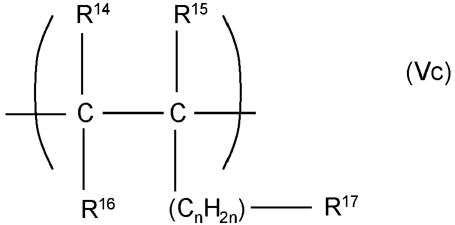
in which
- R¹⁴, R¹⁵ and R¹⁶: are each identical or different and each independently of one another are represented by H and/or an unbranched or branched C₁-C₄ alkyl group;
- n: is identical or different and is represented by 0, 1, 2, 3 and/or 4;
- R¹⁷: is identical or different and is represented by (C₆H₅), OH, OR^{y} where R^{y} is an alkyl group having 1 to 8, preferably 4 carbon atoms, and/or is -COCH₃.

In at least one embodiment, the copolymer comprises not only the structural units (A), (B) and (C) but also one or more structural units (D) which differ from the structural units (A), (B), and (C);
preferably wherein the copolymer comprises from 0.1 to 99.7 mol-% of the one or more structural units (A), from 0.1 to 99.7 mol-% of the one or more structural units (B), from 0.1 to 99.7 mol-% of the one or more structural units (C), and from 0.1 to 99.7 mol-% of the one or more structural units (D);
more preferably wherein the copolymer comprises from 20.0 to 80.0 mol-% of the one or more structural units (A), from 0.4 to 20.0 mol-% of the one or more structural units (B), from 15.5 to 74.6 mol-% of the one or more structural units (C), and from 0.1 to 24.6 mol-% of the one or more structural units (D);
even more preferably wherein the copolymer comprises from 22.0 to 77.6 mol-% of the one or more structural units (A), from 0.5 to 4.4 mol-% of the one or more structural units (B), from 18.0 to 72.0 mol-% of the one or more structural units (C) and from 0.5 to 24.6 mol-% of the one or more structural units (D).

### Cosmetically acceptable component (ii)

The cosmetic composition (or simply "composition" herein) comprises a cosmetically acceptable component (ii).

In at least one embodiment, the cosmetically acceptable component is selected from the group consisting of surfactants, surfactant systems, oily substances, waxes, emulsifiers, coemulsifiers, solubilizers, cationic polymers, film formers, superfatting agents, refatting agents, foam stabilizers, stabilizers, active biogenic substances, preservatives, preservation boosting ingredients, anti-fungal substance, anti-seborrheics and anti-dandruff agents, dyes or pigments, particulate substances, opacifiers, abrasives, absorbents, anticaking agents, bulking agents, pearlizing agents, direct dyes, perfumes or fragrances, carriers, solvents or diluents, propellants, functional acids, active ingredients, skin-brightening agents, self-tanning agents, exfoliants, enzymes, anti-acne agents, depilating agents, deodorants and anti-perspirants, viscosity modifiers, thickening and gelling agents, pH adjusting agents, buffering agents, anti-oxidants, chelants, astringents, sunscreens, sun protection agents, UV filters, skin conditioning agents, emollients, humectants, occlusive agents, pediculocides, anti-foaming agents, flavouring agents, electrolytes and combinations thereof. In at least one embodiment, the cosmetically acceptable component is selected from the group consisting of surfactants, salts, conditioning agents, actives, acidity regulators, antistatic agents, lubricants, moisturizers, oils, preservatives, sequestrants, strengtheners, sun protectors, solvents, or combinations thereof. In at least one embodiment, the cosmetically acceptable component is present in an amount of at least 0.5 % by weight, or from 0.5 to 20 %, by total weight of the composition. Preferably, the cosmetically acceptable component (ii) is selected from the group consisting of: cleansing ingredients, acidity regulators, colorants, further conditioning agents, emulsifiers, film formers, fragrances, glossers, humectants, lubricants, moisturizers, pigments, preservatives, hair penetration enhancers, scalp actives, stabilizers, further surfactants, thickeners, viscosity modifiers, and combinations thereof. More preferably, the cosmetically acceptable component is selected from the group consisting of surfactants, viscosity-modifying polymers and conditioning ingredients. In at least one embodiment, the composition comprises from 0.1 to 20 wt.-% of the copolymer described above and further comprises at least 0.5 wt.-% cosmetically acceptable component selected from the group consisting of surfactants, polymers, conditioning agents, actives, acidity regulators, lubricants, moisturisers, oils, preservatives, sequestrants, strengtheners, sun protectors, and combinations thereof.

In at least one embodiment, the composition comprises a further cosmetically acceptable component being cleansing ingredients. In at least one embodiment, the composition comprises from 0.05 wt.-% to 20 wt.-% cleansing ingredients. In at least one embodiment of the composition, the level of cleansing ingredient is from 1 wt.-% to 50 wt.-% by total weight of the composition, preferably 5 % to 40 %, more preferably 8 wt.-% to 20 wt.-%. In at least one embodiment, the cleansing ingredient is selected from the group consisting of non-polymeric surfactants, saponins, polymeric surfactants, and combinations thereof. In at least one embodiment, the composition comprises a cleansing ingredient, preferably anionic surfactant, as cosmetically acceptable component (ii). Preferably the cleansing ingredient comprises or consists of surfactants.

In at least one embodiment the composition, preferably shampoo composition, preferably comprises from 0.01 to 20 % by weight of one or more copolymers as described above and at least 0.5 wt.-% by weight of further surfactant(s), preferably cleansing anionic or nonionic surfactants, such as sodium laureth sulphate, sodium lauryl sulphate, ammonium laureth sulphate, ammonium lauryl sulphate, olefin sulfonates, olefin sulfates, laureth-3 or 4, cocamide DEA, glucosides, cocamidopropyl betaine, coco betaine, cocoamphodipropionate, sodium methyl 2-sulfolaurate and other laurates, sulfoacetates, sulfosuccinates, lactylates, sultaines, caprylates/ caprates, isethionates, glutamates, taurates, sarcosinates, glucamides, and combinations thereof.

In at least one embodiment, the composition comprises a surfactant system. In at least one embodiment, the surfactant system comprises a surfactant selected from the group consisting of anionic surfactants, cationic surfactants, non-ionic surfactants, zwitterionic surfactants and/or amphoteric surfactants.

In at least one embodiment, the composition comprises a total amount of surfactant of from 0.01 wt.-% to 70 wt.-%, from 0.1 wt.-% to 40 %, from 1 wt.-% to 30 %, from 2 wt.-% to 20 wt.-%.

In at least one embodiment, the composition comprises an anionic surfactant. In at least one embodiment, the composition comprises an anionic surfactant as cosmetically acceptable component (ii). In at least one embodiment, the anionic surfactant is selected from the group consisting of (C₁₀-C₂₀)-alkyl and alkylene carboxylates, alkyl ether carboxylates, fatty alcohol sulfates, fatty alcohol ether sulfates, alkylamide sulfates and sulfonates, fatty acid alkylamide polyglycol ether sulfates, alkanesulfonates and hydroxyalkanesulfonates, olefinsulfonates, acyl esters of isethionates, α-sulfo fatty acid esters, alkylbenzenesulfonates, alkylphenol glycol ether sulfonates, sulfosuccinates, sulfosuccinic monoesters and diesters, fatty alcohol ether phosphates, protein/fatty acid condensation products, alkyl monoglyceride sulfates and sulfonates, alkylglyceride ether sulfonates, fatty acid methyltaurides, fatty acid sarcosinates, sulforicinoleates, acylglutamates, and mixtures thereof. The anionic surfactants (and their mixtures) can be used in the form of their water-soluble or water-dispersible salts, examples being the sodium, potassium, magnesium, ammonium, mono-, di-, and triethanolammonium, and analogous alkylammonium salts. In at least one embodiment, the anionic surfactant is the salt of an anionic surfactant comprising 12 to 14 carbon atoms. In at least one embodiment, the anionic surfactant is selected from the group consisting of sodium lauryl sulfate, sodium laureth sulfate, sodium tridecyl sulfate, sodium trideceth sulfate, sodium myristyl sulfate, sodium myreth sulfate, and mixtures thereof. Typical anionic surfactants for use in compositions of the invention include sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecyl benzene sulphonate, triethanolamine dodecylbenzene sulphonate, sodium cocoyl isethionate, sodium lauryl isethionate, lauryl ether carboxylic acid and sodium N-lauryl sarcosinate. Preferred anionic surfactants are selected from sodium lauryl sulphate and sodium lauryl ether sulphate(n)EO, (where n is from 1 to 3); more preferably sodium lauryl ether sulphate(n)EO, (where n is from 1 to 3); most preferably sodium lauryl ether sulphate(n)EO where n = 1. Preferably the level of alkyl ether sulphate is from 0.5 wt.-% to 25 wt.-% of the total cosmetic composition, more preferably from 3 wt.-% to 18 wt.-%, most preferably from 6 wt.-% to 15 wt.-% of the total composition.

The total amount of anionic surfactant in the composition may range from 0.5 wt.-% to 45 wt.-%, more preferably from 1.5 wt.-% to 20 wt.-%. Compositions of the invention may comprise fatty acyl isethionate, if present preferably at a level of from 1 to 10 wt.-%, more preferably from 2 to 8 wt.-%, most preferably from 2.5 to 7.5 wt.-%. A preferred fatty acyl isethionate product comprises fatty acyl isethionate surfactant at a level of from 40 to 80 wt.-% of the product, as well as free fatty acid and/or fatty acid salt at a level of from 15 to 50 wt.-%.

Preferably, greater than 20 wt.-% and less than 45 wt.-%, more preferably greater than 25 wt.-% and less than 45 wt.-% of the fatty acyl isethionate are of chain length greater than or equal to C6; and greater than 50 wt.-%, preferably greater than 60 wt.-% of the free fatty acid/soap is of chain length C6 to C20.

In addition, the composition may contain isethionates salts which are present typically at levels less than 5 wt.-%, and traces (less than 2 wt.-%) of other impurities. Preferably, a mixture of aliphatic fatty acids is used for the preparation of commercial fatty acyl isethionates surfactants. The resulting fatty acyl isethionate surfactants (e.g., resulting from reaction of alkali metal isethionate and aliphatic fatty acid) preferably should have more than 20 wt.-%, preferably more than 25 wt.-%, but no more than 45 wt.-%, preferably 35 % (on basis of fatty acyl isethionates reaction product) of fatty acyl group with 16 or greater carbon atoms to provide both excellent lather and mildness of the resulting fatty acyl isethionate product. These longer chain fatty acyl isethionate surfactants and fatty acids, i.e. fatty acyl group and fatty acid with 16 or more carbons, can typically form insoluble surfactant/fatty acid crystals in water at ambient temperatures.

In at least one embodiment, the composition comprises an acylglycinate surfactant. In at least one embodiment, the acylglycinate surfactant conforms to the formula (Y): 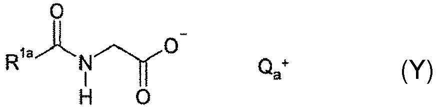 wherein
- R^{1a}: is a linear or branched, saturated alkanoyl group having 6 to 30, preferably 8 to 22, particularly preferably 8 to 18, carbon atoms or is a linear or branched, mono- or polyunsaturated alkenoyl group having 6 to 30, preferably 8 to 22 and particularly preferably 12 to 18 carbon atoms, and
- Qₐ⁺: is a cation.

In at least one embodiment, Qₐ⁺ is selected from the group consisting of Li⁺, Na⁺, K⁺, Mg⁺⁺, Ca⁺⁺, Al⁺⁺⁺, NH₄⁺, a monoalkylammmonium ion, a dialkylammonium ion, a trialkylammonium ion and a tetraalkylammonium ion, or combinations thereof. Optionally R^{1a} is independently from one another, are (C₁-C₂₂)-alkyl radicals or (C₂-C₁₀)-hydroxyalkyl radicals. In at least one embodiment, the acylglycinate surfactant is selected from sodium cocoylglycinate and potassium cocoylglycinate. In at least one embodiment, the acylglycinate surfactant is selected from those conforming to formula (Y), wherein R is C₁₂ alkyl or C₁₄ alkyl. In at least one embodiment, the acylglycinate surfactant is selected from those conforming to formula (Y), wherein R is C₁₆ alkyl or C₁₈ alkyl.

In at least one embodiment, the composition comprises from 0.01 wt.-% to 30 wt.-%, or 1 wt.-% to 25 wt.-%, preferably from 5 wt.-% to 20 wt.-%, more preferably from 12 wt.-% to 18 wt.-% acylglycinate surfactant.

In at least one embodiment, the composition comprises a glutamate surfactant corresponding to formula (Z) or a salt thereof: 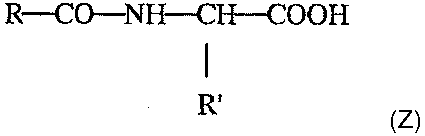 wherein
R' is HOOC-CH₂-CH₂- or M⁺⁻OOC-CH₂-CH₂- wherein M⁺ is a cation; and wherein R is a linear or branched, saturated alkanoyl group having 6 to 30, preferably 8 to 22, more preferably 8 to 18, carbon atoms or is a linear or branched, mono- or polyunsaturated alkenoyl group having 6 to 30, preferably 8 to 22 and more preferably 12 to 18 carbon atoms. In at least one embodiment, M⁺ is a metal cation. In at least one embodiment, M⁺ is selected from the group consisting of Li⁺, Na⁺, K⁺, Mg⁺⁺, Ca⁺⁺, Al⁺⁺⁺, NH₄⁺, a monoalkylammmonium ion, a dialkylammonium ion, a trialkylammonium ion and a tetraalkylammonium ion, or combinations thereof. In at least one embodiment, the glutamate surfactant is selected from sodium cocoyl glutamate and potassium cocoyl glutamate. In at least one embodiment, the glutamate surfactant is selected from those conforming to formula (Z), wherein R is C₁₂ alkyl or C₁₄ alkyl. In at least one embodiment, the glutamate surfactant is selected from those conforming to formula (Z), wherein R is C₁₆ alkyl or C₁₈ alkyl. In at least one embodiment, the composition comprises from 0.01 wt.-% to 30 wt.-%, or 1 wt.-% to 25 wt.-%, preferably from 5 wt.-% to 20 wt.-%, more preferably from 12 wt.-% to 18 wt.-% glutamate surfactant.

In at least one embodiment, the composition comprises a non-ionic surfactant. The non-ionic surfactants may be present in the range 0 to 5 wt.-%. Nonionic surfactants that can be included in compositions of the invention include condensation products of aliphatic primary or secondary linear or branched chain alcohols or phenols with alkylene oxides, usually ethylene oxide and generally having from 6 to 30 ethylene oxide groups. Alkyl ethoxylates are particularly preferred. Most preferred are alky ethoxylates having the formula

R-(OCH₂CH₂)ₙOH

where
R is an alkyl chain of C₁₂ to C₁₅, and n is 5 to 9. Other suitable nonionic surfactants include mono- or di-alkyl alkanolamides. Examples include coco mono- or di-ethanolamide and coco mono-isopropanolamide.

Further nonionic surfactants which can be included in compositions of the invention are the alkyl polyglycosides (APGs). Typically, APG is one which comprises an alkyl group connected (optionally via a bridging group) to a block of one or more glycosyl groups. Preferred APGs are defined by the following formula:

RO-(G)n

wherein
R is a branched or straight chain alkyl group which may be saturated or unsaturated and G is a saccharide group. R may represent a mean alkyl chain length of from about C₅ to about C₂₀. Preferably R represents a mean alkyl chain length of from about C₉ to about C₁₂. G may be selected from C₅ or C₆ monosaccharide residues, and is preferably a glucoside. G may be selected from the group comprising glucose, xylose, lactose, fructose, mannose and derivatives thereof. Preferably G is glucose. The degree of polymerisation, n, may have a value of from about 1 to about 10 or more. Most preferably the value of n lies from about 1.3 to about 1.5. Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in compositions of the invention include the fatty (e.g. C₁₀-C₁₈) N-alkyl (C₁-C₆) polyhydroxy fatty acid amides, such as the C₁₂-C₁₈ N-methyl glucamides, as described for example in WO9206154 and US5194639, and the N-alkoxy polyhydroxy fatty acid amides.

In at least one embodiment, the non-ionic surfactant has an HLB (Hydrophilic Lipophilic Balance) of greater than 12. Optionally, the non-ionic surfactant is selected from the group consisting of ethoxylated or ethoxylated/propoxylated fatty alcohols with a fatty chain comprising from 12 to 22 carbon atoms, ethoxylated sterols, such as stearyl- or lauryl alcohol (EO-7), PEG-16 soya sterol or PEG-10 soya sterol, polyoxyethylene polyoxypropylene block polymers (poloxamers), and mixtures thereof.

In at least one embodiment, the non-ionic surfactant is selected from the group consisting of ethoxylated fatty alcohols, fatty acids, fatty acid glycerides or alkylphenols, in particular addition products of from 2 to 30 mol of ethylene oxide and/or 1 to 5 mol of propylene oxide onto C₈- to C₂₂-fatty alcohols, onto C₁₂- to C₂₂-fatty acids or onto alkyl phenols having 8 to 15 carbon atoms in the alkyl group, C₁₂- to C₂₂-fatty acid mono- and diesters of addition products of from 1 to 30 mol of ethylene oxide onto glycerol, addition products of from 5 to 60 mol of ethylene oxide onto castor oil or onto hydrogenated castor oil, fatty acid sugar esters, in particular esters of sucrose and one or two C₈- to C₂₂-fatty acids, INCI: Sucrose Cocoate, Sucrose Dilaurate, Sucrose Distearate, Sucrose Laurate, Sucrose Myristate, Sucrose Oleate, Sucrose Palmitate, Sucrose Ricinoleate, Sucrose Stearate, esters of sorbitan and one, two or three C₈- to C₂₂-fatty acids and a degree of ethoxylation of from 4 to 20, polyglyceryl fatty acid esters, in particular of one, two or more C₈- to C₂₂-fatty acids and polyglycerol having preferably 2 to 20 glyceryl units, alkyl glucosides, alkyl oligoglucosides and alkyl polyglucosides having C₈ to C₂₂-alkyl groups, e.g. decylglucoside or laurylglucoside, and mixtures thereof.

In at least one embodiment, the non-ionic surfactant is selected from the group consisting of fatty alcohol ethoxylates (alkylpolyethylene glycols), alkylphenol polyethylene glycols, alkylmercaptan polyethylene glycols, fatty amine ethoxylates (alkylaminopolyethylene glycols), fatty acid ethoxylates (acylpolyethylene glycols), polypropylene glycol ethoxylates (Pluronics^{®}), fatty acid alkylol amides, (fatty acid amide polyethylene glycols), N-alkyl-, N-alkoxypolyhydroxy-fatty acid amide, sucrose esters, sorbitol esters, polyglycol ethers, and mixtures thereof.

In at least one embodiment, the composition comprises a fatty N-methyl-N-glucamide surfactant. In at least one embodiment, the fatty N-methyl-N-glucamide surfactant conforms to the formula (X): 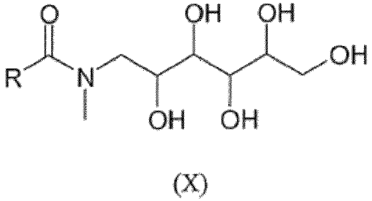 wherein
R is a a linear or branched alkyl or alkenyl group having from 3 to 30 carbon atoms. In at least one embodiment, R is an alkyl group having from 3 to 30 carbon atoms. In at least one embodiment, R is a saturated aliphatic hydrocarbon group which can be linear or branched and can have from 3 to 20 carbon atoms in the hydrocarbon chain, preferably linear or branched. Branched means that a lower alkyl group such as methyl, ethyl or propyl is present as substituent on a linear alkyl chain. In at least one embodiment, R is selected from the group consisting of 1-propyl, 2-propyl, 1-butyl, 2-butyl, 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl, 1-hexyl, 2-hexyl, 3-hexyl, 2-methyl-1-pentyl, 3-methyl-1-pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,2-dimethyl-1-butyl, 2,3-dimethyl-1-butyl, 3,3-dimethyl-1-butyl, 2-ethyl-1-butyl, 2,3-dimethyl-2-butyl, 3,3-dimethyl-2-butyl, 1-heptyl, 1-octyl, 1-nonyl, 1-decyl, 1-undecyl, 1-dodecyl, 1-tetradecyl, 1-hexadecyl and 1-octadecyl. Suitable fatty N-methyl-N-glucamide surfactants are described in WO2013/178700 and EP 0 550 637, which are incorporated herein by reference. In at least one embodiment, the N-methyl-N-glucamide surfactant is selected from those conforming to formula (X), wherein R is C₁₂ alkyl or C₁₄ alkyl. In at least one embodiment, the N-methyl-N-glucamide surfactant is selected from those conforming to formula (X), wherein R is C₁₆ alkyl or C₁₈ alkyl.

In at least one embodiment, the composition comprises from 1 wt.-% to 20 wt.-%, more preferably from 2 wt.-% to 10 wt.-%, even more preferably from 3 wt.-% to 7 wt.-% non-ionic surfactant.

In at least one embodiment, the composition comprises from 1 wt.-% to 20 wt.-%, more preferably from 2 wt.-% to 10 wt.-%, even more preferably from 3 wt.-% to 7 wt.-% fatty N-methyl-N-glucamide surfactant.

Amphoteric or zwitterionic surfactant can be included in the composition in an amount ranging from 0.5 wt.-% to about 8 wt.-%, preferably from 1 wt.-% to 4 wt.-% of the total composition.

In at least one embodiment, the amphoteric surfactants are selected from the group consisting of N-(C₁₂-C₁₈)-alkyl-β-aminopropionates and N-(C₁₂-C₁₈)-alkyl-β-iminodipropionates as alkali metal salts and mono-, di-, and trialkylammonium salts; N-acylaminoalkyl-N,N-dimethylacetobetaine, preferably N-(C₈-C₁₈)-acylaminopropyl-N,N-dimethylacetobetaine, (C₁₂-C₁₈)-alkyl-dimethyl-sulfopropylbetaine, amphosurfactants based on imidazoline (trade name: Miranol^{®}, Steinapon^{®}), preferably the sodium salt of 1-(β-carboxymethyloxyethyl)-1-(carboxymethyl)-2-laurylimidazolinium; amine oxide, e.g., (C₁₂-C₁₈)-alkyl-dimethyl-amine oxide, fatty acid amidoalkyldimethylamine oxide, and mixtures thereof.

In at least one embodiment, the composition comprises a betaine surfactant. Optionally, the betaine surfactant is selected from C₈- to C₁₈-alkylbetaines. In at least one embodiment, the betaine surfactant is selected from the group consisting of cocodimethylcarboxymethylbetaine, lauryldimethylcarboxymethylbetaine, lauryldimethylalphacarboxyethylbetaine, cetyldimethylcarboxymethylbetaine, oleyldimethylgammacarboxypropylbetaine and laurylbis(2-hydroxypropyl)alphacarboxyethylbetaine and combinations thereof. Optionally, the betaine surfactant is selected from C₈- to C₁₈-sulfobetaines. In at least one embodiment, the betaine surfactant is selected from the group consisting of cocodimethylsulfopropylbetaine, stearyldimethylsulfopropylbetaine, lauryldimethyl-sulfoethylbetaine, laurylbis(2-hydroxyethyl)sulfopropylbetaine, and combinations thereof. Optionally, the betaine surfactant is selected from carboxyl derivatives of imidazole, the C₈- to C₁₈-alkyldimethylammonium acetates, the C₈- to C₁₈-alkyldimethylcarbonylmethylammonium salts, and the C₈- to C₁₈-fatty acid alkylamidobetaines, and mixtures thereof. Optionally, the C₈- to C₁₈-fatty acid alkylamidobetaine is selected from coconut fatty acid amidopropylbetaine, N-coconut fatty acid amidoethyl-N-[2-(carboxymethoxy)ethyl]glycerol (CTFA name: Cocoamphocarboxyglycinate), and mixtures thereof. A particularly preferred amphoteric or zwitterionic surfactant is cocamidopropyl betaine. Mixtures of any of the foregoing amphoteric or zwitterionic surfactants may also be suitable. Preferred mixtures are those of cocamidopropyl betaine with further amphoteric or zwitterionic surfactants as described above. A preferred further amphoteric or zwitterionic surfactant is sodium cocoamphoacetate.

In at least one embodiment, the composition comprises from 0.5 wt.-% to 20 wt.-%, preferably from 1 wt.-% to 10 wt.-% amphoteric surfactant.

In at least one embodiment, the composition comprises a surfactant system. In at least one embodiment, the surfactant system comprises at least one surfactant selected from the group consisting of lauryl sulfate, laureth sulfate, cocoamido-propylbetaine, sodium cocoylglutamate, lauroamphoacetate, and mixtures thereof. In at least one embodiment, the surfactant system comprises sodium laureth sulphate, sodium lauryl sulphate, and optionally cocamidopropyl betaine. In at least one embodiment, the surfactant system comprises sodium laureth sulphate, Potassium Cocyl Glutamate, and cocamidopropyl betaine.

Suitable lubricants are, for example, fatty alcohol components having 6 to 18 carbon atoms. The further surfactants may, for example, be chosen from non-polymeric, cationic quaternary ammonium compounds, in particular cetrimonium chloride (CTAC).

Suitable classical cationic conditioning agents include cationic quaternary ammonium salts. Examples of such quaternary ammonium salts include benzyl triethyl ammonium chloride, cetyl trimethylammonium chloride (cetrimonium chloride, CTAC), behentrimonium chloride (BTAC) and cetylpyridinium chloride.

As cationic components, a variety of further cationic polymers are suitable, including quaternized cellulose ethers, copolymers of vinylpyrrolidone, acrylic polymers, including homopolymers or copolymers of dimethyldiallylammonium chloride and acrylamide. Also suitable are various types of homo-or copolymers derived from acrylic or methacrylic acid, acrylamide, methylacrylamide, diacetoneacrylamide.

Typical glossers are silicones. Suitable as silicones are volatile or nonvolatile nonionic silicone fluids, silicone resins, and silicone semisolids or solids. Volatile silicones are linear or cyclic silicones having a measureable vapor pressure, which is defined as a vapor pressure of at least 2 mm of mercury at 20 °C. Also suitable are water insoluble nonvolatile silicone fluids including polyalkyl siloxanes, polyaryl siloxanes, polyalkylaryl siloxanes, polyether siloxane copolymers, amine-functional silicones, and mixtures thereof.

Preferably, the compositions of the present invention contains as a further component a silicone compound. The composition can comprise up to 5 % (e.g. 0.1 to 5 %) by weight of a silicone compound. Suitable silicone compounds include polyalkyl or polyaryl siloxanes. The preferred silicone compounds are polydimethylsiloxane, polydiethylsiloxane, and polymethylphenylsiloxane, e.g. available from Wacker (Germany) or Dow Corning, such as Xiameter PMX DC 200. Silicone compounds can be available as silicone oils or emulsions. The silicone compounds may further be incorporated in the present composition in the form of an emulsion, wherein the emulsion is pre-made and added to the formulation, or made during the formulation process by mechanical mixing with or without the aid of an additional surfactant selected from anionic surfactants, nonionic surfactants, cationic surfactants, and mixtures thereof.

The compositions of the invention contain silicone conditioning agents preferably these are emulsified droplets of a silicone conditioning agent, for enhancing conditioning performance.

Suitable silicones include polydiorganosiloxanes, in particular polydimethylsiloxanes which have the CTFA designation dimethicone. Also suitable for use compositions of the invention (particularly shampoos and conditioners) are polydimethyl siloxanes having hydroxyl end groups, which have the CTFA designation dimethiconol. Also suitable for use in compositions of the invention are silicone gums having a slight degree of cross-linking, as are described for example in WO9631188A1 (Unilever). The viscosity of the emulsified silicone itself (not the emulsion or the final hair conditioning composition) is typically at least 10,000 cSt at 25 °C the viscosity of the silicone itself is preferably at least 60,000 cSt, most preferably at least 500,000 cSt, ideally at least 1 ,000,000 cSt. Preferably the viscosity does not exceed 109 cSt for ease of formulation. Emulsified silicones for use in the shampoo compositions of the invention will typically have an average silicone droplet size in the composition of less than 30, preferably less than 20, more preferably less than 10 micron, ideally from 0.01 to 1 micron. Silicone emulsions having an average silicone droplet size of less than 0.15 micron are generally termed microemulsions.

Silicone particle size may be measured by means of a laser light scattering technique, for example using a 2600D Particle Sizer from Malvern Instruments. Examples of suitable pre-formed emulsions include Xiameter MEM 1785 and microemulsion DC2-1865 available from Dow Corning. These are emulsions / microemulsions of dimethiconol. Cross-linked silicone gums are also available in a pre-emulsified form, which is advantageous for ease of formulation. A further preferred class of silicones for inclusion in shampoos and conditioners of the invention are amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone".

Specific examples of amino functional silicones suitable for use in the invention are the aminosilicone oils DC2-8220, DC2-8166 and DC2-8566 (all ex Dow Corning).

Suitable quaternary silicone polymers are described in EP-A-0 530 974. A preferred quaternary silicone polymer is K3474, ex Goldschmidt.

Also suitable are emulsions of amino functional silicone oils with non-ionic and/or cationic surfactant. Pre-formed emulsions of amino functional silicone are also available from suppliers of silicone oils such as Dow Corning and General Electric. Specific examples include DC939 Cationic Emulsion and the non-ionic emulsions DC2-7224, DC2- 8467, DC2-8177 and DC2-8154 (all ex Dow Corning). A combination of amino and non amino functional silicones may also be used.

The total amount of silicone is preferably from 0.01 wt.-% to 10 wt.-% of the total composition, more preferably from 0.1 wt.-% to 5 wt.-%, most preferably 0.5 wt.-% to 3 wt.-%.

Typical oils to be used in hair compositions are organic oils, which often are esters. The organic oil component may also comprise glyceryl esters of fatty acids, or triglycerides, coconut oil, almond oil, apricot kernel oil, avocado oil, babassu oil, evening primrose oil, camelina sativa seed oil, grape seed oil, macadamia ternifolia seed oil, corn oil, meadowfoam seed oil, mink oil, olive oil, palm kernel oil, safflower oil, sesame oil, soybean oil, sunflower oil, wheat germ oil, and camellia reticulata seed oil. Also suitable as the oil component are sorbitan esters and glyceryl esters as described above. The cosmetic composition of the invention may contain from 0.05 to 5 %, preferably 0.5 to 5 % by weight of at least one oil component.

The composition of the invention can contain from 0.1 to 10 % by weight, preferably from 0.2 to 5 % by weight, more preferably from 0.2 to 3 % by weight of at least one rheology modifying agent, in particular a gelling and thickening agent. Examples are cellulosic thickeners, for example, hydroxyethyl-cellulose, hydroxypropylcellulose, and carboxymethylcellulose, guar gum, such as hydroxypropylguar, gums of microbial origin, such as xanthan gum and scleroglucan gum, and synthetic thickeners, such as crosslinked homo- or copolymers of acrylic acid and/or of acrylamidopropanesulphonic acid. Other rheology modifying agents include fatty acid amides such as coconut diethanolamide and monoethanolamide, and oxyethylenated monoethanolamide of carboxylic acid alkyl ether.

Rheology modifying agents are also known as structuring materials. Common structuring materials include polymeric materials known as "carbomers", including, for example the cross-linked polyacrylic acid polymers available from Lubrizol Corporation under the trademark Carbopol^{®}. Another class of (meth)acylic acid polymers are alkali-swellable emulsion (ASE) polymers. ASE polymers include, for example, Aculyn^{®} 38 copolymer from Dow. Carbomers and ASE polymers belong to a class of materials known as hydrodynamic thickeners. These hydrodynamic thickeners include acid groups in their polymeric structure that, when deprotonated, form anionic charges that repel each other, causing the polymer chains to expand and entangle. Expansion and chain entanglement can give rise to thickening and suspending effects provided by the deprotonated polymers. The properties of these hydrodynamic thickeners are impacted by their molecular weight, acid group content, degree of cross-linking, and extent of swelling. These thickeners are also known as "space filling" or "volume excluding", and tend to increase both viscosity and yield point as the concentration thereof is increased. In use, hydrodyamic polymers commonly give rise to compositions that exhibit shear thinning or non-Newtonian behavior. Another class of (meth)acrylic acid based rheology modifiers are hydrophobically modified alkali swellable (HASE) polymers. Like ASE polymers, the HASE polymers include acid groups, the deprotonation of which gives rise to polymer swelling. Additionally, the HASE polymers include hydrophobic side groups, chains or blocks that give rise to associative interactions with each other, as well as with other hydrophobic species present in the compositions in which they are employed, for example, hydrophobic groups of surfactants, fatty acids, other thickening agents, and the like. Association creates hydrophobic regions distributed throughout the polymer chain network. This can also help to enhance the properties of the materials as solubilizing agents. Aculyn^{®} 22 and Aculyn^{®} 28 copolymers from Dow and Aqua SF-1^{®} copolymer from Lubrizol Corporation are among the commonly used HASE materials. U.S. Patent 4,529,773 (Witiak et al.) reports alkali-soluble emulsion polymers activated by neutralization to a pH above 6.5, and subsequently acidified in the presence of a surfactant. These are described as useful thickeners in acidic compositions. The polymers can be formed from the copolymerization of a monomer system that includes: (1) methacrylic or acrylic acid, (2) methacrylic or acrylic acid ester of a C₈-C₃₀ alkyl or, as therein more particularly described, a hydrocarbyl monoether of polyethylene glycol, (3) a C₁-C₄ alkyl acrylate or methacrylate, and, optionally, (4) a small amount of a polyethylenically unsaturated monomer.

Generally, the composition according to the present invention can be used for topical application to hair, but also skin or nail treatment is possible. The composition is also suitable for cleansing animal hair, preferably mammalian hair.

The composition of the present invention can also comprise as cosmetically acceptable component (ii) a fatty compound. The fatty compound is included in the composition at a level of from 0.1 to 20 % by weight, preferably from 1.0 to10 % by weight. The fatty compound is selected from the group consisting of fatty alcohols (e.g. cetyl alkohol, stearyl alcohol or cetearyl alcohol), fatty acids, fatty alcohol derivatives, fatty acid derivatives, and mixtures thereof.

It is understood that the components disclosed can in some instances fall into more than one classification, e.g., some fatty alcohol derivatives can also be classified as fatty acid derivatives. However, a given classification, is not intended to be a limitation on that particular compound, but is done so for convenience of classification and nomenclature. Non-limiting examples are found in International Cosmetic Ingredient Dictionary and Handbook, Fourteenth Edition (2014), and CTFA Cosmetic Ingredient Handbook, Second Edition, 1992. Preferably, fatty alcohols have 14 to 30 or 16 to 22 carbon atoms. These fatty alcohols are saturated and can be linear or branched. Examples of fatty alcohols are cetyl alcohol, stearyl alcohol, behenyl alcohol, and mixtures thereof. Preferred fatty acids have from 10 to 30 or from 12 to 22 carbon atoms. These fatty acids can be saturated and can be linear or branched. Also included herein are salts of these fatty acids. Examples of fatty acids are lauric acid, palmitic acid, stearic acid, behenic acid, sebacic acid, and mixtures thereof.

In at least one embodiment, the composition comprises fatty alcohol derivatives and/or fatty acid derivatives. The fatty alcohol derivatives and fatty acid derivatives useful herein include alkyl ethers of fatty alcohols, alkoxylated fatty alcohols, alkyl ethers of alkoxylated fatty alcohols, esters of fatty alcohols, fatty acid esters of compounds having esterifiable hydroxy groups, hydroxy-substituted fatty acids, and mixtures thereof. Examples of fatty alcohol derivatives and fatty acid derivatives include methyl stearyl ether; polyoxyethylene ethers of behenyl alcohol; ethyl stearate, cetyl stearate, cetyl palmitate, stearyl stearate, myristyl myristate, polyoxyethylene cetyl ether stearate, polyoxyethylene stearyl ether stearate, polyoxyethylene lauryl ether stearate, ethyleneglycol monostearate, polyoxyethylene monostearate, polyoxyethylene distearate, propyleneglycol monostearate, propyleneglycol distearate, trimethylolpropane distearate, sorbitan stearate, poly glyceryl stearate, glyceryl monostearate, glyceryl distearate, glyceryl tristearate, and mixtures thereof.

Compositions according to the present invention may also comprise a dispersed, non-volatile, water-insoluble oily conditioning agent. By "insoluble" is meant that the material is not soluble in water (distilled or equivalent) at a concentration of 0.1 % (w/w), at 25 °C.

In at least one embodiment, the composition comprises an oily or fatty material. Suitable oily or fatty materials are selected from hydrocarbon oils, fatty esters and mixtures thereof. Straight chain hydrocarbon oils will preferably contain from about 12 to about 30 carbon atoms. Also suitable are polymeric hydrocarbons of alkenyl monomers, such as C₂-C₆ alkenyl monomers. Specific examples of suitable hydrocarbon oils include: paraffin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used.

Suitable fatty esters are characterised by having at least 10 carbon atoms, and include esters with hydrocarbyl chains derived from fatty acids or alcohols, monocarboxylic acid esters include esters of alcohols and/or acids of the formula R'COOR in which R' and R independently denote alkyl or alkenyl radicals and the sum of carbon atoms in R' and R is at least 10, preferably at least 20. Di- and trialkyl and alkenyl esters of carboxylic acids can also be used. Particularly preferred fatty esters are mono-, di- and triglycerides, more specifically the mono-, di-, and tri-esters of glycerol and long chain carboxylic acids such as C₁-C₂₂ carboxylic acids. Preferred materials include cocoa butter, palm stearin, sunflower oil, soyabean oil and coconut oil.

In at least one embodiment, the composition comprises an oily or fatty material. The oily or fatty material is suitably present at a level of from 0.05 to 10, preferably from 0.2 to 5, more preferably from about 0.5 to 3 wt.-%.

The compositions of the present invention may comprise an aqueous carrier. The level and species of the aqueous carrier are selected according to the compatibility with other components and other desired characteristic of the composition. The carrier may include water and water solutions of lower alkyl alcohols and polyhydric alcohols. The lower alkyl alcohols useful herein are monohydric alcohols having 1 to 6 carbons, often ethanol and/or isopropanol. The useful polyhydric alcohols include propylene glycol, hexylene glycol, glycerin, and propane diol. Preferably, the aqueous carrier is substantially water. Deionized water is preferably used. Water from natural sources, including minerals can also be used, depending on the desired characteristic of the cosmetic composition. Generally, the cosmetic compositions of the present invention can comprise up to 80 %, often even up to 95 % by weight of water.

The compositions of the present invention may also include other components being suitable for rendering the composition more cosmetically or aesthetically acceptable or to provide them with additional usage benefits. Such other components can generally be used individually at levels of from 0.001 % to 5 % by weight. A wide variety of further components can be formulated into the present composition. These include other conditioning agents, such as hydrolysed collagen, vitamin E, or panthenol, panthenyl ethyl ether, hydrolysed keratin, proteins, plant extracts, nutrients; and emollients such as PPG-3 myristyl ether, trimethyl pentanol hydroxyethyl ether; hair-fixative polymers such as amphoteric fixative polymers, cationic fixative polymers, anionic fixative polymers, nonionic fixative polymers, and silicone grafted copolymers; preservatives such as benzyl alcohol, methyl paraben, propyl paraben and imidazolidinyl urea; pH adjusting agents, such as citric acid, sodium citrate, succinic acid, phosphoric acid, sodium hydroxide, sodium carbonate; salts, in general, such as potassium acetate and sodium chloride; coloring agents; hair oxidizing (bleaching) agents, such as hydrogen peroxide, perborate and persulfate salts; hair reducing agents such as the thioglycolates; perfumes; and sequestering agents, such as disodium ethylene-diamine tetraacetate; ultraviolet and infrared screening and absorbing agents such as octyl salicylate; and anti-dandruff agents such as zinc pyrithione and salicylic acid.

In at least one embodiment, the composition comprises a solvent, preferably wherein the solvent comprises water and/or alcohol. Solvent is useful for providing the compounds used in present invention in liquid form. In at least one embodiment, the solvent is cosmetically acceptable. In at least one embodiment, the composition comprises at least 10 wt.-% water. Water is useful for economic reasons but also because it is cosmetically acceptable. In a preferred embodiment, the composition comprises an aqueous, alcoholic or aqueous-alcoholic solvent, and wherein the aqueous, alcoholic or aqueous-alcoholic solvent comprises water, ethanol, propanol, isopropanol, 1,2-propylene glycol, 1,3-propylene glycol, isobutanol, butanol, butyl glycol, butyl diglycol, glycerol, or a mixture thereof; preferably wherein the aqueous, alcoholic or aqueous-alcoholic solvent comprises water, ethanol, propanol, isopropanol, 1,2-propylene glycol, 1,3-propylene glycol, glycerol, or mixtures thereof; more preferably wherein the aqueous, alcoholic or aqueous-alcoholic solvent comprises water, isopropanol, 1,2-propylene glycol, 1,3-propylene glycol, or mixtures thereof; even more preferably wherein the aqueous, alcoholic or aqueous-alcoholic solvent consists of water or consists of a mixture of water and an alcohol wherein the alcohol is selected from the group consisting of isopropanol, 1,2-propylene glycol and 1,3-propylene glycol. Natural solvents can also be used. In at least one embodiment, the composition comprises a solvent selected from the group consisting of plant oil, honey, plant-derived sugar compositions, and mixtures thereof. In at least one embodiment, the composition comprises from 0.5 wt.-% to 90 wt.-%, preferably from 1.0 wt.-% to 80 wt.-%, even more preferably from 5.0 wt.-% to 70 wt.-% of at least one carrier, solvent and/ or diluent.

In at least one embodiment, the composition comprises an anti-fungal substance. In at least one embodiment, the anti-fungal substance is selected from the group consisting of ketoconazole, oxiconazole, bifonazole, butoconazole, cloconazole, clotrimazole, econazole, enilconazole, fenticonazole, isoconazole, miconazole, sulconazole, tioconazole, fluconazole, itraconazole, terconazole, naftifine and terbinafine, zinc pyrithione, octopirox, and combinations thereof. In at least one embodiment, the composition comprises a total amount of anti-fungal substance in the composition of from 0.1 wt.-% to 1 wt.-%. In at least one embodiment, the composition comprises a pyridinethione anti-dandruff particulates, for example 1-hydroxy-2-pyridinethione salts, are highly preferred particulate anti-dandruff agents. The concentration of pyridinethione antidandruff particulate may ranges from 0.1 % to 4 %, by weight of the formulation, preferably from 0.1 % to 3 %, more preferably from 0.3 % to 2 %. Preferred pyridinethione salts include those formed from heavy metals such as zinc, tin, cadmium, magnesium, aluminum and zirconium, preferably zinc, more preferably the zinc salt of 1-hydroxy-2-pyridinethione (known as "zinc pyridinethione" or "ZPT"), more preferably 1-hydroxy-2-pyridinethione salts in platelet particle form. Salts formed from other cations, such as sodium, may also be suitable. Pyridinethione anti-dandruff agents are described, for example, in US 2,809,971; US 3,236,733; US 3,753,196; US 3,761,418; US 4,345,080; US 4,323,683; US 4,379,753; and US 4,470,982, which are incorporated herein by reference. It is contemplated that when ZPT is used as the anti-dandruff particulate in the compositions herein, that the growth or regrowth of hair may be stimulated or regulated, or both, or that hair loss may be reduced or inhibited, or that hair may appear thicker or fuller.

Preferably salt is present at levels from 0.1 to 1 wt.-% of the total composition to adjust the product viscosity. Preferably NaOH is present at levels from 0.1 to 1 wt.-% of the total composition to adjust the pH of the formulation.

The shampoo composition of the present invention may contain as a further component (F) a polysorbate for adjusting rheology, for example, polysorbate-20, polysorbate-21, polysorbate-40, polysorbate-60, and mixtures thereof. The polysorbate can be contained in the composition in amounts up to 5 % (e.g. 0.1 to 5 %) by weight.

The composition can also contain a polypropylene glycol. Polypropylene glycols are those having a weight average molecular weight of from 200 to 100000 g/mol. The polypropylene glycol useful herein may be either water-soluble, water-insoluble, or may have a limited solubility in water, depending upon the degree of polymerization and whether other moieties are attached thereto. The desired solubility of the polypropylene glycol in water will depend in large part upon the form of the composition. The polypropylene glycol can be included in the composition of the present invention at a level of up to 10 % by weight.

The composition can also contain, as a further component, low melting point oil selected from the group consisting of hydrocarbons having from 10 to 40 carbon atoms; unsaturated fatty alcohols having from 10 to 30 carbon atoms such as oleyl alcohol; unsaturated fatty acids having from about 10 to about 30 carbon atoms; fatty acid derivatives; fatty alcohol derivatives; ester oils such as pentaerythritol ester oils, trirnethylol ester oils, citrate ester oils, and glyceryl ester oils; poly [alpha]-olefin oils; and mixitures thereof. Preferred low melting point oils herein are selected from the group consisting of: ester oils such as pentaerythritol ester oils, trimethylol ester oils, citrate ester oils, and glyceryl ester oils; poly [alpha]-olefin oils; and mixtures thereof. Particularly useful pentaerythritol ester oils and trimethylol ester oils herein include pentaerythritol tetraisostearate, pentaerythritol tetraoleate, trimethylolpropane triisostearate, trimethylolpropane trioleate, and mixtures thereof. Particularly useful glyceryl ester include triisostearin, triolein and trilinolein.

The cosmetic composition can also contain, as a further component, a cationic polymer. Cationic polymers may be present in the composition of the invention for further enhancing deposition performance.

Suitable cationic polymers may be homopolymers which are cationically substituted or may be formed from two or more types of monomers. The weight average (M_{w}) molecular weight of the polymers will generally be between 100 000 and 2 million g/mol. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C₁-C₇ alkyl groups, more preferably C₁-C₃ alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerized in the amine form and then converted to ammonium by quaternization.

The cationic polymers can comprise mixtures of monomer units derived from amine- and/or quaternary ammonium-substituted monomer and/or compatible spacer monomers.

Suitable cationic polymers include, for exampl cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively; mineral acid salts of aminoalkyl esters of homo-and co-polymers of unsaturated carboxylic acids having from 3 to 5 carbon atoms, (as described in US 4,009,256); cationic polyacrylamides (as described in WO95/2231 1 ).

Other cationic polymers that can be used include cationic polysaccharide polymers, such as cationic cellulose derivatives, cationic starch derivatives, and cationic guar gum derivatives.

Cationic polysaccharide polymers suitable for use in compositions of the invention include monomers of the formula:

A-O-[R-N⁺(R¹ )(R²)(R³)X"]

wherein
- A: is an anhydroglucose residual group, such as a starch or cellulose anhydroglucose residual,
- R: is an alkylene, oxyalkylene, polyoxyalkylene, or hydroxyalkylene group, or combination thereof,
- R¹, R² and R³: independently represent alkyl, aryl, alkylaryl, arylalkyi, alkoxyalkyi, or alkoxyaryl groups, each group containing up to about 18 carbon atoms.

The total number of carbon atoms for each cationic moiety (i.e., the sum of carbon atoms in R¹, R² and R³) is preferably about 20 or less, and X is an anionic counterion. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Amerchol Corporation, for instance under the tradename Polymer LM-200. Other suitable cationic polysaccharide polymers include quaternary nitrogen- containing cellulose ethers (e.g. as described in US 3,962,418), and copolymers of etherified cellulose and starch (e.g. as described in US 3,958,581).

A particularly suitable type of cationic polysaccharide polymer that can be used is a cationic guar gum derivative, such as guar hydroxypropyltrimethylammonium chloride (commercially available from Rhodia in their JAGUAR trademark series). Examples of such materials are JAGUAR C13S, JAGUAR C14, JAGUAR C15, JAGUAR C17 and JAGUAR C16 Jaguar CHT and JAGUAR C162.

Mixtures of any of the above cationic polymers may be used.

Cationic polymer may be present in the composition of the invention at levels of from 0.01 to 5 wt.-%, preferably from 0.05 to 1 wt.-%, more preferably from 0.08 to 0.5 % cationic polymer, based on the total weight of the composition.

In at least one embodiment, the cationic polymers have a number average molecular weight of at least about 5000, typically from 10000 to 10 million (g/mol) and are selected from the group consisting of: copolymers of vinyl monomers having cationic amine or quaternary ammonium functionalities with water soluble spacer monomers such as acrylamide, methacrylamide, alkyl and dialkyl acrylamides, alkyl and dialkyl methacrylamides, alkyl acrylate, alkyl methacrylate, vinyl caprolactone, and vinyl pyrrolidone. Other suitable spacer monomers include vinyl esters, vinyl alcohol, maleic anhydride, propylene glycol, and ethylene glycol. Preferred cationic polymers are cationic celluloses, cationic starches, and cationic guar gums. Commercially available cationic guar polymers are e.g. Jaguar^{®} from Rhodia.

In at least one embodiment, the composition comprises a preservative or preservative system. Examples of suitable preservatives include benzyl alcohol, piroctone olamine, phenoxyethanol, parabens, pentanediol, benzoic acid/sodium benzoate, sorbic acid/potassium sorbate, and other organic acids used to provide antimicrobial protection. Preservation boosting ingredients include anisic acid, lactic acid, sorbitan caprylate, ethylhexylglycerin, caprylyl glycol, octanediol, and similar substances. In at least one embodiment, the composition comprises 0.01 to 5 wt.-%, particularly preferably from 0.05 wt.-% to 1 wt.-% of at least one preservative. Suitable preservatives are the substances listed in the International Cosmetic Ingredient Dictionary and Handbook, 9th Edition with the function "preservatives". In at least one embodiment, the preservative is selected from the group consisting of phenoxyethanol, benzyl paraben, butyl paraben, ethyl paraben, isobutyl paraben, isopropyl paraben, methyl paraben, propyl paraben, iodopropynyl butylcarbamate, methyldibromoglutaronitrile, DMDM hydantoin and combinations thereof. In at least one embodiment, the composition comprises a preservative selected from the group consisting of cetyltrimethyl ammoniumchloride, cetylpyridinium chloride, benzethonium chloride, diisobutylethoxyethyldimethyl benzylammoniumchloride, sodium N-lauryl sarcosinate, sodium-N-palmethylsarcosinate, Lauroylsarcosine, N-myristoylglycine, potassium-N-laurylsarcosine, trimethylammoniumchloride, sodium aluminium chlorohydroxylactate, triethylcitrate, tricetylmethylammoniumchloride, 2,4,4'-trichloro-2'-hydroxydiphenylether (Triclosan), phenoxyethanol, 1,5-pentandiol, 1,6-hexandiol, 3,4,4'-trichlorocarbanilide (Triclocarban), diaminoalkylamide, L-lysine hexadecylamide, heavy metal citrate salts, salicylate, piroctose, zinc salts, pyrithione and its heavy metal salts, zinc pyrithione, zinc phenol sulfate, farnesol, ketoconazol, oxiconazol, bifonazole, butoconazole, cloconazole, clotrimazole, econazole, enilconazole, fenticonazole, isoconazole, miconazole, sulconazole, tioconazole, fluconazole, itraconazole, terconazole, naftifine, terbinafine, selenium disulfide, Octopirox^{®}, methylchloroisothiazolinone, methylisothiazolinone, methyldibromo glutaronitrile, AgCl, chloroxylenol, sodium salts of diethylhexylsulfosuccinate, sodiumbenzoate, phenoxyethanol, benzylalkohol, phenoxyisopropanol, paraben, such as butyl-, ethyl-, methyl- und propylparaben, and their salts, pentandiol, 1,2-octanediol, ethylhexylglycerinw, benzylalcohol, sorbic acid, benzoic acid, lactic acid, imidazolidinyl urea, diazolidinyl urea, dimethylol dimethyl hydantoin (DMDMH), sodium salts of hydroxymethyl glycinate, hydroxyethylglycine of sorbic acid and combinations thereof. In at least one embodiment, the preservative is selected from the group consisting of phenoxyethanol, benzyl paraben, butyl paraben, ethyl paraben, isobutyl paraben, isopropyl paraben, methyl paraben, propyl paraben, iodopropynyl butylcarbamate, methyldibromoglutaronitrile, DMDM hydantoin and combinations thereof. In at least one embodiment, the composition is substantially free of parabens.

In at least one embodiment, the composition comprises a perfume or fragrance. In at least one embodiment, the composition comprises at least one perfume or fragrance. Individual fragrance compounds, e.g. the synthetic products of the type of esters, ethers, aldehydes, ketones, alcohols and hydrocarbons can be used as fragrance or perfume oils. Fragrance compounds of the ester type are e.g. benzyl acetate, phenoxyethyl isobutyrate, p-tert-butylcyclohexyl acetate, linalyl acetate, dimethylbenzylcarbinyl acetate, phenylethyl acetate, linalyl benzoate, benzyl formate, ethyl-methylphenyl glycinate, allylcyclohexyl propionate, styrallyl propionate and benzyl salicylate. The ethers include for example benzyl ethyl ether, the aldehydes include e.g. the linear alkanals with 8 to 18 carbon atoms, citral, citronellal, citronellyloxyacetaldehyde, cyclamen aldehyde, hydroxycitronellal, lilial and bourgeonal, the ketones include e.g. the ionones, alpha-isomethylionone and methyl-cedryl ketone, the alcohols include anethole, citronellol, eugenol, geraniol, linalol, phenylethyl alcohol and terpineol, and the hydrocarbons include mainly the terpenes and balsams. Preferably, mixtures of various fragrances are used, which together produce an attractive perfume note.

Perfume oils can also contain mixtures of natural odoriferous substances that can be obtained from vegetable or animal sources, e.g. pine oil, citrus oil, jasmine oil, lily oil, rose oil, or ylang-ylang oil. Essential oils of lower volatility, which are used mostly as flavor components, are also suitable as perfume oils, e.g. sage oil, chamomile oil, clove oil, melissa oil, mint oil, cinnamon leaf oil, linden flower oil, juniper berry oil, vetiver oil, olibanum oil, galbanum oil and labdanum oil. In at least one embodiment, the composition comprises from 0.01 wt.-% to 3.0 wt.-%, preferably from 0.05 wt.-% to 2.0 wt.-%, even more preferably from 0.1 wt.-% to 1.0 wt.-% of at least one perfume or fragrance.

The compositions of the present invention can be in the form of rinse-off products or 'dry shampoo' products, can be opaque or transparent, and can be formulated in a wide variety of product forms, including creams, gels, emulsions, mousses and sprays. In at least one embodiment, the composition is a hair cleansing composition. Preferably, the hair cleanser composition of the present invention is in the form of a rinse-off product.

In at least one embodiment, the composition is a shampoo for cleansing keratin fibres, preferably is a hair cleansing composition

In at least one embodiment, the shampoo composition is silicone-free.

In at least one embodiment, the shampoo composition is sulfate-free.

In at least one embodiment, the composition comprises a hair conditioning agent. In at least one embodiment, the hair conditioning agent is a water insoluble, water dispersible, non-volatile, liquid that forms emulsified, liquid particles. In at least one embodiment, the hair conditioning agent is a silicone (e.g., silicone oil, cationic silicone, silicone gum, high refractive silicone, and silicone resin), an organic conditioning oil (e.g., hydrocarbon oils, polyolefins, and fatty esters), or combinations thereof.

In at least one embodiment, the hair conditioning agent is a silicone, and wherein the composition comprises from 0.01 % to 10 %, or from 0.1 % to 5 % silicone hair conditioning agent, by total weight of the composition. Suitable silicone hair conditioning agents, and optional suspending agents for the silicone, are described in US 5,104,646. In at least one embodiment, the composition comprises a silicone gum selected from the group consisting of polydimethylsiloxane, (polydimethylsiloxane) (methylvinylsiloxane) copolymer, poly(dimethylsiloxane) (diphenylsiloxane) (methylvinylsiloxane) copolymer, and mixtures thereof.

In at least one embodiment, the composition comprises a terminal aminosilicone. "Terminal aminosilicone" as defined herein means silicone comprising one or more amino groups at one or both ends of the silicone backbone. In at least one embodiment, the composition is substantially free of any silicone compound comprising pendant amino groups. In an embodiment, the composition is substantially free of any silicone compound other than terminal aminosilicones. In at least one embodiment, the amino group at least one terminus of the silicone backbone of the terminal aminosilicone is selected from the group consisting of primary amines, secondary amines and tertiary amines. In at least one embodiment, the composition comprises a terminal aminosilicone conforming to Formula (S):

(R^{F})ₐG₃₋ₐ-Si-(-OSiG₂)ₙ-O-SiG₃₋ₐ(R^{F})ₐ (S)

wherein
- G: is hydrogen, phenyl, hydroxy, or C₁-C₈ alkyl, preferably methyl;
- a: is an integer having a value from 1 to 3, preferably 1; b is 0, 1 or 2, preferably 1; n is a number from 0 to 1,999;
- R^{F}: is a monovalent radical conforming to the general formula C_{q}H_{2q}L, wherein q is an integer having a value from 2 to 8 and L is selected from the following groups: -N(R^{T})CH₂-CH₂-N(R^{T})₂; -N(R^{T})₂; -N(R^{T})₃A⁻; -N(R^{T})CH₂-CH₂-NR^{T}H₂A⁻; wherein R^{T} is hydrogen, phenyl, benzyl, or a saturated hydrocarbon radical, preferably an alkyl radical from having from 1 to 20 carbon atoms; A⁻ is a halide ion.

In at least one embodiment, the terminal aminosilicone corresponding to Formula (S) has a = 1, q = 3, G = methyl, n is from 1000 to 2500, alternatively from 1500 to 1700; and L is -N(CH₃)₂. A suitable terminal aminosilicone corresponding to Formula (III) has a = O, G = methyl, n is from 100 to 1500, or from 200 to 800, and L is selected from the following groups: -N(R^{T})CH₂-CH₂-N(R^{T})₂; -N(R^{T})₂; -N(R^{T})₃A⁻; -N(R^{T})CH₂-CH₂-NR^{T}H₂A⁻; wherein R^{T} is hydrogen, phenyl, benzyl, or a saturated hydrocarbon radical, preferably an alkyl radical from having from 1 to 20 carbon atoms; A⁻ is a halide ion, alternatively L is -NH₂. In at least one embodiment, the terminal aminosilicone is selected from the group consisting of bis-aminomethyl dimethicone, bisaminoethyl dimethicone, bis-aminopropyl dimethicone, bis-aminobutyl dimethicone, and mixtures thereof. In an embodiment, the viscosity of the terminal aminosilicone is from 1,000 to 30,000 cPs, or from 5,000 to 20,000 cPs measured at 25 °C.

In at least one embodiment, the composition comprises from 0.1 % to 20 %, or from 0.5 % to 10 %, or from 1 % to 6 % terminal aminosilicone, by total weight of the composition.

In at least one embodiment, the composition comprises a high melting point fatty compound. The high melting point fatty compound has a melting point of 25 °C or higher. In at least one embodiment, the high melting point fatty compound is selected from the group consisting of a fatty alcohol, fatty acid, fatty alcohol derivative, fatty acid derivative, and mixtures thereof. Non-limiting examples of the high melting point compounds are found in International Cosmetic Ingredient Dictionary, Fifth Edition, 1993, and CTFA Cosmetic Ingredient Handbook, Second Edition, 1992. The composition may comprise from 0.1 % to 40 %, or from 1 % to 30 %, or from 1.5 % to 16 %, or from 1.5 % to 8 % of a high melting point fatty compound, by total weight of the composition. This is advantageous in view of providing improved conditioning benefits such as slippery feel during the application to wet hair, softness and moisturized feel on dry hair. In at least one embodiment, the fatty alcohol is selected from the group consisting of: cetyl alcohol, stearyl alcohol, behenyl alcohol, and mixtures thereof. In at least one embodiment, the composition comprises a linear fatty alcohol, wherein the linear fatty alcohol is also comprised in the lamellar gel matrix. The lamellar gel matrix is suitable for providing various conditioning benefits such as slippery feel during the application to wet hair and softness and moisturized feel on dry hair. The linear fatty alcohol may comprise from 8 to 24 carbon atoms. In an embodiment, the linear fatty alcohol is selected from the group consisting of: cetyl alcohol, stearyl alcohol, and mixtures thereof. In an embodiment, the weight ratio of total linear fatty alcohol to terminal aminosilicone is from 0.5:1 to 10:1, or from 1:1 to 5:1, or from 2.4:1 to 2.7:1.

In at least one embodiment, the lamellar gel matrix comprises a cationic surfactant and a high melting point fatty compound. In view of providing the lamellar gel matrix, the cationic surfactant and the high melting point fatty compound are contained at a level such that the weight ratio of the cationic surfactant to the high melting point fatty compound is in the range of from 1: 1 to 1: 10, or from 1: 1 to 1:6.

In at least one embodiment, the composition comprises a cationic surfactant. In at least one embodiment, the composition comprises from 0.05 % to 3.0 %, or from 0.075 % to 2.0 %, or from 0.1 % to 1.0 %, of cationic surfactant by total weight of the composition. In at least one embodiment, the cationic surfactant is comprised in a lamellar gel matrix. In other words, the composition comprises a lamellar gel matrix and the lamellar gel matrix comprises the cationic surfactant. In an embodiment, cationic surfactant is according to Formula (C): 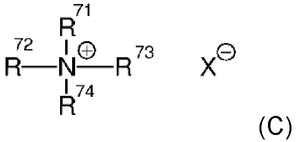 wherein
at least one of R⁷¹, R⁷², R⁷³ and R⁷⁴ is selected from an aliphatic group of from 8 to 30 carbon atoms, an aromatic, alkoxy, polyoxyalkylene, alkylamido, hydroxyalkyl, aryl, or an alkylaryl group having up to 22 carbon atoms;
the remainder of R⁷¹, R⁷², R⁷³ and R⁷⁴ are independently selected from the group consisting of an aliphatic group consisting of from 1 to 22 carbon atoms, and an aromatic, alkoxy, polyoxyalkylene, alkylamido, hydroxyalkyl, aryl or alkylaryl group having up to 22 carbon atoms;
X is selected from the group consisting of: halogen, acetate, citrate, lactate, glycolate, phosphate, nitrate, sulfonate, sulfate, alkylsulfate, alkyl sulfonate radicals, and combinations thereof.

In at least one embodiment, the cationic surfactant is selected from the group consisting of behenyl trimethyl ammonium chloride, methyl sulfate or ethyl sulfate, and stearyl trimethyl ammonium chloride, methyl sulfate or ethyl sulfate. It is believed that a longer alkyl group provides improved smoothness and soft feeling on wet and dry hair, compared to cationic surfactants with a shorter alkyl group. It is also believed that such cationic surfactants can provide reduced irritation, compared to those having a shorter alkyl group.

In at least one embodiment, the cationic surfactant is a di-long alkyl quatemized ammonium salt selected from the group consisting of: dialkyl (14-18) dimethyl ammonium chloride, ditallow alkyl dimethyl ammonium chloride, dihydrogenated tallow alkyl dimethyl ammonium chloride, distearyl dimethyl ammonium chloride, dicetyl dimethyl ammonium chloride, and mixtures thereof.

In at least one embodiment, the cationic surfactant is a tertiary amido amine having an alkyl group of from 12 to 22 carbons. The tertiary amido amine may be selected from the group consisting of stearamidopropyldimethyl-, stearamidopropyldiethyl-, stearamidoethyldiethyl-, stearamidoethyldimethyl-, palmitamidopropyldimethyl-, palmitamidopropyldiethyl-, palmitamidoethyldiethyl-, palmitamidoethyldimethyl-, behenamidopropyldimethy1-, behenamidopropyldiethyl-, behenamidoethyldiethyl-, behenamidoethyldimethyl-, arachidamidopropy ldimethy 1-, arachidamidopropyldiethyl-, arachidamidoethyldiethyl-, and arachidamidoethyldimethyl-amine, diethylaminoethylstearamide, and mixtures thereof. A tertiary amido amine may be used in combination with an acid. The acid is typically used as a salt-forming anion. In an embodiment, the acid is selected from the group consisting of lactic acid, malic acid, hydrochloric acid, 1-glumatic acid, acetic acid, citric acid, and mixtures thereof.

In at least one embodiment, the cationic surfactant is selected from the group consisting of cetyltrimonium chloride (CTAC), stearyltrimonium chloride (STAC), behentrimonium methosulfate, stearoylamidopropyldimethyl amine (SAPDMA), distearyldimethylammonium chloride, and mixtures thereof.

### Example Embodiments of the First Aspect

In a preferred embodiment, the first aspect relates to a cosmetic composition, preferably a hair cleansing composition, comprising:
(i) A copolymer comprising:
   (A) from 5.0 mol-% to 40.0 mol-% of one or more cationic structural units (A); and
   (B) from 3.0 mol-% to 45 mol-% of one or more macromonomeric structural units (B); and
   (C) from 15.0 mol-% to 92.0 mol-% of one or more structural units (C); and
(ii) A cosmetically acceptable component;

wherein the one or more cationic structural units (A) represent the polymerization product of at least one monomer species selected from the group consisting of diallyldimethylammonium chloride (DADMAC), (3-(Mmethacryloylamino)propyl)trimethylammonium chloride (MAPTAC), (3-(acrylamidopropyl)trimethylammonium chloride (AAPTAC);
and wherein the one or more structural units (C) represent the polymerization product of at least one monomer species selected from the group consisting of acrylamide, methacrylamide, N-methylacrylamide, N,N'-dimethylacrylamide, N-ethylacrylamide, N-cyclohexylacrylamide, N-benzylacrylamide, N-methylolacrylamide, N-isopropylacrylamide, and N-tert-butylacrylamide, and preferably selected from the group consisting of N,N'-dimethylacrylamide (DMAA) and N-isopropylacrylamide (NIPAM); and
wherein the one or more macromonomeric structural units (B) are represented by the general formula (III): 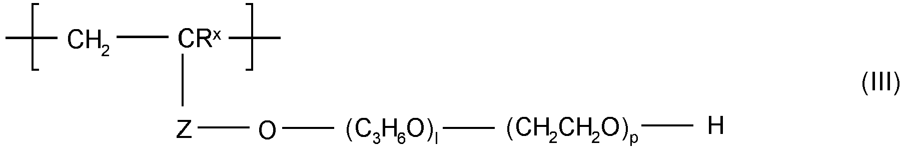 in which
   - R^{x}: is identical or different and is represented by methyl,
   - Z: is identical or different and is represented by C=O,
   - I: is on molar average a number from 0 to 6 and preferably from 0 to 5, more preferably 1 to 4, most preferably 2 to 3 and
   - p: is, on molar average, a number from 1 to 150, preferably from 4 to 150, and more preferably from 8 to 150 and most preferably from 10 to 25 or 30 to 50 or 100 to 130,
and wherein the weight average molecular weight M_{w} of the copolymer is from 10 000 to 200 000 g/mol.

In a preferred embodiment, the first aspect relates to a cosmetic composition, preferably a hair cleansing composition, comprising:
(i) A copolymer comprising:
   (A) from 5.0 mol-% to 40.0 mol-% of one or more cationic structural units (A); and
   (B) from 3.0 mol-% to 45 mol-% of one or more macromonomeric structural units (B); and
   (C) from 15.0 mol-% to 92.0 mol-% of one or more structural units (C); and
(ii) A cosmetically acceptable component selected from the group consisting of surfactants, surfactant systems, oily substances, waxes, emulsifiers, coemulsifiers, solubilizers, cationic polymers, film formers, superfatting agents, refatting agents, foam stabilizers, stabilizers, active biogenic substances, preservatives, preservation boosting ingredients, anti-fungal substance, anti-seborrheics and anti-dandruff agents, dyes or pigments, particulate substances, opacifiers, abrasives, absorbents, anticaking agents, bulking agents, pearlizing agents, direct dyes, perfumes or fragrances, carriers, solvents or diluents, propellants, functional acids, active ingredients, skin-brightening agents, self-tanning agents, exfoliants, enzymes, anti-acne agents, depilating agents, deodorants and antiperspirants, viscosity modifiers, thickening and gelling agents, pH adjusting agents, buffering agents, anti-oxidants, chelants, astringents, sunscreens, sun protection agents, UV filters, skin conditioning agents, emollients, humectants, occlusive agents, pediculocides, anti-foaming agents, flavouring agents, electrolytes and combinations thereof;

wherein the one or more cationic structural units (A) represent the polymerization product of at least one monomer species selected from the group consisting of diallyldimethylammonium chloride (DADMAC), (3-(Mmethacryloylamino)propyl)trimethylammonium chloride (MAPTAC), (3-(acrylamidopropyl)trimethylammonium chloride (AAPTAC);
and wherein the one or more structural units (C) represent the polymerization product of at least one monomer species selected from the group consisting of acrylamide, methacrylamide, N-methylacrylamide, N,N'-dimethylacrylamide, N-ethylacrylamide, N-cyclohexylacrylamide, N-benzylacrylamide, N-methylolacrylamide, N-isopropylacrylamide, and N-tert-butylacrylamide, and preferably selected from the group consisting of N,N'-dimethylacrylamide (DMAA) and N-isopropylacrylamide (NIPAM); and
wherein the one or more macromonomeric structural units (B) are represented by the general formula (III): 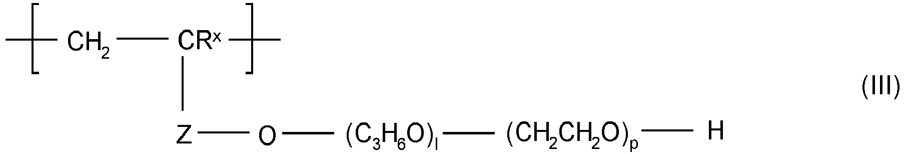 in which
   - R^{x}: is identical or different and is represented by methyl,
   - Z: is identical or different and is represented by C=O,
   - I: is on molar average a number from 0 to 6 and preferably from 0 to 5, more preferably 1 to 4, most preferably 2 to 3 and
   - p: is, on molar average, a number from 1 to 150, preferably from 4 to 150, and more preferably from 8 to 150 and most preferably from 10 to 25 or 30 to 50 or 100 to 130,
and wherein the weight average molecular weight M_{w} of the copolymer is from 10 000 to 200 000 g/mol.

In a preferred embodiment, the first aspect relates to a cosmetic composition, preferably a hair cleansing composition, comprising:
(i) A copolymer comprising:
   (A) from 5.0 mol-% to 40.0 mol-% of one or more cationic structural units (A); and
   (B) from 3.0 mol-% to 45 mol-% of one or more macromonomeric structural units (B); and
   (C) from 15.0 mol-% to 92.0 mol-% of one or more structural units (C); and
(ii) from 10 wt.% to 50 wt.% anionic surfactant;
(iii) optionally a further surfactant selected from the group consisting of non-ionic surfactants, zwitterionic surfactants, amphoteric surfactants and combinations thereof;
(iv) at least one further cosmetically acceptable component;

wherein the one or more cationic structural units (A) represent the polymerization product of at least one monomer species selected from the group consisting of diallyldimethylammonium chloride (DADMAC), (3-(Mmethacryloylamino)propyl)trimethylammonium chloride (MAPTAC), (3-(acrylamidopropyl)trimethylammonium chloride (AAPTAC);
and wherein the one or more structural units (C) represent the polymerization product of at least one monomer species selected from the group consisting of acrylamide, methacrylamide, N-methylacrylamide, N,N'-dimethylacrylamide, N-ethylacrylamide, N-cyclohexylacrylamide, N-benzylacrylamide, N-methylolacrylamide, N-isopropylacrylamide, and N-tert-butylacrylamide, and preferably selected from the group consisting of N,N'-dimethylacrylamide (DMAA) and N-isopropylacrylamide (NIPAM); and
wherein the one or more macromonomeric structural units (B) are represented by the general formula (III): 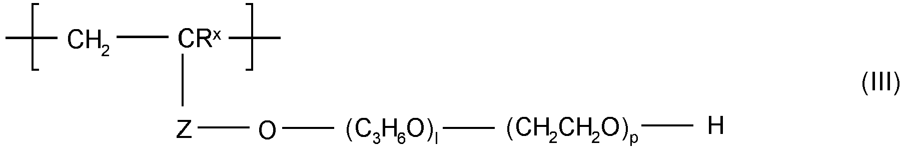 in which
   - R^{x}: is identical or different and is represented by methyl,
   - Z: is identical or different and is represented by C=O,
   - I: is on molar average a number from 0 to 6 and preferably from 0 to 5, more preferably 1 to 4, most preferably 2 to 3 and
   - p: is, on molar average, a number from 1 to 150, preferably from 4 to 150, and more preferably from 8 to 150 and most preferably from 10 to 25 or 30 to 50 or 100 to 130,
and wherein the weight average molecular weight M_{w} of the copolymer is from 10 000 to 200 000 g/mol.

In a preferred embodiment, the first aspect relates to a cosmetic composition, preferably a hair cleansing composition, comprising:
(i) from 0.1 wt-% to 15 wt-% of a copolymer consisting of:
   (A) from 5.0 mol-% to 40.0 mol-% of one or more cationic structural units (A); and
   (B) from 3.0 mol-% to 45 mol-% of one or more macromonomeric structural units (B); and
   (C) from 15.0 mol-% to 92.0 mol-% of one or more structural units (C);
      and
(ii) at least 10 wt.% of a cosmetically acceptable component;

wherein the one or more cationic structural units (A) represent the polymerization product of at least one monomer species selected from the group consisting of diallyldimethylammonium chloride (DADMAC), (3-(Mmethacryloylamino)propyl)trimethylammonium chloride (MAPTAC), (3-(acrylamidopropyl)trimethylammonium chloride (AAPTAC);
and wherein the one or more structural units (C) represent the polymerization product of at least one monomer species selected from the group consisting of acrylamide, methacrylamide, N-methylacrylamide, N,N'-dimethylacrylamide, N-ethylacrylamide, N-cyclohexylacrylamide, N-benzylacrylamide, N-methylolacrylamide, N-isopropylacrylamide, and N-tert-butylacrylamide, and preferably selected from the group consisting of N,N'-dimethylacrylamide (DMAA) and N-isopropylacrylamide (NIPAM); and
wherein the one or more macromonomeric structural units (B) are represented by the general formula (III): 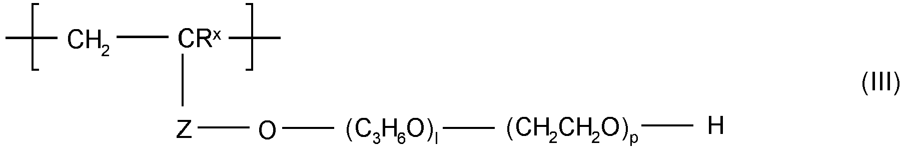 in which
   - R^{x}: is identical or different and is represented by methyl,
   - Z: is identical or different and is represented by C=O,
   - I: is on molar average a number from 0 to 6 and preferably from 0 to 5, more preferably 1 to 4, most preferably 2 to 3 and

   - p: is, on molar average, a number from 1 to 150, preferably from 4 to 150, and more preferably from 8 to 150 and most preferably from 10 to 25 or 30 to 50 or 100 to 130,
and wherein the weight average molecular weight M_{w} of the copolymer is from 10 000 to 200 000 g/mol.

### Second Aspect

A second aspect relates to a method of using a cosmetic composition comprising applying the composition according to the first aspect to keratinous material, preferably onto keratinous material. In at least one embodiment, the keratinous material is hair or skin, preferably mammalian hair or skin, more preferably human hair or skin. In at least one embodiment, the keratinous material is keratinous fibres, preferably human scalp hair or human facial hair or human chest hair.

### Third Aspect

A third aspect relates to the use of a composition for volumising keratinous fibres, wherein the composition is according to the first aspect. In at least one embodiment of the third aspect, the cosmetic composition comprises:
(i) A copolymer comprising:
   (A) from 0.1 mol-% to 99.8 mol-% of one or more cationic structural units (A); and
   (B) from 0.1 mol-% to 99.8 mol-% of one or more macromonomeric structural units (B); and
   (C) from 0.1 mol-% to 99.8 mol-% of one or more structural units (C) which differ from the structural units (A) and (B); and
(ii) A cosmetically acceptable component;

wherein the one or more cationic structural units (A) are represented by the following general formulae (I) and/or (II): 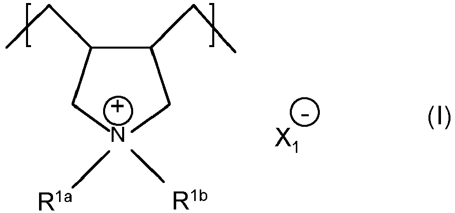 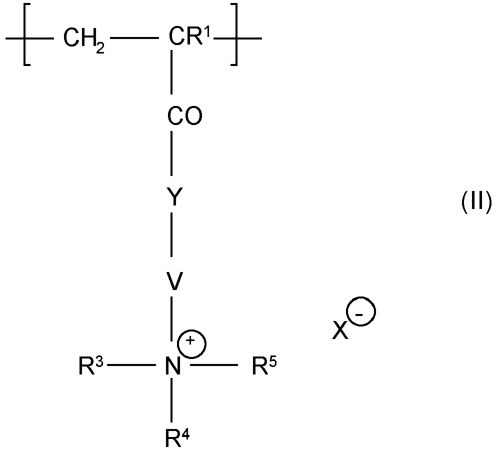
   - R¹ and R^{1a}: are each identical or different and, independently of one another, are each hydrogen and/or a methyl radical,
   - R^{1b}, R³, R⁴ and R⁵: are each identical or different and, independently of one another, are each represented by hydrogen, an aliphatic hydrocarbon radical having 1 to 20 carbon atoms, preferably 1 to 4 carbon atoms, a cycloaliphatic hydrocarbon radical having 5 to 20 carbon atoms, preferably 5 to 8 carbon atoms, an aryl radical having 6 to 14 carbon atoms and/or polyethylene glycol (PEG); preferably are each identical or different and, independently of one another, are each represented by hydrogen and/or methyl; more preferably are each methyl,
   - Y: is identical or different and is represented by oxygen, NH and/or NR³,
   - V: is identical or different and is represented by -(CH₂)ₓ-,

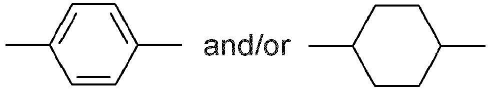

   - x: is identical or different and is represented by an integer from 1 to 6,
   - X and X₁: are each identical or different and, independently of one another, are each represented by a halogen atom, C₁ to C₄ alkyl sulfate and/or C₁ to C₄- alkylsulfonate,
   and the one or more macromonomeric structural units (B) are represented by the general formula (III): 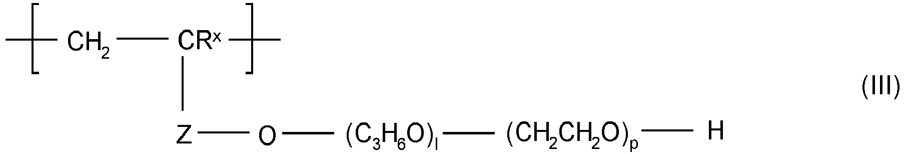 in which
   - R^{x}: is identical or different and is represented by H and/or methyl,
   - Z: is identical or different and is represented by C=O and/or O(CH₂)₄, preferably Z is O(CH₂)₄,
   - I: is on molar average a number from 0 to 6, preferably from 0 to 5, and
   - p: is, on molar average, a number from 1 to 150; preferably from 4 to 150; more preferably from 8 to 150,
   and the one or more structural units (C) are selected from the group consisting of the polymerization product of at least one cyclic N-vinyl-substituted lactam having 5 to 7 ring atoms and structural units of the following general formulae (IV) and (V): 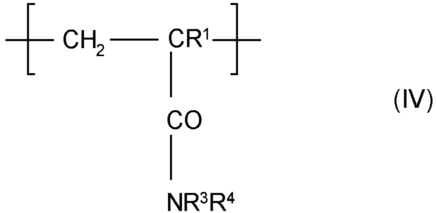 in which
   - R¹: is identical or different and is hydrogen and/or methyl, and
   - R³ and R⁴: are each identical or different and independently of one another are each represented by hydrogen, an aliphatic hydrocarbon radical having 1 to 20, preferably 1 to 4, carbon atoms, a cycloaliphatic hydrocarbon radical having 5 to 20, preferably 5 to 8, carbon atoms, an aryl radical having 6 to 14 Carbon atoms, an alkylaryl radical having 7 to 14 carbon atoms, a branched or unbranched C₁-C₅ monohydroxyalkyl group and/or polyethylene glycol (PEG) 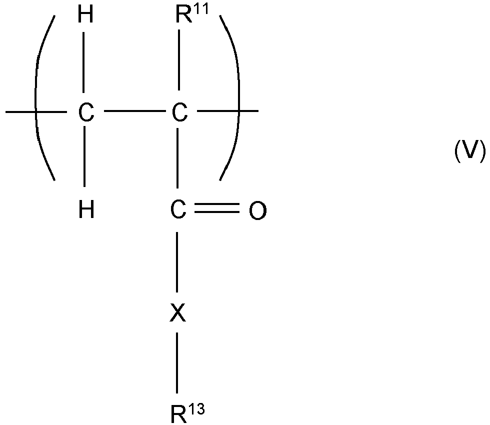
   in which
   - R¹¹: is identical or different and is represented by H and/or methyl;
   - X: is identical or different and is represented by NH-(CₙH₂ₙ) where n = 1, 2, 3 or 4; and
   - R¹³: is identical or different and is represented by OH, N(CH₃)₂, SO₃H, PO₃H₂, O-PO₃H₂ and/or para-substituted C₆H₄-SO₃H,
and where, among the structural units of the formula (V) in which R¹³ is N(CH₃)₂, preference is given to those structural units which represent the polymerization product of at least one monomer species selected from the group consisting of [3-(methacryloylamino)propyl]dimethylamine (R¹¹ = methyl; X = NH-(CₙH₂ₙ) where n = 3, and R¹³ = N(CH₃)₂) and [3-(acryloylamino)propyl]dimethylamine (R¹¹ = H; X = NH-(CₙH₂ₙ) where n = 3, and R¹³ = N(CH₃)₂),
and wherein the weight average molecular weight M_{w} of the copolymer is from 10 000 to 200 000 g/mol.

The preferred features of the copolymer are described in more detail in the first aspect.

In an alternative embodiment, the third aspect relates to the use of a copolymer for volumising keratinous fibres, wherein the copolymer comprises:
(A) from 0.1 mol-% to 99.8 mol-% of one or more cationic structural units (A); and
(B) from 0.1 mol-% to 99.8 mol-% of one or more macromonomeric structural units (B); and
(C) from 0.1 mol-% to 99.8 mol-% of one or more structural units (C) which differ from the structural units (A) and (B);

wherein the one or more cationic structural units (A) are represented by the following general formulae (I) and/or (II): 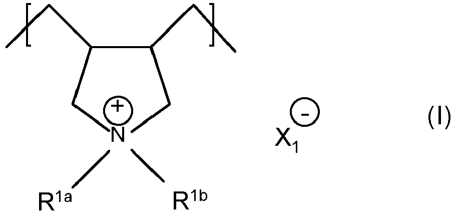 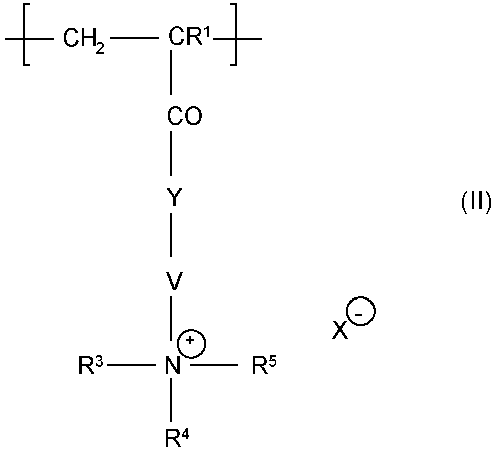
   - R¹ and R^{1a}: are each identical or different and, independently of one another, are each hydrogen and/or a methyl radical,
   - R^{1b}, R³, R⁴ and R⁵: are each identical or different and, independently of one another, are each represented by hydrogen, an aliphatic hydrocarbon radical having 1 to 20 carbon atoms, preferably 1 to 4 carbon atoms, a cycloaliphatic hydrocarbon radical having 5 to 20 carbon atoms, preferably 5 to 8 carbon atoms, an aryl radical having 6 to 14 carbon atoms and/or polyethylene glycol (PEG); preferably are each identical or different and, independently of one another, are each represented by hydrogen and/or methyl; more preferably are each methyl,
   - Y: is identical or different and is represented by oxygen, NH and/or NR³,
   - V: is identical or different and is represented by -(CH₂)ₓ-,

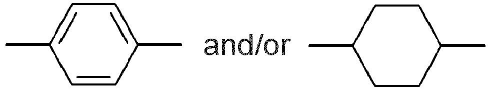

   - x: is identical or different and is represented by an integer from 1 to 6,

   - X and X₁: are each identical or different and, independently of one another, are each represented by a halogen atom, C₁ to C₄ alkyl sulfate and/or C₁ to C₄- alkylsulfonate,
   and the one or more macromonomeric structural units (B) are represented by the general formula (III): 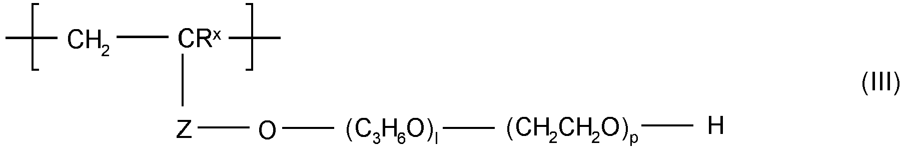 in which
   - R^{x}: is identical or different and is represented by H and/or methyl,
   - Z: is identical or different and is represented by C=O and/or O(CH₂)₄, preferably Z is O(CH₂)₄,
   - I: is on molar average a number from 0 to 6, preferably from 0 to 5, and
   - p: is, on molar average, a number from 1 to 150; preferably from 4 to 150; more preferably from 8 to 150,
   and the one or more structural units (C) are selected from the group consisting of the polymerization product of at least one cyclic N-vinyl-substituted lactam having 5 to 7 ring atoms and structural units of the following general formulae (IV) and (V): 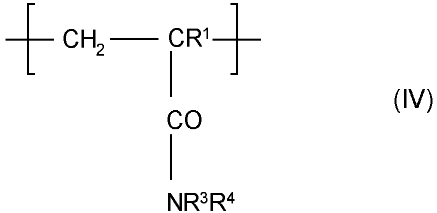 in which
   - R¹: is identical or different and is hydrogen and/or methyl, and
   - R³ and R⁴: are each identical or different and independently of one another are each represented by hydrogen, an aliphatic hydrocarbon radical having 1 to 20, preferably 1 to 4, carbon atoms, a cycloaliphatic hydrocarbon radical having 5 to 20, preferably 5 to 8, carbon atoms, an aryl radical having 6 to 14 Carbon atoms, an alkylaryl radical having 7 to 14 carbon atoms, a branched or unbranched C₁-C₅ monohydroxyalkyl group and/or polyethylene glycol (PEG) 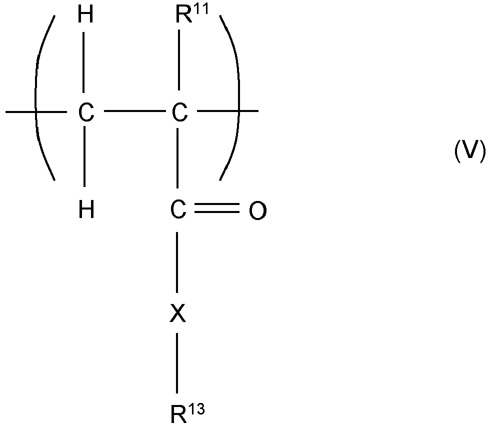
   in which
   - R¹¹: is identical or different and is represented by H and/or methyl;
   - X: is identical or different and is represented by NH-(CₙH₂ₙ) where n = 1, 2, 3 or 4; and
   - R¹³: is identical or different and is represented by OH, N(CH₃)₂, SO₃H, PO₃H₂, O-PO₃H₂ and/or para-substituted C₆H₄-SO₃H, and where, among the structural units of the formula (V) in which R¹³ is N(CH₃)₂, preference is given to those structural units which represent the polymerization product of at least one monomer species selected from the group consisting of [3-(methacryloylamino)propyl]dimethylamine (R¹¹ = methyl; X = NH-(CₙH₂ₙ) where n = 3, and R¹³ = N(CH₃)₂) and [3-(acryloylamino)propyl]dimethylamine (R¹¹ = H; X = NH-(CₙH₂ₙ) where n = 3, and R¹³ = N(CH₃)₂),
and wherein the weight average molecular weight M_{w} of the copolymer is from 10 000 to 200 000 g/mol.

The preferred features of the copolymer are described in more detail in the first aspect.

### Fourth Aspect

A fourth aspect relates to a process for formulating a cosmetic composition comprising providing a copolymer and a cosmetically acceptable component and mixing the copolymer and a cosmetically acceptable component together to form a cosmetic composition, wherein the copolymer comprises:
(A) from 0.1 mol-% to 99.8 mol-% of one or more cationic structural units (A); and
(B) from 0.1 mol-% to 99.8 mol-% of one or more macromonomeric structural units (B); and
(C) from 0.1 mol-% to 99.8 mol-% of one or more structural units (C) which differ from the structural units (A) and (B);

wherein the one or more cationic structural units (A) are represented by the following general formulae (I) and/or (II): 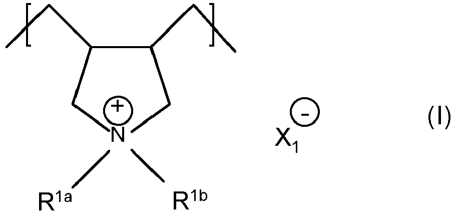 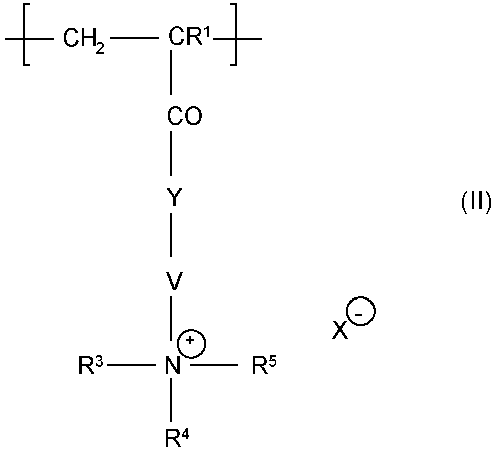
   - R¹ and R^{1a}: are each identical or different and, independently of one another, are each hydrogen and/or a methyl radical,
   - R^{1b}, R³, R⁴ and R⁵: are each identical or different and, independently of one another, are each represented by hydrogen, an aliphatic hydrocarbon radical having 1 to 20 carbon atoms, preferably 1 to 4 carbon atoms, a cycloaliphatic hydrocarbon radical having 5 to 20 carbon atoms, preferably 5 to 8 carbon atoms, an aryl radical having 6 to 14 carbon atoms and/or polyethylene glycol (PEG); preferably are each identical or different and, independently of one another, are each represented by hydrogen and/or methyl; more preferably are each methyl,
   - Y: is identical or different and is represented by oxygen, NH and/or NR³,
   - V: is identical or different and is represented by -(CH₂)ₓ-,

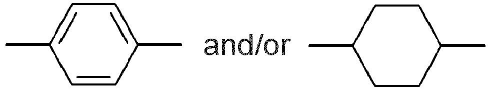

   - x: is identical or different and is represented by an integer from 1 to 6,

   - X and X₁: are each identical or different and, independently of one another, are each represented by a halogen atom, C₁ to C₄ alkyl sulfate and/or C₁ to C₄- alkylsulfonate,
   and the one or more macromonomeric structural units (B) are represented by the general formula (III): 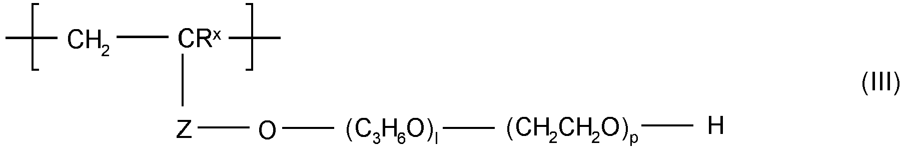 in which
   - R^{x}: is identical or different and is represented by H and/or methyl,
   - Z: is identical or different and is represented by C=O and/or O(CH₂)₄, preferably Z is O(CH₂)₄,
   - I: is on molar average a number from 0 to 6, preferably from 0 to 5, and
   - p: is, on molar average, a number from 1 to 150; preferably from 4 to 150; more preferably from 8 to 150,
   and the one or more structural units (C) are selected from the group consisting of the polymerization product of at least one cyclic N-vinyl-substituted lactam having 5 to 7 ring atoms and structural units of the following general formulae (IV) and (V): 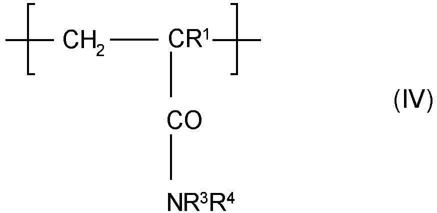 in which
   - R¹: is identical or different and is hydrogen and/or methyl, and
   - R³ and R⁴: are each identical or different and independently of one another are each represented by hydrogen, an aliphatic hydrocarbon radical having 1 to 20, preferably 1 to 4, carbon atoms, a cycloaliphatic hydrocarbon radical having 5 to 20, preferably 5 to 8, carbon atoms, an aryl radical having 6 to 14 Carbon atoms, an alkylaryl radical having 7 to 14 carbon atoms, a branched or unbranched C₁-C₅ monohydroxyalkyl group and/or polyethylene glycol (PEG) 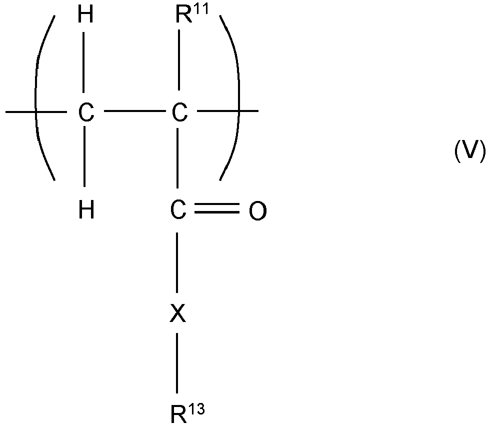
   in which
   - R¹¹: is identical or different and is represented by H and/or methyl;
   - X: is identical or different and is represented by NH-(CₙH₂ₙ) where n = 1, 2, 3 or 4; and
   - R¹³: is identical or different and is represented by OH, N(CH₃)₂, SO₃H, PO₃H₂, O-PO₃H₂ and/or para-substituted C₆H₄-SO₃H,
and where, among the structural units of the formula (V) in which R¹³ is N(CH₃)₂, preference is given to those structural units which represent the polymerization product of at least one monomer species selected from the group consisting of [3-(methacryloylamino)propyl]dimethylamine (R¹¹ = methyl; X = NH-(CₙH₂ₙ) where n = 3, and R¹³ = N(CH₃)₂) and [3-(acryloylamino)propyl]dimethylamine (R¹¹ = H; X = NH-(CₙH₂ₙ) where n = 3, and R¹³ = N(CH₃)₂),
and wherein the weight average molecular weight M_{w} of the copolymer is from 10 000 to 200 000 g/mol.

The preferred features of the copolymer are described in more detail in the first aspect.

### Fifth Aspect

A fifth aspect relates to a cosmetic product comprising a receptacle comprising the composition according to the first aspect. In at least one embodiment, the product comprises an opening for dispensing the composition. In at least one embodiment, the opening is equipped with a closure. In at least one embodiment, the receptacle comprises plastics.

### Sixth Aspect

A sixth aspect relates to a process for synthesising a copolymer according to the first aspect.

In at least one embodiment, the process comprises:
(I) Creating an aqueous reaction mixture, wherein the reaction mixture comprises a cationic monomer (a), a macromonomer (b) and a solvent;
(II) Heating the reaction mixture to a temperature of at least 40 °C;
(III) Adding an initiator solution to the reaction mixture;
(IV) Stirring the reaction mixture at a temperature of at least 40 °C for at least 10 minutes;
(V) Optionally isolating a copolymer;

wherein the cationic monomer (a) is selected from the group consisting of [2-(acryloyloxy)ethyl]trimethylammonium chloride, [2-(acryloylamino)-ethyl]trimethylammonium chloride, [2-(acryloyloxy)ethyl]trimethylammonium methosulfate, [2-(methacryloyloxy)ethyl]trimethylammonium chloride and methosulfate, [3-(acryloylamino)propyl]trimethylammonium chloride, [3-(methacryloylamino)propyl]trimethylammonium chloride and diallyldimethylammonium chloride (DADMAC), preferably selected from the group consisting of [3-(acryloylamino)propyl]trimethylammonium chloride, [3-(methacryloylamino)propyl]trimethylammonium chloride, and
diallyldimethylammonium chloride, and more preferably selected from the group consisting of [3-(methacryloylamino)propyl]trimethylammonium chloride and diallyldimethylammonium chloride, most preferably wherein the one or more cationic structural units (A) of the copolymer represent the polymerization product of diallyldimethylammonium chloride (DADMAC);
and preferably wherein the macromonomer (b) is selected from the group consisting of (meth)acrylic acid esters of a polyethylene glycol, (meth)acrylic acid esters of a polypropylene glycol, and (meth)acrylic acid esters of a polyglycol comprising both oxyethylene and oxypropylene monomers.

In at least one embodiment, the step (IV) results in radical polymerisation of the monomers.

In at least one embodiment, after step (I) but prior to step (II), the reaction mixture is flushed with nitrogen gas for at least 5 minutes, preferably at least 10 minutes, more preferably at least 20 minutes, more preferably at least 30 minutes, more preferably at least 45 minutes, even more preferably at least 60 minutes, most preferably at least 90 minutes.

In at least one embodiment of the step (IV), the reaction mixture is stirred at a temperature of at least 45 °C, or at least 50 °C, or within the range of 55 °C to 110 °C, or 60 °C to 105 °C, or 70 °C to 100 °C. In at least one embodiment of the step (IV), the reaction mixture is stirred for at least 20 minutes, preferably at least 30 minutes, more preferably at least 45 minutes, even more preferably at least 60 minutes, most preferably at least 90 minutes.

In at least one embodiment of the step (II), the reaction mixture is heated to a temperature of at least 45 °C, or at least 50 °C, or within the range of 55 °C to 110 °C, or 60 °C to 105 °C, or 70 °C to 100 °C. In at least one embodiment of the step (II), the reaction mixture is heated for at least 20 minutes, preferably at least 30 minutes, more preferably at least 45 minutes, even more preferably at least 60 minutes, most preferably at least 90 minutes.

The reaction mixture comprises monomer (c), which is different from cationic monomer (a) and a macromonomer (b).

In at least one embodiment, the monomer (c) is selected from the group consisting of acrylamide, methacrylamide, N-methylacrylamide, N,N'-dimethylacrylamide, N-ethylacrylamide, N-cyclohexylacrylamide, N-benzylacrylamide, N-methylolacrylamide, N-isopropylacrylamide, and N-tert-butylacrylamide, and preferably selected from the group consisting of N,N'-dimethylacrylamide (DMAA) and N-isopropylacrylamide (NIPAM).

The reaction mixture comprises copolymer comprises from 0.1 to 99.8 mol-% of the cationic monomer (a), from 0.1 to 99.8 mol-% of the macromonomer (b), and from 0.1 to 99.8 mol-% of monomer (c);
more preferably wherein the copolymer comprises from 20.0 to 80.0 mol-% of the cationic monomer (a), from 0.4 to 20.0 mol-% of the macromonomer (b), and from 15.5 to 74.6 mol-% of the monomer (c);
even more preferably wherein the copolymer comprises from 22.0 to 77.6 mol-% of the cationic monomer (a), from 0.5 to 4.4 mol-% of the macromonomer (b), and from 18.0 to 74.5 mol-% of the monomer (c).

In at least one embodiment, the initiator solution comprises an initiator selected from the group consisting of 2,2'-azobis[2-(2-imidazolin-2-yl)propane]dihydrochloride, dimethyl 2,2'-azobis(2-methylpropionate), and combinations thereof.

The reaction mixture is an aqueous reaction mixture and therefore comprises water molecules. The aqueous reaction mixture comprises a solvent. In at least one embodiment, the reaction mixture comprises an aqueous, alcoholic or aqueous-alcoholic solvent, and wherein the aqueous, alcoholic or aqueous-alcoholic solvent comprises water, ethanol, propanol, isopropanol, 1,2-propylene glycol, 1,3-propylene glycol, isobutanol, *tert.*-butanol, butanol, butyl glycol, butyl diglycol, glycerol, or a mixture thereof. In at least one embodiment, the solvent is selected from water, *tert*.-butanol, isopropanol and combinations thereof.

In at least one embodiment, the initiator solution comprises solvent.

In at least one embodiment, the initiator solution is metering into the reaction mixture gradually over a period of at least 20 minutes, preferably at least 30 minutes, more preferably at least 45 minutes, even more preferably at least 60 minutes, most preferably at least 90 minutes.

In at least one embodiment of step (V), solvent is removed by using a rotary evaporator.

In at least one embodiment of step (V), the copolymer is isolated as a solid.

### EXAMPLES & EXPERIMENTAL

The examples which follow are intended to illustrate the subject matter of the invention without restricting it thereto.

### General protocol for preparing the copolymers:

In a multi-neck flask equipped with a mechanical stirrer ("KPG stirrer"), reflux condenser, and N₂ connection, the amounts of chemicals stated in Table 1 (except for the initiator) are dissolved under nitrogen (5 litres/hour) in the stated amount of distilled water or solvent for the Copolymer Examples indicated in Table 1. It should be borne in mind that certain monomers are employed in aqueous form.

The indicated solvent in the Table 1 is added in addition to the water introduced by way of the monomers to the stated concentration.

The solution of the monomers is subsequently flushed with nitrogen for 30 minutes and heated to 60 °C if using VA-44 as initiator, or to 80 °C if using V-601 as initiator. In the next step, the amount of initiator (VA-44 or V-601) indicated in Table 1 is dissolved in 10 g of solvent and metered in over a period of 90 minutes. After the end of the metering, stirring is continued for a further hour at an internal temperature of 60 °C if using VA-44 as initiator or at an internal temperature of 80 °C if using V-601 as initiator. The solid is subsequently analysed to investigate the conversion rate in the reaction, and any unreacted monomers are reacted, where appropriate, by minor addition of a 10 wt.-% solution of the initiator already used beforehand, until complete conversion is achieved. Thereafter the organic solvents of the reaction mixtures are removed under reduced pressure by means of a rotary evaporator (if present in the polymerization mixture) and the final product is cooled to room temperature (20 - 23 °C).

**Table A: measured weight average molecular weights (Mw)**

| Copolymer Example (see Table 1) | M_{W} |
|---|---|
| 1 | 1.98 × 10⁵ g/mol |
| 2 | 1.10 × 10⁵ g/mol |
| 3 | 1.73 × 10⁵ g/mol |
| 4 | 1.31 × 10⁵ g/mol |
| 5 | 2.66 × 10⁴ g/mol |
| 8 | 1.51 × 10⁵ g/mol |
| 11 | 9.75 × 10⁴ g/mol |
| 12 | 1.13 × 10⁵ g/mol |
| 13 | 3.74 × 10⁴ g/mol |
| 14 | 7.20 × 10⁴ g/mol |
| 15 | 1.91 × 10⁴ g/mol |
| 16 | 2.19 × 10⁴ g/mol |

The methodology for calculating the Mw of the copolymers is in the Definitions and General section above.

### Experimental

Polymers are included in the shampoo formulation below as per the following protocol.
Mix ingredients of phase A until clear
Add phase B and disperse with agitation until fully hydrated
Add the corresponding phase C and stir until fully dispersed
Adjust pH and viscosity with phase D.

The following is a description of how the experiments in Table 2 were carried out including the formula card of the shampoo chassis used.

**Table B:**

| Phase | Ingredient | INCI | Invention Shampoo | Benchmark | | |
|---|---|---|---|---|---|---|
| | | | | Shampoo 1 | Shampoo 2 | Shampoo 3 |
| A | Water | Aqua | 83.60 | 83.60 | 83.60 | 83.60 |
| | Genapol LRO | Sodium Lauryl Ether Sulfate | 14.00 | 14.00 | 14.00 | 14.00 |
| | Dehyton PK 45 | Cocamido-propyl Betaine (BASF) | 1.50 | 1.50 | 1.50 | 1.50 |
| B | Carbopol 980 | Carbomer (Lubrizol) | 0.40 | 0.40 | 0.40 | 0.40 |
| C1 | Copolymer* | n.a. | 0.50 | 0.00 | 0.00 | 0.00 |
| C2 | Ucare Polymer JR400 | Poly-quaternium-10 | 0.00 | 0.50 | 0.00 | 0.00 |
| C3 | Jaguar^{®} C-162 | Guar Hydroxypropyl-trimonium Chloride | 0.00 | 0.00 | 0.50 | 0.00 |
| D | NaCl | Sodium Chloride | 2.20 | 2.20 | 2.20 | 2.20 |
| | NaOH (10 %) | Sodium Hydroxide | 2.00 | 2.00 | 2.00 | 2.00 |

| | | | | | | |
|---|---|---|---|---|---|---|
| KEY = * copolymer according to the present invention; all quantities are wt.-% active material. | | | | | | |

European bleached (4 h) hair tresses (2 cm wide, 15 cm long) are thoroughly washed with a 10 % solution of SLES (anionic surfactant) to strip the hair from any production residue. Tresses are dried. Shine, volume and frizz are measured for the baseline record. Pictures are taken on light box (reference/starting point) - volume for a switch washed in SLES is calculated as 100 %. Swatches are washed twice in chassis shampoo formulation containing 0.5 % copolymer according to the invention or benchmark polymer. Pictures are taken on light box. This washing step is repeated a second time. Swatches are washed twice in chassis shampoo formulation containing 0.5% copolymer according to the invention or benchmark polymer.

Pictures are taken on light box.

Calculation of the volume with Imaged photo processing software.

Bulk hair and frizz of hair samples are measured in cm² and volume increase/ decrease calculated in % vs SLES (100 %).

The results are summarised in Table 2.

In the "Versus "PQ10"" section, the Invention Shampoo is compared Benchmark Shampoo 1. In the "Versus "JAGUAR"" section, the Invention Shampoo is compared Benchmark Shampoo 2. In the "Versus "without polymer"" section, the Invention Shampoo is compared Benchmark Shampoo 3.

**Table 1: Example copolymers**

| Copolymer example | Monomer B [Name (mol-%)] | Monomer C [Name (mol-%)] | Monomer A [Name (mol-%)] | Total monomer (wt-%) | Solvent | NaHP [mol-%] | Initiator [mol-%] |
|---|---|---|---|---|---|---|---|
| 1 | MA5000 (3.8) | NIPAM (62.5) | DADMAC (33.7) | 21.6 | Water | 1.1 | VA-44 (3.8) |
| 2 | MA5000 (3.8) | NIPAM (62.5) | DADMAC (33.7) | 21.4 | Water | 1.1 | VA-44 (7.6) |
| 3 | MA5000 (3.8) | NIPAM (62.5) | DADMAC (33.7) | 22.3 | Water | 1.1 | VA-44 (3.3) |
| 4 | MA5000 (4.9) | NIPAM (80.8) | DADMAC (14.3) | 22.3 | Water | 1.5 | VA-44 (4.3) |
| 5 | MA5000 (3.8) | NIPAM (62.5) | DADMAC (33.7) | 22.3 | Water | 1.1 | VA-44 (3.3) |
| 6 | MA5000 (4.4) | DMAA (82.7) | DADMAC (12.9) | 22.3 | Water | 1.3 | VA-44 (3.8) |
| 7 | MA5000 (4.1) | NIPAM (68.8) | MAPTAC (27.1) | 22.3 | Water | 1.2 | VA-44 (3.6) |
| 8 | MA5000 (4.4) | DMAA (82.7) | DADMAC (12.9) | 22.2 | Water | 2.6 | VA-44 (3.8) |
| 9 | MA5000 (4.1) | NIPAM (68.7) | MAPTAC (27.1) | 23.4 | Water | 2.5 | VA-44 (3.6) |
| 10 | MA2000 (11.3) | NIPAM (75.3) | DADMAC (13.4) | 21.9 | Water | 1.4 | VA-44 (4.0) |
| 11 | MA2000 (9.7) | NIPAM (64.7) | MAPTAC (25.6) | 21.9 | Water | 2.3 | VA-44 (3.4) |
| 12 | MA2000 (10.2) | DMAA (77.7) | DADMAC (12.1) | 21.9 | Water | 2.5 | VA-44 (3.6) |
| 13 | MA2000 (11.3) | NIPAM (75.3) | DADMAC (13.4) | 21.9 | Water | 1.4 | VA-44 (4.0) |
| 14 | MA-1000 (30.2) | NIPAM (45.4) | AAPTAC (24.4) | 26.9 | Water/t-BuOH | 2.2 | VA-44 (3.4) |
| 15 | MA750 (43.2) | NIPAM (34.0) | MAPTAC (22.8) | 32.0 | i-PrOH | 1.3 | V-601 (3.2) |
| 16 | MA750 (43.2) | NIPAM (34.0) | MAPTAC (22.8) | 31.9 | i-PrOH | 1.3 | V-601 (3.5) |

| | | | | | | | |
|---|---|---|---|---|---|---|---|
| KEY: MA5000 = Polyglycol MA 5000/50 (Clariant, 50 wt.-% in water); MA2000 = Polyglycol MA 2000/55 (Clariant, 55 wt.-% in water); MA-1000 = Polyglycol MA 1000/70 (Clariant, 70 wt.-% in water); MA750 = Polyglycol MA 750 (Clariant); NIPAM = N-isopropylacrylamide (Sigma-Aldrich); DMAA = N,N-dimethylacrylamide (Sigma-Aldrich); DADMAC = diallyldimethylammonium chloride (Sigma-Aldrich, 65 wt.-% in water); MAPTAC = (3-(methacryloylamino)propyl)trimethylammonium chloride (Sigma-Aldrich, 50 wt.-% in water); AAPTAC = 3-(acrylamidopropyl)trimethylammonium chloride (Sigma-Aldrich, 75 wt.-% in water); t-BuOH = tert.-butanol; i-PrOH = isopropanol; NaHP = sodium hypophosphite; VA-44 = 2,2'-azobis[2-(2-imidazolin-2-yl)propane]dihydrochloride (Wako); V-601 = dimethyl 2,2'-azobis(2-methylpropionate) (Wako). | | | | | | | |

**Table 2: Experimental data**

| Copolymer Example | Wash | Versus "PQ10" | | | Versus "JAGUAR" | | | Versus "without polymer" | | |
|---|---|---|---|---|---|---|---|---|---|---|
| | | Shine | Volume | Frizz | Shine | Volume | Frizz | Shine | Volume | Frizz |
| 1 | 1. | ∘ | ∘ | + | ∘ | ∘ | + | ∘ | ∘ | + |
| | 2. | ∘ | + | + | ∘ | + | + | ∘ | ∘ | + |
| 2 | 1. | ∘ | + | - | ∘ | + | - | ∘ | + | - |
| | 2. | ∘ | + | - | ∘ | + | - | ∘ | + | - |
| 14 | 1. | ∘ | + | - | ∘ | + | - | ∘ | + | - |
| | 2. | ∘ | + | - | ∘ | + | - | + | ∘ | - |
| 16 | 1. | ∘ | + | - | ∘ | ∘ | - | ∘ | + | - |
| | 2. | ∘ | ∘ | - | ∘ | ∘ | - | ∘ | ∘ | - |

| | | | | | | | | | | |
|---|---|---|---|---|---|---|---|---|---|---|
| KEY: = PQ10 = Polyquaternium-10 (Ucare Polymer JR400, which is a quaternized hydroxyethylcellulose); JAGUAR = Jaguar^{®} C-162 from Rhodia; - means the current invention included at 0.5 % in a shampoo formulation performed less well than the benchmark; o means the current invention included at 0.5 % in a shampoo formulation performed as well as the benchmark, + means the current invention included at 0.5 % in a shampoo formulation performed better than the benchmark (more volume, less frizz). | | | | | | | | | | |

**Table 3: Example cosmetic compositions**

| Example | I | II | III | IV | V | VI |
|---|---|---|---|---|---|---|
| | Volumising Shampoo | Conditioning Shampoo | Beard cleanser | Styling mousse | Volumising serum | Daily lotion with SPF |
| Copolymer from Table 1 ^{#} | 1.0 | 1.0 | 1.0 | 5.0 | 0.5 | 1.0 |
| Sodium Laureth Sulfate¹ | 35.0 | 40.0 | 15.0 | 10.0 | - | - |
| Laureth-3² | 1.5 | - | - | - | 1.0 | - |
| Glycol Distearate and Cocamide MEA and PPG-4-Deceth-4³ | - | 2.0 | - | - | - | - |
| Coco Betaine⁴ | - | - | 3.0 | - | - | - |
| Cocamidopropyl Betaine⁵ | 8.0 | - | 4.0 | - | - | - |
| Sodium Laureth Sulfate (and) Glycol Distearate (and) Cocamide MEA⁶ | - | 5.0 | - | - | - | - |
| Sodium Cocoyl Isethionate (and) Stearic Acid⁷ | - | 3.6 | - | - | - | - |
| Cocoyl Methyl Glucosamide⁸ | - | - | 3.00 | - | 1.0 | - |
| Capryloyl/ Capryl Methyl Glucosamide⁹ | - | - | 1.00 | - | - | - |
| PEG-7 Glyceryl Cocoate¹⁰ | - | 0.5 | - | - | - | - |
| Sodium Benzoate | - | - | 0.45 | 0.2 | 0.2 | - |
| Guar Hydroxypropyltrimonium Chloride¹¹ | 0.1 | - | - | 0.2 | - | - |
| DMDM Hydantoin¹² | - | 0.2 | - | - | - | - |
| Piroctone Olamine ¹³ | 0.5 | - | - | 0.5 | 0.5 | - |
| Hydroxypropyl Cellulose¹⁴ | - | - | - | - | 1.0 | - |
| Cl 14200 | 0.1 | 0.1 | - | - | - | - |
| Fragrance | 0.3 | 0.3 | 0.2 | 0.2 | 0.1 | - |
| Citric acid (50%) | 0.6 | 0.5 | 0.5 | 0.3 | 0.2 | - |
| Sodium Chloride | 1.2 | 1.2 | - | - | - | - |
| Ethanol denat. | - | - | | 10.0 | 30.0 | - |
| Glycerin | - | - | - | - | - | 5.0 |
| Xanthan gum¹⁵ | - | - | - | - | - | 0.2 |
| Sucrose Polystearate and Cetearyl Alcohol and Olea Europae (Olive) oil Unsaponifiables¹⁶ | - | - | - | - | - | 2.0 |
| Trilaureth-4 Phosphate¹⁷ | - | - | - | - | - | 2.0 |
| Hydrogenated Ethylhexyl Olivate and Hydrogenated Olive oil Unsaponifiables¹⁸ | - | - | - | - | - | 3.0 |
| Ethylhexyl Methoxycinnamate¹⁹ | - | - | - | - | - | 10.0 |
| Caprylic/Capric Triglycerides²⁰ | - | - | - | - | - | 2.0 |
| Dimethicone²¹ | - | - | - | - | - | 1.0 |
| Diethylamino Hydroxybenzoyl Hexyl Benzoate²² | - | - | - | - | - | 3.0 |
| Cram be abyssinica seed oil²³ | - | - | - | - | - | 3.0 |
| Ammonium Acryloyldimethylta urate/ VP Copolymer²⁴ | - | - | - | - | - | 0.3 |
| Methylene bis-Benzotriazolyl Tetramethylbutylp henol, Aqua, Decyl Glucoside, Propylene Glycol, Xanthan Gum²⁵ | - | - | - | - | - | 6.0 |
| Phenoxyethanol and Sorbitan Caprylate²⁶ | - | - | - | - | - | 0.7 |
| Sodium Hydroxide (10 % aq.) | - | - | - | - | - | QS |
| Water | QSP | QSP | QSP | QSP | QSP | QSP |
| Total | 100% | 100% | 100% | 100% | 100% | 100% |

| | | | | | | |
|---|---|---|---|---|---|---|
| KEY: All numbers in the tables are wt% unless otherwise stated; QSP = sufficient quantity for 100 %; QS = amount as necessary; # = see above for examples, 1 = Genapol^{®} LRO from Clariant, 2 = Genapol^{®} LA 030 from Clariant, 3 = Genapol^{®} PGL from Clariant, 4 = Genagen^{®} KB from Clariant, 5 = Genagen^{®} CAB 318 from Clariant, 6 = Genapol^{®} PGM from Clariant, 7 = Hostapon^{®} SCI-65 from Clariant, 8 = GlucoTain^{®} Care from Clariant, 9 = GlucoTain^{®} Clear from Clariant, 10 = Cetiol^{®} HE from Cognis, 11 = Jaguar^{®} Excel C-162 from Rhodia, 12 = Nipaguard^{®} DMDMH from Clariant, 13 = Octopirox^{®} from Clariant, 14 = Klucel^{®} from Ashland, 15 = Keltrol CG-SFT from Kelco, 16 = Plantasens^{®} Natural Emulsifier HP10 from Clariant, 17 = Hostaphat^{®} KL 340D from Clariant, 18 = Plantasens^{®} Olive LD from Clariant, 19 = Cinnamon from Vivimed Ltd, 20 = Velsan^{®} CCT from Clariant, 21 = Xiameter^{®} 0200 (20cst) from Dow Corning Inc, 22 = Uvinul^{®} A Plus from BASF GmbH, 23= Plantasens^{®} Abyssinian Oil from Clariant, 24= Aristoflex^{®} AVC from Clariant, 25= Vivzole-M from Vivimed Ltd, 26 = Nipaguard^{®} SCP from Clariant. | | | | | | |

**Table 3: Example cosmetic compositions (continued)**

| Example | VII | VIII | IX |
|---|---|---|---|
| | Sulfate-free shampoo | Sensitive scalp Shampoo | Anti-dandruff shampoo |
| Copolymer from Table 1 ^{#} | 1.0 | 1.0 | 1.0 |
| Sodium Laureth Sulfate¹ | - | - | 38.9 |
| Laureth-3² | - | - | - |
| Glycol Distearate and Cocamide MEA and PPG-4-Deceth-4³ | - | - | - |
| Coco Betaine⁴ | - | - | 4.0 |
| Cocamidopropyl Betaine⁵ | - | 15.0 | - |
| Sodium Laureth Sulfate (and) Glycol Distearate (and) Cocamide MEA⁶ | - | - | - |
| Sodium Cocoyl Isethionate (and) Stearic Acid⁷ | - | - | 4.0 |
| Cocoyl Methyl Glucosamide⁸ | - | - | - |
| Capryloyl/ Capryl Methyl Glucosamide⁹ | 1.0 | - | - |
| PEG-7 Glyceryl Cocoate¹⁰ | - | - | - |
| Sodium Benzoate | - | - | 0.5 |
| Guar Hydroxypropyltrim onium Chloride¹¹ | 0.2 | - | - |
| DMDM Hydantoin¹² | - | - | - |
| Piroctone Olamine¹³ | - | - | 0.5 |
| Hydroxypropyl Cellulose¹⁴ | - | - | - |
| Cl 14200 | 0.2 | - | 0.2 |
| Fragrance | 0.2 | - | 0.2 |
| Citric acid (50 %) | - | 0.5 | 0.5 |
| Sodium Hydroxide (30%) | - | - | - |
| Sodium Chloride | 1.0 | - | 0.5 |
| Ethanol denat. | - | - | - |
| Glycerin | - | 2.0 | - |
| Xanthan gum¹⁵ | - | - | - |
| Sodium Olefin Sulfonate¹⁶ | 19.7 | - | - |
| Lauroyl/Myristoyl Methyl Glucamide and Coco Betaine¹⁷ | 9.5 | - | - |
| Sodium Benzoate | 0.5 | - | - |
| Trisodium Citrate | 0.7 | - | - |
| Sorbitan Caprylate (and) Proapanediol (and) Benzonic Acid¹⁸ | - | 1.2 | 1.0 |
| Sodium Methyl Cocoyl Taurate¹⁹ | - | 3.1 | 4.0 |
| Polysorbate 20²⁰ | - | 2.0 | - |
| PEG-150 Polyglyceryl-2 Tristearate (and) Laureth-3 (and) Dipropylene Glycol²¹ | - | 4.0 | - |
| Trideceth-9 PG Amodimethicone (and) Trideceth-12²² | 0.5 | - | 0.2 |
| Water | QSP | QSP | QSP |
| Total | 100% | 100% | 100% |

| | | | |
|---|---|---|---|
| KEY: as first part of Table 3. | | | |

### Example methods of use

Examples I, II, VII, VIII, or IX from Table 3 is applied to wet hair in an amount of about 2 ml per 2 gram of hair (dry weight). Tap water is employed to create a lather and spread the composition throughout the hair and scalp. The composition is immediately rinsed from the hair. The hair may further be conditioned.

## Claims

1. A cosmetic composition comprising:
(i) A copolymer comprising:
(A) from 0.1 mol-% to 99.8 mol-% of one or more cationic structural units (A); and
(B) from 0.1 mol-% to 99.8 mol-% of one or more macromonomeric structural units (B); and
(C) from 0.1 mol-% to 99.8 mol-% of one or more structural units (C) which differ from the structural units (A) and (B); and
(ii) A cosmetically acceptable component;
wherein the one or more cationic structural units (A) are represented by the following general formulae (I) and/or (II): 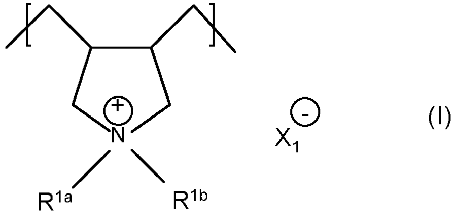 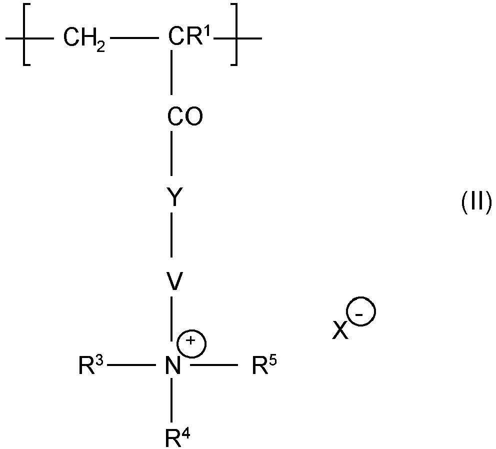
R¹ and R^{1a} are each identical or different and, independently of one another, are each hydrogen and/or a methyl radical,
R^{1b}, R³, R⁴ and R⁵ are each identical or different and, independently of one another, are each represented by hydrogen, an aliphatic hydrocarbon radical having 1 to 20 carbon atoms, preferably 1 to 4 carbon atoms, a cycloaliphatic hydrocarbon radical having 5 to 20 carbon atoms, preferably 5 to 8 carbon atoms, an aryl radical having 6 to 14 carbon atoms and/or polyethylene glycol (PEG); preferably are each identical or different and, independently of one another, are each represented by hydrogen and/or methyl; more preferably are each methyl,
Y is identical or different and is represented by oxygen, NH and/or NR³,
V is identical or different and is represented by -(CH₂)ₓ-, 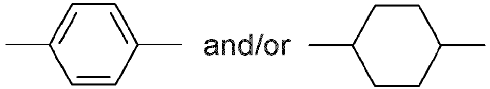
x is identical or different and is represented by an integer from 1 to 6,
X and X₁ are each identical or different and, independently of one another, are each represented by a halogen atom, C₁ to C₄ alkyl sulfate and/or C₁ to C₄- alkylsulfonate,
and the one or more macromonomeric structural units (B) are represented by the general formula (III): 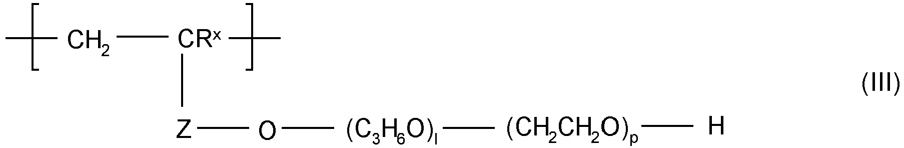 in which
R^{x} is identical or different and is represented by H and/or methyl,
Z is identical or different and is represented by C=O and/or O(CH₂)₄, preferably Z is O(CH₂)₄,
I is on molar average a number from 0 to 6, preferably from 0 to 5, and
p is, on molar average, a number from 1 to 150; preferably from 4 to 150; more preferably from 8 to 150,
and the one or more structural units (C) are selected from the group consisting of the polymerization product of at least one cyclic N-vinyl-substituted lactam having 5 to 7 ring atoms and structural units of the following general formulae (IV) and (V): 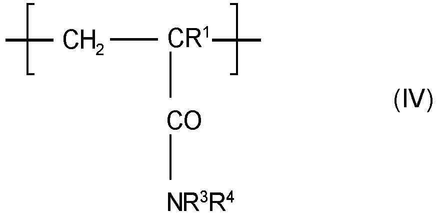 in which
R¹ is identical or different and is hydrogen and/or methyl, and
R³ and R⁴ are each identical or different and independently of one another are each represented by hydrogen, an aliphatic hydrocarbon radical having 1 to 20, preferably 1 to 4, carbon atoms, a cycloaliphatic hydrocarbon radical having 5 to 20, preferably 5 to 8, carbon atoms, an aryl radical having 6 to 14 Carbon atoms, an alkylaryl radical having 7 to 14 carbon atoms, a branched or unbranched C₁-C₅ monohydroxyalkyl group and/or polyethylene glycol (PEG) 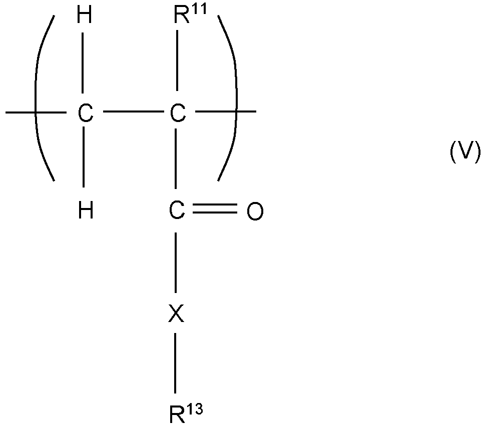
in which
R¹¹ is identical or different and is represented by H and/or methyl;
X is identical or different and is represented by NH-(CₙH₂ₙ) where n = 1, 2, 3 or 4; and
R¹³ is identical or different and is represented by OH, N(CH₃)₂, SO₃H, PO₃H₂, O-PO₃H₂ and/or para-substituted C₆H₄-SO₃H,
and where, among the structural units of the formula (V) in which R¹³ is N(CH₃)₂, preference is given to those structural units which represent the polymerization product of at least one monomer species selected from the group consisting of [3-(methacryloylamino)propyl]dimethylamine (R¹¹ = methyl; X = NH-(CₙH₂ₙ) where n = 3, and R¹³ = N(CH₃)₂) and [3-(acryloylamino)propyl]dimethylamine (R¹¹ = H; X = NH-(CₙH₂ₙ) where n = 3, and R¹³ = N(CH₃)₂),
and wherein the weight average molecular weight M_{w} of the copolymer is from 10 000 to 200 000 g/mol.

2. The composition according to claim 1, wherein the composition comprises a cleansing ingredient, preferably anionic surfactant, as cosmetically acceptable component (ii).

3. The composition according to any of the preceding claims, wherein the one or more cationic structural units (A) of the copolymer represent the polymerization product of at least one monomer species selected from the group consisting of [2-(acryloyloxy)ethyl]trimethylammonium chloride, [2-(acryloylamino)-ethyl]trimethylammonium chloride, [2-(acryloyloxy)ethyl]trimethylammonium methosulfate, [2-(methacryloyloxy)ethyl]trimethylammonium chloride and methosulfate, [3-(acryloylamino)propyl]trimethylammonium chloride, [3-(methacryloylamino)propyl]trimethylammonium chloride and diallyldimethylammonium chloride (DADMAC), preferably selected from the group consisting of [3-(acryloylamino)propyl]trimethylammonium chloride, [3-(methacryloylamino)propyl]trimethylammonium chloride, and diallyldimethylammonium chloride, and more preferably selected from the group consisting of [3-(methacryloylamino)propyl]trimethylammonium chloride and diallyldimethylammonium chloride, most preferably wherein the one or more cationic structural units (A) of the copolymer represent the polymerization product of diallyldimethylammonium chloride (DADMAC).

4. The composition according to any of the preceding claims, wherein the one or more macromonomeric structural units (B) of the formula (III) of the copolymer represent the polymerization product of at least one monomer species selected from the group consisting of polyethylene glycol vinyloxybutyl ether, polyethylene glycol-co-polypropylene glycol vinyloxybutyl ether (with I on molar average a number ≥ 1), polyethylene glycol (meth)acrylate and polyethylene glycol-co-polypropylene glycol (meth)acrylate (with I on molar average a number ≥ 1).

5. The composition according to any of the preceding claims, wherein the copolymer comprises from 20.0 to 80.0 mol-% of the one or more structural units (A), from 0.4 to 20.0 mol-% of the one or more structural units (B), and from 15.5 to 74.6 mol-% of one or more structural units (C);
preferably wherein the copolymer comprises from 22.0 to 77.6 mol-% of the one or more structural units (A), from 0.5 to 4.4 mol-% of the one or more structural units (B), and from 18.0 to 74.5 mol-% of one or more structural units (C).

6. The composition according to any of the preceding claims, wherein the one or more structural units (C) of the copolymer represent the polymerization product of at least one monomer species selected from the group consisting of non-cationic acrylamides, non-cationic methacrylamides, and cyclic N-vinyl-substituted lactams having 5 to 7 ring atoms.

7. The composition according to any of the preceding claims, wherein the one or more structural units (C) are selected from the general formula (IV).

8. The composition according to any of the preceding claims, wherein the one or more structural units (C) represent the polymerization product of at least one monomer species selected from the group consisting of acrylamide, methacrylamide, N-methylacrylamide, N,N'-dimethylacrylamide, N-ethylacrylamide, N-cyclohexylacrylamide, N-benzylacrylamide, N-methylolacrylamide, N-isopropylacrylamide, and N-tert-butylacrylamide, and preferably selected from the group consisting of N,N'-dimethylacrylamide and N-isopropylacrylamide.

9. The composition according to any of the preceding claims, wherein the weight average molecular weight M_{w} of the copolymer is from 15 000 to 200 000 g/mol, and preferably from 20 000 to 200 000 g/mol.

10. The composition according to any of the preceding claims, wherein the cosmetically acceptable component (ii) is selected from the group consisting of: cleansing ingredients, acidity regulators, colorants, further conditioning agents, emulsifiers, film formers, fragrances, glossers, humectants, lubricants, moisturizers, pigments, preservatives, hair penetration enhancers, scalp actives, stabilizers, further surfactants, thickeners, viscosity modifiers, and combinations thereof; more preferably, the cosmetically acceptable component is selected from the group consisting of surfactants, viscosity-modifying polymers and conditioning ingredients; even more preferably the composition comprises from 0.1 to 20 wt.-% of the copolymer (i) and further comprises at least 0.5 wt.-% cosmetically acceptable component selected from the group consisting of surfactants, polymers, conditioning agents, actives, acidity regulators, lubricants, moisturisers, oils, preservatives, sequestrants, strengtheners, sun protectors, and combinations thereof.

11. The composition according to any of the preceding claims, wherein the composition is a shampoo for cleansing keratin fibres, preferably is a hair cleansing composition.

12. A method of using a cosmetic composition comprising applying the composition according to any of claims 1 to 11 to keratinous material.

13. Use of a cosmetic composition for volumising keratinous fibres, wherein the cosmetic composition is according to any of claims 1 to 11.

14. A process for formulating a cosmetic composition comprising providing a copolymer and a cosmetically acceptable component and mixing the copolymer and a cosmetically acceptable component together to form a cosmetic composition,
wherein the copolymer comprises:
(A) from 0.1 mol-% to 99.8 mol-% of one or more cationic structural units (A); and
(B) from 0.1 mol-% to 99.8 mol-% of one or more macromonomeric structural units (B);
and
(C) from 0.1 mol-% to 99.8 mol-% of one or more structural units (C) which differ from the structural units (A) and (B);
wherein the one or more cationic structural units (A) are represented by the following general formulae (I) and/or (II): 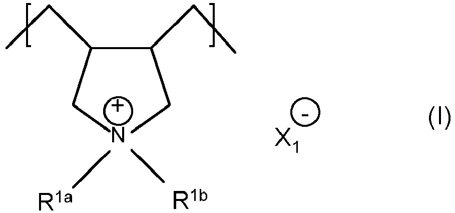 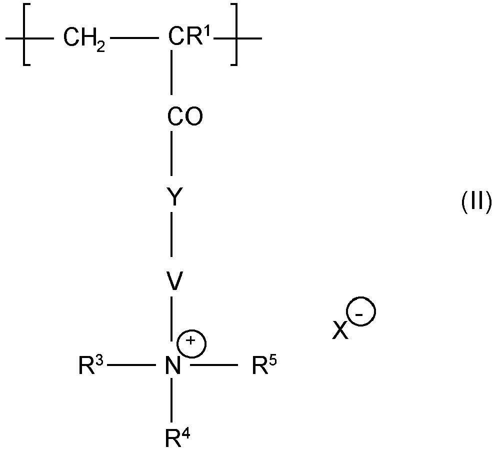
R¹ and R^{1a} are each identical or different and, independently of one another, are each hydrogen and/or a methyl radical,
R^{1b}, R³, R⁴ and R⁵ are each identical or different and, independently of one another, are each represented by hydrogen, an aliphatic hydrocarbon radical having 1 to 20 carbon atoms, preferably 1 to 4 carbon atoms, a cycloaliphatic hydrocarbon radical having 5 to 20 carbon atoms, preferably 5 to 8 carbon atoms, an aryl radical having 6 to 14 carbon atoms and/or polyethylene glycol (PEG), and preferably are each identical or different and, independently of one another, are each represented by hydrogen and/or methyl, and more preferably are each methyl,
Y is identical or different and is represented by oxygen, NH and/or NR³,
V is identical or different and is represented by -(CH₂)ₓ-, 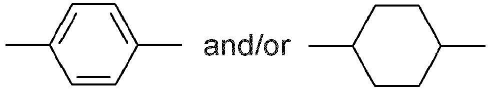
x is identical or different and is represented by an integer from 1 to 6,
X and X₁ are each identical or different and, independently of one another, are each represented by a halogen atom, C₁ to C₄ alkyl sulfate and/or C₁ to C₄- alkylsulfonate,
and the one or more macromonomeric structural units (B) are represented by the general formula (III): 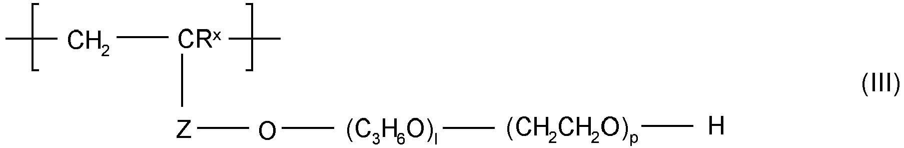 in which
R^{x} is identical or different and is represented by H and/or methyl,
Z is identical or different and is represented by C=O and/or O(CH₂)₄ and is preferably O(CH₂)₄,
I is on molar average a number from 0 to 6 and preferably from 0 to 5, and
p is, on molar average, a number from 1 to 150, preferably from 4 to 150, and more preferably from 8 to 150,
and the one or more structural units (C) are selected from the group consisting of the polymerization product of at least one cyclic N-vinyl-substituted lactam having 5 to 7 ring atoms and structural units of the following general formulae (IV) and (V): 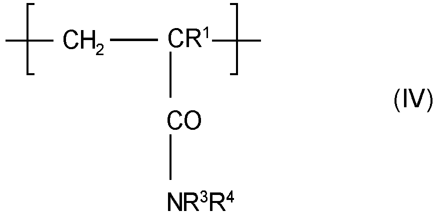 in which
R¹ is identical or different and is hydrogen and/or methyl, and
R³ and R⁴ are each identical or different and independently of one another are each represented by hydrogen, an aliphatic hydrocarbon radical having 1 to 20, preferably 1 to 4, carbon atoms, a cycloaliphatic hydrocarbon radical having 5 to 20, preferably 5 to 8, carbon atoms, an aryl radical having 6 to 14 Carbon atoms, an alkylaryl radical having 7 to 14 carbon atoms, a branched or unbranched C₁-C₅ monohydroxyalkyl group and/or polyethylene glycol (PEG) 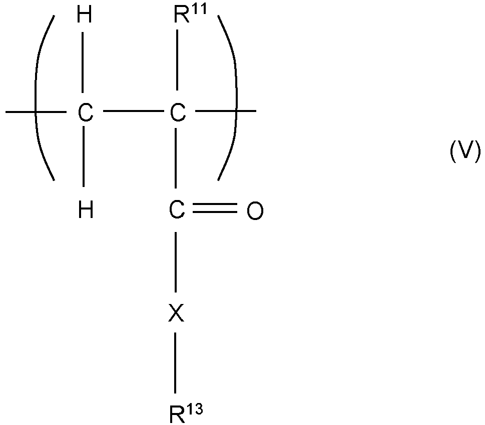
in which
R¹¹ is identical or different and is represented by H and/or methyl;
X is identical or different and is represented by NH-(CₙH₂ₙ) where n = 1, 2, 3 or 4; and
R¹³ is identical or different and is represented by OH, N(CH₃)₂, SO₃H, PO₃H₂, O-PO₃H₂ and/or para-substituted C₆H₄-SO₃H,
and where, among the structural units of the formula (V) in which R¹³ is N(CH₃)₂, preference is given to those structural units which represent the polymerization product of at least one monomer species selected from the group consisting of [3-(methacryloylamino)propyl]dimethylamine (R¹¹ = methyl; X = NH-(CₙH₂ₙ) where n = 3, and R¹³ = N(CH₃)₂) and [3-(acryloylamino)propyl]dimethylamine (R¹¹ = H; X = NH-(CₙH₂ₙ) where n = 3, and R¹³ = N(CH₃)₂),
and wherein the weight average molecular weight M_{w} of the copolymer is from 10 000 to 200 000 g/mol.

## Patentansprüche

1. Kosmetische Zusammensetzung, umfassend:
(i) ein Copolymer, umfassend:
(A) 0,1 Mol-% bis 99,8 Mol-% einer oder mehrerer kationischer Struktureinheiten (A) und
(B) 0,1 Mol-% bis 99,8 Mol-% einer oder mehrerer makromonomerer Struktureinheiten (B) und
(C) 0,1 Mol-% bis 99,8 Mol-% einer oder mehrerer Struktureinheiten (C), die von den Struktureinheiten (A) und (B) verschieden sind; und
(ii) eine kosmetisch unbedenkliche Komponente;
wobei die eine oder die mehreren kationischen Struktureinheiten (A) durch die folgenden allgemeinen Formeln (I) und/oder (II) repräsentiert werden: 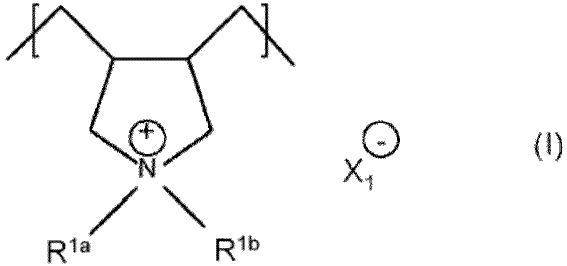 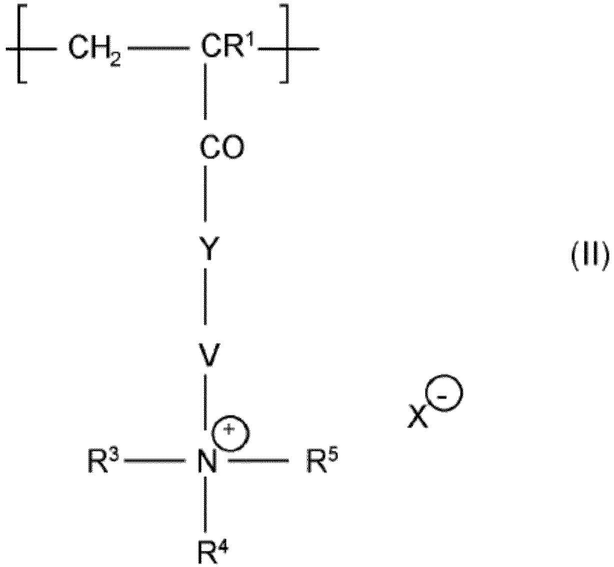
R¹ und R^{1a} jeweils gleich oder verschieden sind und unabhängig voneinander jeweils für Wasserstoff und/oder einen Methylrest stehen,
R^{1b}, R³, R⁴ und R⁵ jeweils gleich oder verschieden sind und unabhängig voneinander jeweils durch Wasserstoff, einen aliphatischen Kohlenwasserstoffrest mit 1 bis 20 Kohlenstoffatomen, vorzugsweise 1 bis 4 Kohlenstoffatomen, einen cycloaliphatischen Kohlenwasserstoffrest mit 5 bis 20 Kohlenstoffatomen, vorzugsweise 5 bis 8 Kohlenstoffatomen, einen Arylrest mit 6 bis 14 Kohlenstoffatomen und/oder Polyethylenglykol (PEG) repräsentiert werden, vorzugsweise jeweils gleich oder verschieden sind und unabhängig voneinander jeweils durch Wasserstoff und/oder Methyl repräsentiert werden und weiter bevorzugt jeweils Methyl bedeuten,
Y gleich oder verschieden ist und durch Sauerstoff, NH und/oder NR³ repräsentiert wird,
V gleich oder verschieden ist und durch-(CH₂)ₓ-, 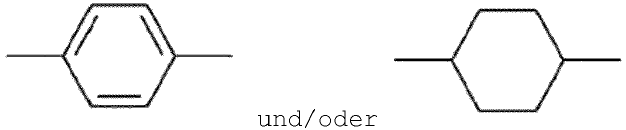
x repräsentiert wird, gleich oder verschieden ist und durch eine ganze Zahl von 1 bis 6 repräsentiert wird,
X und X₁ jeweils gleich oder verschieden sind und unabhängig voneinander jeweils durch ein Halogenatom, C₁- bis C₄-Alkylsulfat und/oder C₁- bis C₄-Alkylsulfonat repräsentiert werden, und die eine oder die mehreren makromonomeren Struktureinheiten (B) durch die allgemeine Formel (III) repräsentiert werden: 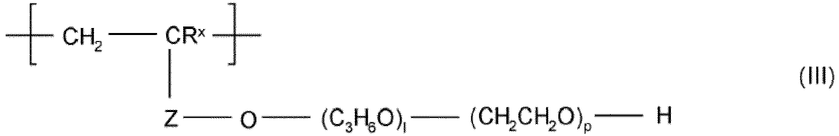
worin
R^{x} gleich oder verschieden ist und durch H und/oder Methyl repräsentiert wird,
Z gleich oder verschieden ist und durch C=O und/oder O(CH₂)₄ repräsentiert wird und vorzugsweise O(CH₂)₄ ist,
1 im molaren Mittel eine Zahl von 0 bis 6 und vorzugsweise von 0 bis 5 ist, und
p im molaren Mittel eine Zahl von 1 bis 150, vorzugsweise von 4 bis 150 und besonders bevorzugt von 8 bis 150 ist,
und die eine oder die mehreren Struktureinheiten (C) aus der Gruppe bestehend aus dem Polymerisationsprodukt von mindestens einem cyclischen N-Vinyl-substituierten Lactam mit 5 bis 7 Ringatomen und Struktureinheiten der folgenden allgemeinen Formeln (IV) und (V) ausgewählt sind: 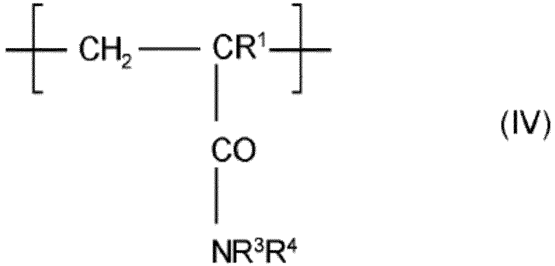 worin
R¹ gleich oder verschieden ist und Wasserstoff und/oder Methyl bedeutet und
R³ und R⁴ jeweils gleich oder verschieden sind und unabhängig voneinander jeweils durch Wasserstoff, einen aliphatischen Kohlenwasserstoffrest mit 1 bis 20 Kohlenstoffatomen, vorzugsweise 1 bis 4 Kohlenstoffatomen, einen cycloaliphatischen Kohlenwasserstoffrest mit 5 bis 20 Kohlenstoffatomen, vorzugsweise 5 bis 8 Kohlenstoffatomen, einen Arylrest mit 6 bis 14 Kohlenstoffatomen, einen Alkylarylrest mit 7 bis 14 Kohlenstoffatomen, eine verzweigte oder unverzweigte C₁-C₅-Monohydroxyalkylgruppe und/oder Polyethylenglykol (PEG) repräsentiert werden, 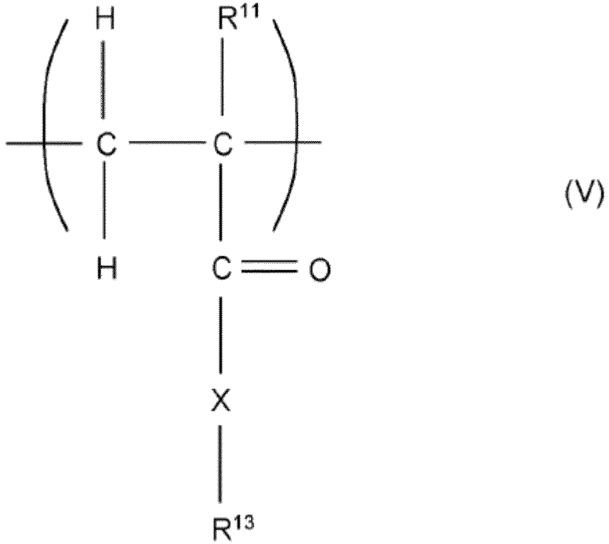
worin
R¹¹ gleich oder verschieden ist und durch H und/oder Methyl repräsentiert wird;
X gleich oder verschieden ist und durch NH-(CₙH₂ₙ) mit n = 1, 2, 3 oder 4 repräsentiert wird; und
R¹³ gleich oder verschieden ist und durch OH, N(CH₃)₂, SO₃H, PO₃H₂, O-PO₃H₂ und/oder para-substituiertes C₆H₄-SO₃H repräsentiert wird,
und wobei unter den Struktureinheiten der Formel (V), in denen R¹³ N(CH₃)₂ ist, diejenigen Struktureinheiten bevorzugt sind, welche das Polymerisationsprodukt mindestens einer Monomerspezies ausgewählt aus der Gruppe bestehend aus [3-(Methacryloylamino)propyl]dimethylamin (R¹¹ = Methyl; X = NH-(CₙH₂ₙ) mit n = 3 und R¹³ = N(CH₃)₂) und [3-(Acryloylamino)propyl]dimethylamin (R¹¹ = H; X = NH-(CₙH₂ₙ) mit n = 3 und R¹³ = N(CH₃)₂) darstellen,
und wobei das gewichtsmittlere Molekulargewicht M_{w} des Copolymers 10.000 bis 200.000 g/mol beträgt.

2. Zusammensetzung nach Anspruch 1, wobei die Zusammensetzung als kosmetisch unbedenkliche Komponente (ii) einen reinigenden Bestandteil, vorzugsweise ein anionisches Tensid, umfasst.

3. Zusammensetzung nach einem der vorhergehenden Ansprüche, wobei die eine oder die mehreren kationischen Struktureinheiten (A) des Copolymers das Polymerisationsprodukt mindestens einer Monomerspezies ausgewählt aus der Gruppe bestehend aus [2-(Acryloyloxy)-ethyl]trimethylammoniumchlorid, [2-(Acryloylamino)-ethyl]trimethylammoniumchlorid, [2-(Acryloyloxy)ethyl]-trimethylammoniummethosulfat, [2-(Methacryloyloxy)-ethyl]trimethylammoniumchlorid bzw. -methosulfat, [3-(Acryloylamino)propyl]trimethylammoniumchlorid, [3-(Methacryloylamino)propyl]trimethylammoniumchlorid und Diallyldimethylammoniumchlorid (DADMAC), vorzugsweise ausgewählt aus der Gruppe bestehend aus [3-(Acryloylamino)propyl]trimethylammoniumchlorid, [3-(Methacryloylamino)propyl]trimethylammoniumchlorid und Diallyldimethylammoniumchlorid und weiter bevorzugt ausgewählt aus der Gruppe bestehend aus [3-(Methacryloylamino)propyl]trimethylammoniumchlorid und Diallyldimethylammoniumchlorid darstellen, ganz besonders bevorzugt wobei die eine oder die mehreren kationischen Struktureinheiten (A) des Copolymers das Polymerisationsprodukt von Diallyldimethylammoniumchlorid (DADMAC) darstellen.

4. Zusammensetzung nach einem der vorhergehenden Ansprüche, wobei die eine oder die mehreren makromonomeren Struktureinheiten (B) der Formel (III) des Copolymers das Polymerisationsprodukt mindestens einer Monomerspezies ausgewählt aus der Gruppe bestehend aus Polyethylenglykolvinyloxybutylether, Polyethylenglykolco-polypropylenglykol-vinyloxybutylether (mit 1 im molaren Mittel eine Zahl ≥ 1), Polyethylenglykol(meth)-acrylat und Polyethylenglykol-co-polypropylenglykol-(meth)acrylat (mit 1 im molaren Mittel eine Zahl ≥ 1) darstellen.

5. Zusammensetzung nach einem der vorhergehenden Ansprüche, wobei das Copolymer 20,0 bis 80,0 Mol-% der einen oder der mehreren Struktureinheiten (A), 0,4 bis 20,0 Mol-% der einen oder der mehreren Struktureinheiten (B) und 15,5 bis 74,6 Mol-% einer oder mehrerer Struktureinheiten (C) umfasst,
vorzugsweise wobei das Copolymer 22,0 bis 77,6 Mol-% der einen oder der mehreren Struktureinheiten (A), 0,5 bis 4,4 Mol-% der einen oder der mehreren Struktureinheiten (B) und 18,0 bis 74,5 Mol-% einer oder mehrerer Struktureinheiten (C) umfasst.

6. Zusammensetzung nach einem der vorhergehenden Ansprüche, wobei das eine oder die mehreren Struktureinheiten (C) des Copolymers das Polymerisationsprodukt mindestens einer Monomerspezies ausgewählt aus der Gruppe bestehend aus nichtkationischen Acrylamiden, nichtkationischen Methacrylamiden und cyclischen N-Vinyl-substituierten Lactamen mit 5 bis 7 Ringatomen darstellen.

7. Zusammensetzung nach einem der vorhergehenden Ansprüche, wobei das eine oder die mehreren Struktureinheiten (C) aus der allgemeinen Formel (IV) ausgewählt sind.

8. Zusammensetzung nach einem der vorhergehenden Ansprüche, wobei die eine oder die mehreren Struktureinheiten (C) das Polymerisationsprodukt mindestens einer Monomerspezies ausgewählt aus der Gruppe bestehend aus Acrylamid, Methacrylamid, N-Methylacrylamid, N,N'-Dimethylacrylamid, N-Ethylacrylamid, N-Cyclohexylacrylamid, N-Benzylacrylamid, N-Methylolacrylamid, N-Isopropylacrylamid und N-tert-Butylacrylamid und vorzugsweise ausgewählt aus der Gruppe bestehend aus N,N'-Dimethylacrylamid und N-Isopropylacrylamid darstellen.

9. Zusammensetzung nach einem der vorhergehenden Ansprüche, wobei das gewichtsmittlere Molekulargewicht M_{w} des Copolymers 15.000 bis 200.000 g/mol und vorzugsweise 20.000 bis 200.000 g/mol beträgt.

10. Zusammensetzung nach einem der vorhergehenden Ansprüche, wobei die kosmetisch unbedenkliche Komponente (ii) ausgewählt ist aus der Gruppe bestehend aus: reinigenden Bestandteilen, Säureregulatoren, Farbmitteln, weiteren Konditionierungsmitteln, Emulgatoren, Filmbildnern, Duftstoffen, Glanzmitteln, Feuchthaltemitteln, Gleitmitteln, Feuchtigkeitsmitteln, Pigmenten, Konservierungsstoffen, Haarpenetrationsverbesserern, Kopfhautwirkstoffen, Stabilisatoren, weiteren Tensiden, Verdickern, Viskositätsmodifikatoren und Kombinationen davon; weiter bevorzugt die kosmetisch unbedenkliche Komponente aus der Gruppe bestehend aus Tensiden, viskositätsmodifizierenden Polymeren und konditionierenden Bestandteilen ausgewählt ist; noch weiter bevorzugt die Zusammensetzung 0,1 bis 20 Gew.-% des Copolymers (i) umfasst und ferner mindestens 0,5 Gew.-% kosmetisch unbedenkliche Komponenten ausgewählt aus der Gruppe bestehend aus Tensiden, Polymeren, Konditionierungsmitteln, Wirkstoffen, Säureregulatoren, Gleitmitteln, Feuchtigkeitsmitteln, Ölen, Konservierungsstoffen, Sequestriermitteln, Verstärkungsmitteln, Sonnenschutzmitteln und Kombinationen davon umfasst.

11. Zusammensetzung nach einem der vorhergehenden Ansprüche, wobei es sich bei der Zusammensetzung um ein Shampoo zur Reinigung von Keratinfasern und vorzugsweise um eine Haarreinigungszusammensetzung handelt.

12. Verfahren zur Verwendung einer kosmetischen Zusammensetzung, umfassend das Aufbringen der Zusammensetzung nach einem der Ansprüche 1 bis 11 auf Keratinmaterial.

13. Verwendung einer kosmetischen Zusammensetzung zur Volumengebung von Keratinfasern, wobei die kosmetische Zusammensetzung gemäß einem der Ansprüche 1 bis 11 ist.

14. Verfahren zum Formulieren einer kosmetischen Zusammensetzung, umfassend das Bereitstellen eines Copolymers und einer kosmetisch unbedenklichen Komponente und das Mischen des Copolymers und einer kosmetisch unbedenklichen Komponente miteinander zur Bildung einer kosmetischen Zusammensetzung,
wobei das Copolymer Folgendes umfasst:
(A) 0,1 Mol-% bis 99,8 Mol-% einer oder mehrerer kationischer Struktureinheiten (A) und
(B) 0,1 Mol-% bis 99,8 Mol-% einer oder mehrerer makromonomerer Struktureinheiten (B) und
(C) 0,1 Mol-% bis 99,8 Mol-% einer oder mehrerer Struktureinheiten (C), die von den Struktureinheiten (A) und (B) verschieden sind;
wobei die eine oder die mehreren kationischen Struktureinheiten (A) durch die folgenden allgemeinen Formeln (I) und/oder (II) repräsentiert werden: 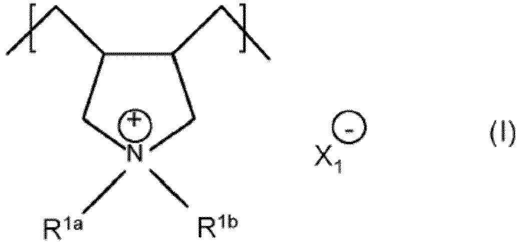 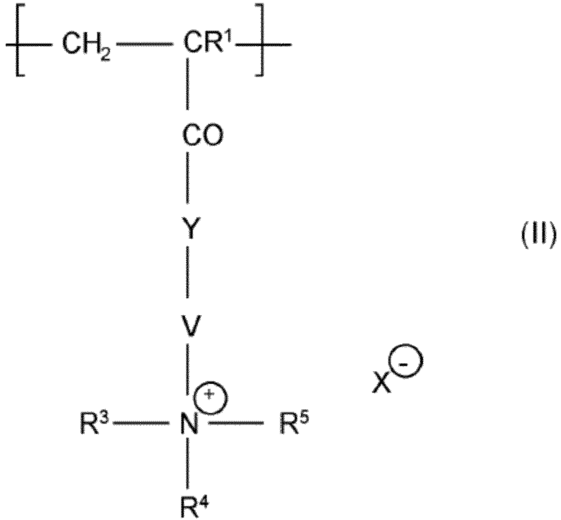
R¹ und R^{1a} jeweils gleich oder verschieden sind und unabhängig voneinander jeweils für Wasserstoff und/oder einen Methylrest stehen,
R^{1b}, R³, R⁴ und R⁵ jeweils gleich oder verschieden sind und unabhängig voneinander jeweils durch Wasserstoff, einen aliphatischen Kohlenwasserstoffrest mit 1 bis 20 Kohlenstoffatomen, vorzugsweise 1 bis 4 Kohlenstoffatomen, einen cycloaliphatischen Kohlenwasserstoffrest mit 5 bis 20 Kohlenstoffatomen, vorzugsweise 5 bis 8 Kohlenstoffatomen, einen Arylrest mit 6 bis 14 Kohlenstoffatomen und/oder Polyethylenglykol (PEG) repräsentiert werden, und vorzugsweise jeweils gleich oder verschieden sind und unabhängig voneinander jeweils durch Wasserstoff und/oder Methyl repräsentiert werden und weiter bevorzugt jeweils Methyl bedeuten,
Y gleich oder verschieden ist und durch Sauerstoff, NH und/oder NR³ repräsentiert wird,
V gleich oder verschieden ist und durch-(CH2)ₓ-, 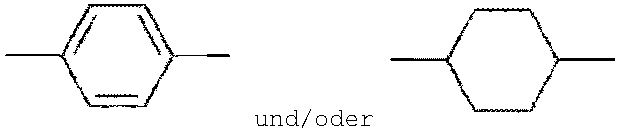
x repräsentiert wird, gleich oder verschieden ist und durch eine ganze Zahl von 1 bis 6 repräsentiert wird,
X und X₁ jeweils gleich oder verschieden sind und unabhängig voneinander jeweils durch ein Halogenatom, C₁- bis C₄-Alkylsulfat und/oder C₁- bis C₄-Alkylsulfonat repräsentiert werden,
und die eine oder die mehreren makromonomeren Struktureinheiten (B) durch die allgemeine Formel (III) repräsentiert werden: 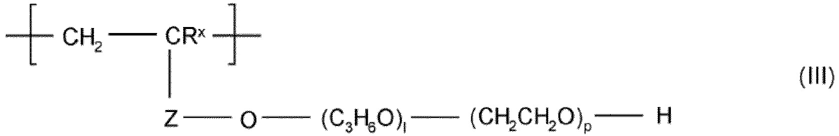 worin
R^{x} gleich oder verschieden ist und durch H und/oder Methyl repräsentiert wird,
Z gleich oder verschieden ist und durch C=O und/oder O(CH₂)₄ repräsentiert wird und vorzugsweise O(CH₂)₄ ist,
1 im molaren Mittel eine Zahl von 0 bis 6 und vorzugsweise von 0 bis 5 ist, und
p im molaren Mittel eine Zahl von 1 bis 150, vorzugsweise von 4 bis 150 und besonders bevorzugt von 8 bis 150 ist,
und die eine oder die mehreren Struktureinheiten (C) aus der Gruppe bestehend aus dem Polymerisationsprodukt von mindestens einem cyclischen N-Vinyl-substituierten Lactam mit 5 bis 7 Ringatomen und Struktureinheiten der folgenden allgemeinen Formeln (IV) und (V) ausgewählt sind: 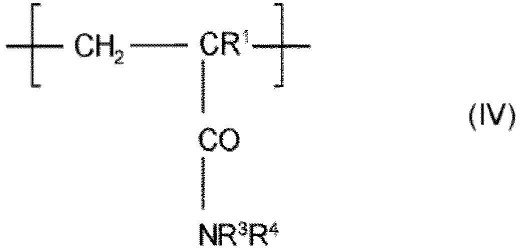 worin
R¹ gleich oder verschieden ist und Wasserstoff und/oder Methyl bedeutet und
R³ und R⁴ jeweils gleich oder verschieden sind und unabhängig voneinander jeweils durch Wasserstoff, einen aliphatischen Kohlenwasserstoffrest mit 1 bis 20 Kohlenstoffatomen, vorzugsweise 1 bis 4 Kohlenstoffatomen, einen cycloaliphatischen Kohlenwasserstoffrest mit 5 bis 20 Kohlenstoffatomen, vorzugsweise 5 bis 8 Kohlenstoffatomen, einen Arylrest mit 6 bis 14 Kohlenstoffatomen, einen Alkylarylrest mit 7 bis 14 Kohlenstoffatomen, eine verzweigte oder unverzweigte C₁-C₅-Monohydroxyalkylgruppe und/oder Polyethylenglykol (PEG) repräsentiert werden, 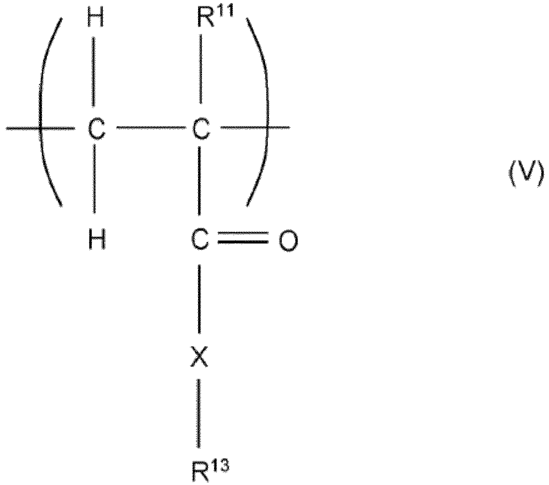
worin
R¹¹ gleich oder verschieden ist und durch H und/oder Methyl repräsentiert wird;
X gleich oder verschieden ist und durch NH-(CₙH₂ₙ) mit n = 1, 2, 3 oder 4 repräsentiert wird; und
R¹³ gleich oder verschieden ist und durch OH, N(CH₃)₂, SO₃H, PO₃H₂, O-PO₃H₂ und/oder para-substituiertes C₆H₄-SO₃H repräsentiert wird,
und wobei unter den Struktureinheiten der Formel (V), in denen R¹³ N(CH₃)₂ ist, diejenigen Struktureinheiten bevorzugt sind, welche das Polymerisationsprodukt mindestens einer Monomerspezies ausgewählt aus der Gruppe bestehend aus [3-(Methacryloylamino)propyl]dimethylamin (R¹¹ = Methyl; X = NH-(CₙH₂ₙ) mit n = 3 und R¹³ = N(CH₃)₂) und [3-(Acryloylamino)propyl]dimethylamin (R¹¹ = H; X = NH-(CₙH₂ₙ) mit n = 3 und R¹³ = N(CH₃)₂) darstellen,
und wobei das gewichtsmittlere Molekulargewicht M_{w} des Copolymers 10.000 bis 200.000 g/mol beträgt.

## Revendications

1. Composition cosmétique comprenant :
(i) un copolymère comprenant :
(A) de 0,1 % en moles à 99,8 % en moles d'un ou plusieurs motifs structuraux cationiques (A) ; et
(B) de 0,1 % en moles à 99,8 % en moles d'un ou plusieurs motifs structuraux macromonomériques (B) ; et
(C) de 0,1 % en moles à 99,8 % en moles d'un ou plusieurs motifs structuraux (C) qui diffèrent des motifs structuraux (A) et (B) ; et
(ii) un composant acceptable sur le plan cosmétique ; et
le ou les motifs structuraux cationiques (A) étant représentés par les formules générales suivantes (I) et/ou (II) : 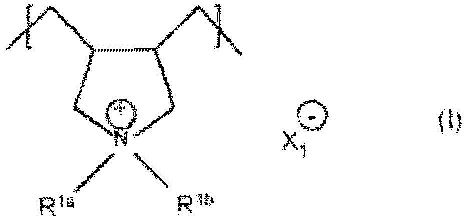 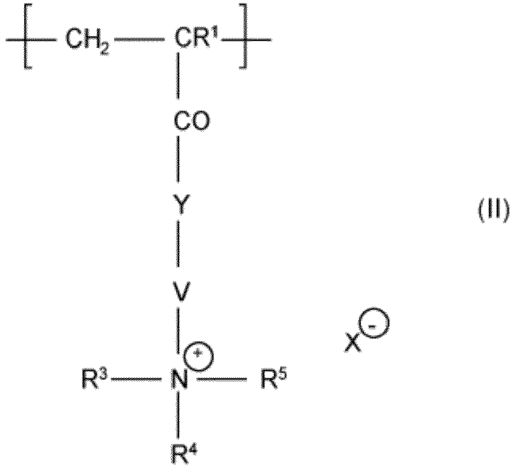
R¹ et R^{1a} étant chacun identiques ou différents et, indépendamment l'un de l'autre, étant chacun hydrogène et/ou un radical méthyle, R^{1b}, R³, R⁴ et R⁵ étant chacun identiques ou différents et, indépendamment l'un de l'autre, chacun représentés par hydrogène, un radical hydrocarboné aliphatique possédant 1 à 20 atomes de carbone, préférablement 1 à 4 atomes de carbone, un radical hydrocarboné cycloaliphatique possédant 5 à 20 atomes de carbone, préférablement 5 à 8 atomes de carbone, un radical aryle possédant 6 à 14 atomes de carbone et/ou un polyéthylèneglycol (PEG); préférablement étant chacun identiques ou différents et, indépendamment l'un de l'autre, étant chacun représentés par hydrogène et/ou méthyle ; plus préférablement étant chacun méthyle,
Y étant identique ou différent et étant représenté par oxygène, NH et/ou NR3,
V étant identique ou différent et étant représenté par -(CH₂)ₓ-, 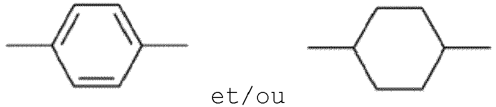
x étant identique ou différent et étant représenté par un entier de 1 à 6,
X et X₁ étant chacun identiques ou différents et, indépendamment l'un de l'autre, étant chacun représentés par un atome d'halogène, sulfate de C₁ à C₄ alkyle et/ou C₁ à C₄- alkylsulfonate,
et le ou les motifs structuraux macromonomériques (B) étant représentés par la formule générale (III) : 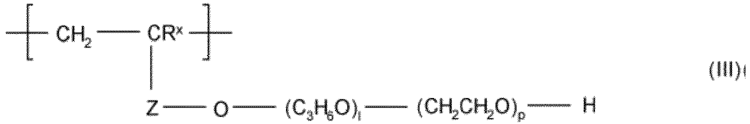
dans laquelle
R^{x} est identique ou différent et est représenté par H et/ou méthyle,
Z est identique ou différent et est représenté par C=O et/ou O(CH₂)₄, préférablement Z est O(CH₂)₄,
I est en moyenne molaire un nombre de 0 à 6, préférablement de 0 à 5, et
p est, en moyenne molaire, un nombre de 1 à 150 ; préférablement de 4 à 150 ; plus préférablement de 8 à 150,
et le ou les motifs structuraux (C) étant choisis dans le groupe constitué par le produit de polymérisation d'au moins un lactame N-vinyl-substitué cyclique possédant 5 à 7 atomes de cycle et des motifs structuraux des formules générales suivantes (IV) et (V) : 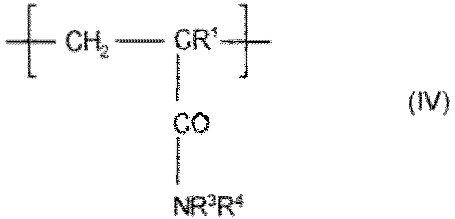
dans laquelle
R¹ est identique ou différent et est hydrogène et/ou méthyle, et
R³ et R⁴ sont chacun identiques ou différents et indépendamment l'un de l'autre sont chacun représentés par hydrogène, un radical hydrocarboné aliphatique possédant 1 à 20, préférablement 1 à 4, atomes de carbone, un radical hydrocarboné cycloaliphatique possédant 5 à 20, préférablement 5 à 8, atomes de carbone, un radical aryle possédant 6 à 14 atomes de carbone, un radical alkylaryle possédant 7 à 14 atomes de carbone, un groupe monohydroxyalkyle en C₁-C₅ ramifié ou non ramifié et/ou un polyéthylèneglycol (PEG) 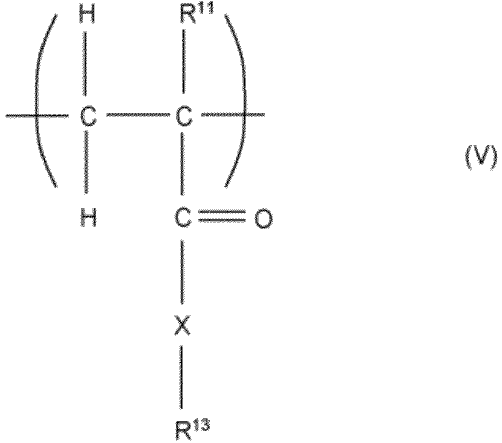
dans laquelle
R¹¹ est identique ou différent et est représenté par H et/ou méthyle ;
X est identique ou différent et est représenté par NH-(CₙH₂ₙ) où n = 1, 2, 3 ou 4 ; et
R¹³ est identique ou différent et est représenté par OH, N(CH₃)₂, SO₃H, PO₃H₂, O-PO₃H₂ et/ou C₆H₄-SO₃H substitué en para,
et où, parmi les motifs structuraux de la formule (V) dans laquelle R¹³ est N(CH₃)₂, préférence est donnée aux motifs structuraux qui représentent le produit de polymérisation d'au moins une espèce monomérique choisie dans le groupe constitué par [3-(méthacryloylamino)propyl]diméthylamine (R¹¹ = méthyle ; X = NH-(CₙH₂ₙ) où n = 3, et R¹³ = N(CH₃)₂) et [3-(acryloylamino)propyl]diméthylamine (R¹¹ = H ; X = NH-(CₙH₂ₙ) où n = 3, et R¹³ = N(CH₃)₂),
et le poids moléculaire moyen en poids M_{w} du copolymère étant de 10 000 à 200 000 g/mole.

2. Composition selon la revendication 1, la composition comprenant un ingrédient de nettoyage, préférablement un tensioactif anionique, en tant que composant acceptable sur le plan cosmétique (ii).

3. Composition selon l'une quelconque des revendications précédentes, le ou les motifs structuraux cationiques (A) du copolymère représentant le produit de polymérisation d'au moins une espèce monomérique choisie dans le groupe constitué par le chlorure de [2-(acryloyloxy)éthyl]triméthylammonium, le chlorure de [2-(acryloylamino)-éthyl]triméthylammonium, le méthosulfate de [2-(acryloyloxy)éthyl]triméthylammonium, le chlorure et le méthosulfate de [2-(méthacryloyloxy)éthyl]triméthylammonium, le chlorure de [3-(acryloylamino)propyl]triméthylammonium, le chlorure de [3-(méthacryloylamino)propyl]triméthylammonium et le chlorure de diallyldiméthylammonium (DADMAC), préférablement choisi dans le groupe constitué par le chlorure de [3-(acryloylamino)propyl]triméthylammonium, le chlorure de [3-(méthacryloylamino)propyl]triméthylammonium, et le chlorure de diallyldiméthylammonium, et plus préférablement choisi dans le groupe constitué par le chlorure de [3-(méthacryloylamino)propyl]triméthylammonium et le chlorure de diallyldiméthylammonium, le plus préférablement, le ou les motifs structuraux cationiques (A) du copolymère représentant le produit de polymérisation du chlorure de diallyldiméthylammonium (DADMAC).

4. Composition selon l'une quelconque des revendications précédentes, le ou les motifs structuraux macromonomériques (B) de formule (III) du copolymère représentant le produit de polymérisation d'au moins une espèce monomérique choisie dans le groupe constitué par l'éther vinyloxybutylique de polyéthylèneglycol, l'éther vinyloxybutylique de polyéthylèneglycol-co-polypropylèneglycol (I étant en moyenne molaire un nombre ≥ ; 1), un(méth)acrylate de polyéthylène glycol et un (méth)acrylate de polyéthylène glycol-co-polypropylène glycol (I étant en moyenne molaire un nombre ≥ 1).

5. Composition selon l'une quelconque des revendications précédentes, le copolymère comprenant de 20,0 à 80,0 % en moles du ou des motifs structuraux (A), de 0,4 à 20,0 % en moles du ou des motifs structuraux (B), et de 15,5 à 74,6 % en moles du ou des motifs structuraux (C) ;
préférablement, le copolymère comprenant de 22,0 à 77,6 % en moles du ou des motifs structuraux (A), de 0,5 à 4,4 % en moles du ou des motifs structuraux (B), et de 18,0 à 74,5 % en moles du ou des motifs structuraux (C).

6. Composition selon l'une quelconque des revendications précédentes, le ou les motifs structuraux (C) du copolymère représentant le produit de polymérisation d'au moins une espèce monomérique choisie dans le groupe constitué par des acrylamides non cationiques, des méthacrylamides non cationiques et des lactames N-vinyl-substitués cycliques possédant 5 à 7 atomes de cycle.

7. Composition selon l'une quelconque des revendications précédentes, le ou les motifs structuraux (C) étant choisis parmi la formule générale (IV).

8. Composition selon l'une quelconque des revendications précédentes, le ou les motifs structuraux (C) représentant le produit de polymérisation d'au moins une espèce monomérique choisie dans le groupe constitué par l'acrylamide, le méthacrylamide, le N-méthylacrylamide, le N,N'-diméthylacrylamide, le N-éthylacrylamide, le N-cyclohexylacrylamide, le N-benzylacrylamide, le N-méthylolacrylamide, le N-isopropylacrylamide et le N-tert-butylacrylamide, et préférablement choisi dans le groupe constitué par le N,N'-diméthylacrylamide et le N-isopropylacrylamide.

9. Composition selon l'une quelconque des revendications précédentes, le poids moléculaire moyen en poids M_{w} du copolymère étant de 15 000 à 200 000 g/mole, et préférablement de 20 000 à 200 000 g/mole.

10. Composition selon l'une quelconque des revendications précédentes, le composant acceptable sur le plan cosmétique (ii) étant choisi dans le groupe constitué par : des ingrédients de nettoyage, des régulateurs d'acidité, des colorants, d'autres agents de conditionnement, des émulsifiants, des agents filmogènes, des fragrances, des agents brillants, des humectants, des lubrifiants, des hydratants, des pigments, des conservateurs, des agents améliorant la pénétration dans les cheveux, des agents actifs pour le cuir chevelu, des stabilisants, d'autres agents tensioactifs, des épaississants, des modificateurs de viscosité, et des combinaisons correspondantes ; plus préférablement, le composant acceptable sur le plan cosmétique étant choisi dans le groupe constitué par les agents tensioactifs, les polymères modificateurs de viscosité et les ingrédients de conditionnement ; encore plus préférablement, la composition comprenant de 0,1 à 20 % en poids % en poids du copolymère (i) et comprenant en outre au moins 0,5 % en poids de composant acceptable sur le plan cosmétique choisi dans le groupe constitué par les tensioactifs, les polymères, les agents de conditionnement, les agents actifs, les régulateurs d'acidité, les lubrifiants, les agents hydratants, les huiles, les conservateurs, les agents séquestrants, les renforçateurs, les agents de protection solaire, et des combinaisons correspondantes.

11. Composition selon l'une quelconque des revendications précédentes, la composition étant un shampooing pour le nettoyage de fibres de kératine, préférablement étant une composition de nettoyage pour cheveux.

12. Procédé d'utilisation d'une composition cosmétique comprenant l'application de la composition selon l'une quelconque des revendications 1 à 11 sur une matière kératinique.

13. Utilisation d'une composition cosmétique pour donner du volume à des fibres kératiniques, la composition cosmétique étant selon l'une quelconque des revendications 1 à 11.

14. Procédé pour la formulation d'une composition cosmétique comprenant la fourniture d'un copolymère et d'un composant acceptable sur le plan cosmétique et le mélange du copolymère et d'un composant acceptable sur le plan cosmétique ensemble pour former une composition cosmétique,
le copolymère comprenant :
(A) de 0,1 % en moles à 99,8 % en moles d'un ou plusieurs motifs structuraux cationiques (A) ; et
(B) de 0,1 % en moles à 99,8 % en moles d'un ou plusieurs motifs structuraux macromonomériques (B) ; et
(C) de 0,1 % en moles à 99,8 % en moles d'un ou plusieurs motifs structuraux (C) qui diffèrent des motifs structuraux (A) et (B) ;
le ou les motifs structuraux cationiques (A) étant représentés par les formules générales suivantes (I) et/ou (II) : 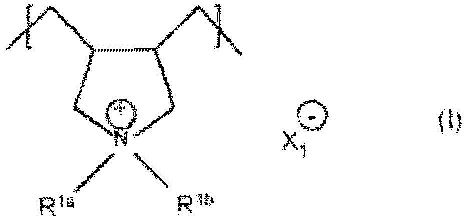 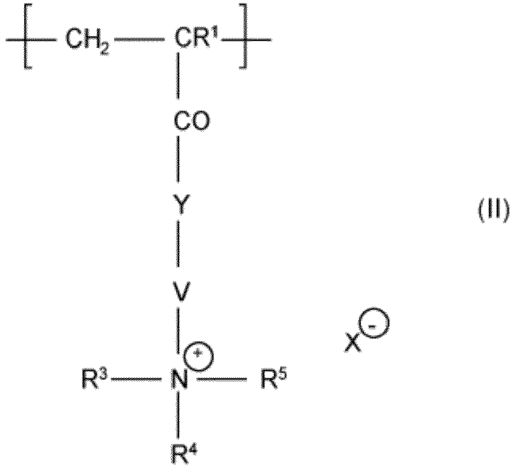
R¹ et R^{1a} étant chacun identiques ou différents et, indépendamment l'un de l'autre, étant chacun hydrogène et/ou un radical méthyle, R^{1b}, R³, R⁴ et R⁵ étant chacun identiques ou différents et, indépendamment l'un de l'autre, chacun représentés par hydrogène, un radical hydrocarboné aliphatique possédant 1 à 20 atomes de carbone, préférablement 1 à 4 atomes de carbone, un radical hydrocarboné cycloaliphatique possédant 5 à 20 atomes de carbone, préférablement 5 à 8 atomes de carbone, un radical aryle possédant 6 à 14 atomes de carbone et/ou un polyéthylèneglycol (PEG); préférablement étant chacun identiques ou différents et, indépendamment l'un de l'autre, étant chacun représentés par hydrogène et/ou méthyle ; plus préférablement étant chacun méthyle,
Y étant identique ou différent et étant représenté par oxygène, NH et/ou NR³,
V étant identique ou différent et étant représenté par -(CH₂)ₓ-, 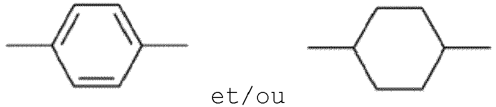
x étant identique ou différent et étant représenté par un entier de 1 à 6,
X et X₁ étant chacun identiques ou différents et, indépendamment l'un de l'autre, étant chacun représentés par un atome d'halogène, sulfate de C₁ à C₄ alkyle et/ou C₁ à C₄-alkylsulfonate,
et le ou les motifs structuraux macromonomériques (B) étant représentés par la formule générale (III) : 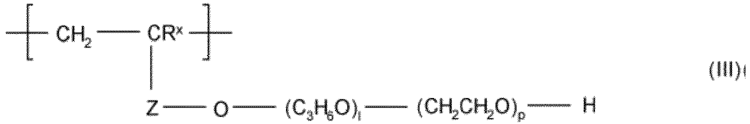
dans laquelle
R^{x} est identique ou différent et est représenté par H et/ou méthyle,
Z est identique ou différent et est représenté par C=O et/ou O(CH₂)₄, préférablement Z est O(CH₂)₄,
I est en moyenne molaire un nombre de 0 à 6, préférablement de 0 à 5, et
p est, en moyenne molaire, un nombre de 1 à 150 ; préférablement de 4 à 150 ; plus préférablement de 8 à 150,
et le ou les motifs structuraux (C) étant choisis dans le groupe constitué par le produit de polymérisation d'au moins un lactame N-vinyl-substitué cyclique possédant 5 à 7 atomes de cycle et des motifs structuraux des formules générales suivantes (IV) et (V) : 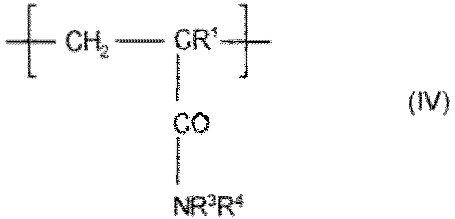
dans laquelle
R¹ est identique ou différent et est hydrogène et/ou méthyle, et
R³ et R⁴ sont chacun identiques ou différents et indépendamment l'un de l'autre sont chacun représentés par hydrogène, un radical hydrocarboné aliphatique possédant 1 à 20, préférablement 1 à 4, atomes de carbone, un radical hydrocarboné cycloaliphatique possédant 5 à 20, préférablement 5 à 8, atomes de carbone, un radical aryle possédant 6 à 14 atomes de carbone, un radical alkylaryle possédant 7 à 14 atomes de carbone, un groupe monohydroxyalkyle en C₁ à C₅ ramifié ou non ramifié et/ou un polyéthylèneglycol (PEG) 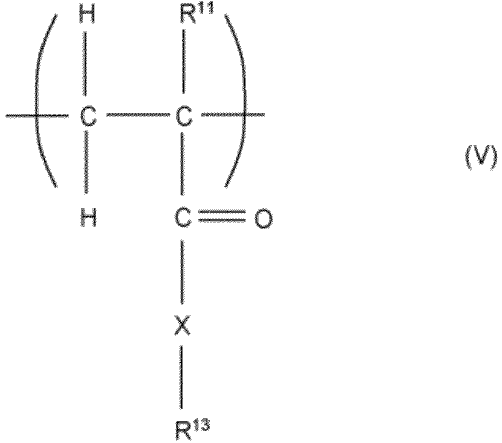
dans laquelle
R¹¹ est identique ou différent et est représenté par H et/ou méthyle ;
X est identique ou différent et est représenté par NH-(CₙH₂ₙ) où n = 1, 2, 3 ou 4 ; et
R¹³ est identique ou différent et est représenté par OH, N(CH₃)₂, SO₃H, PO₃H₂, O-PO₃H₂ et/ou C₆H₄-SO₃H substitué en para,
et où, parmi les motifs structuraux de la formule (V) dans laquelle R¹³ est N(CH₃)₂, préférence est donnée aux motifs structuraux qui représentent le produit de polymérisation d'au moins une espèce monomérique choisie dans le groupe constitué par [3-(méthacryloylamino)propyl]diméthylamine (R¹¹ = méthyle ; X = NH-(CₙH₂ₙ) où n = 3, et R¹³ = N(CH₃)₂) et [3-(acryloylamino)propyl]diméthylamine (R¹¹ = H ; X = NH-(CₙH₂ₙ) où n = 3, et R¹³ = N(CH₃)₂),
et le poids moléculaire moyen en poids M_{w} du copolymère étant de 10 000 à 200 000 g/mole.
